# EUROPEAN PATENT SPECIFICATION

(11) **EP 3 045 530 B1**
(45) Date of publication and mention of the grant of the patent: **11.07.2018**
(21) Application number: 14844108.2
(22) Date of filing: 10.09.2014
(51) Int. Cl.: C12N 5/07, C12N 5/074, C12N 5/10, C07K 14/78

(54) **CULTURE METHOD FOR PLURIPOTENT STEM CELLS AND KIT FOR CULTURE OF PLURIPOTENT STEM CELLS USED THEREIN**
KULTURVERFAHREN FÜR PLURIPOTENTE STAMMZELLEN SOWIE KIT FÜR DARIN VERWENDETE PLURIPOTENTE STAMMZELLEN
PROCÉDÉ DE CULTURE DE CELLULES SOUCHES PLURIPOTENTES ET KIT DE CULTURE ASSOCIÉS

(30) Priority: 10.09.2013 JP 2013187441
(43) Date of publication of application: 20.07.2016
(73) Proprietor: Fujifilm Corporation, Minato-ku Tokyo 106-8620 (JP)
(72) Inventor: HAGIYA, Keita, Ashigarakami-gun Kanagawa 258-8577 (JP); MURAKAMI, Yuta, Ashigarakami-gun Kanagawa 258-8577 (JP); YOSHINO, Yuichi, Ashigarakami-gun Kanagawa 258-8577 (JP); NOMIYAMA, Sanae, Ashigarakami-gun Kanagawa 258-8577 (JP); HANDO, Rie, Ashigarakami-gun Kanagawa 258-8577 (JP); IWAKI, Yoshihide, Ashigarakami-gun Kanagawa 258-8577 (JP); SASAKI, Tasuku, Ashigarakami-gun Kanagawa 258-8577 (JP)
(74) Representative: Hoffmann Eitle
(86) International application number: PCT/JP2014/073948
(87) International publication number: WO 2015/037623

(56) References cited:
- EP-A1- 2 457 998
- WO-A1-2013/164970
- WO-A2-2011/058558
- WO-A2-2011/058558
- US-A1- 2012 301 962
- US-A1- 2012 301 962
- Marie-Véronique Clément ET AL: "Original Research Communication The In Vitro Cytotoxicity of Ascorbate Depends on the Culture Medium Used to Perform the Assay and Involves Hydrogen Peroxide", , 1 January 2001 (2001-01-01), XP055272127, Retrieved from the Internet: URL:http://online.liebertpub.com/doi/abs/1 0.1089/152308601750100687 [retrieved on 2016-05-12]
- GUOKAI CHEN ET AL: "Chemically defined conditions for human iPSC derivation and culture", NATURE METHODS, vol. 8, no. 5, 1 May 2011 (2011-05-01), pages 424-429, XP055092759, ISSN: 1548-7091, DOI: 10.1038/nmeth.1593
- PROWSE A B J ET AL: "Long term culture of human embryonic stem cells on recombinant vitronectin in ascorbate free media", BIOMATERIALS, ELSEVIER SCIENCE PUBLISHERS BV., BARKING, GB, vol. 31, no. 32, 1 November 2010 (2010-11-01), pages 8281-8288, XP027259533, ISSN: 0142-9612 [retrieved on 2010-08-02]
- VERONIKA AKOPIAN ET AL: "Comparison of defined culture systems for feeder cell free propagation of human embryonic stem cells", IN VITRO CELLULAR & DEVELOPMENTAL BIOLOGY. ANIMAL., vol. 46, no. 3-4, 26 February 2010 (2010-02-26), pages 247-258, XP055269715, ISSN: 1071-2690, DOI: 10.1007/s11626-010-9297-z
- L.G. VILLA-DIAZ ET AL: "Concise Review: The Evolution of human pluripotent stem cell culture: From feeder cells to synthetic coatings", STEM CELLS, vol. 31, no. 1, 1 January 2013 (2013-01-01), pages 1-7, XP055061175, ISSN: 1066-5099, DOI: 10.1002/stem.1260
- STEFAN R BRAAM ET AL: "Recombinant Vitronectin Is a Functionally Defined Substrate That Supports Human Embryonic Stem Cell Self-Renewal via V 5 Integrin", STEM CELLS, ALPHAMED PRESS, DAYTON, OH, US, vol. 26, 1 January 2008 (2008-01-01), pages 2257-2265, XP007912130, ISSN: 1066-5099, DOI: 10.1634/STEMCELLS.2008-0291 [retrieved on 2008-07-03]
- ZARA MELKOUMIAN ET AL: "Synthetic peptide-acrylate surfaces for long-term self-renewal and cardiomyocyte differentiation of human embryonic stem cells", NATURE BIOTECHNOLOGY, NATURE PUBLISHING GROUP, US, vol. 28, no. 6, 1 June 2010 (2010-06-01), pages 606-610, XP002636474, ISSN: 1087-0156, DOI: 10.1038/NBT.1629 [retrieved on 2010-05-30]
- CHEN GUOKAI ET AL.: 'Chemically defined conditions for human iPSC derivation and culture' NATURE METHODS vol. 8, no. 5, May 2011, pages 424 - 429, XP055092759
- ' COMPARISON OF DEFINED CULTURE SYSTEMS FOR FEEDER CELL FREE PROPAGATION OF HUMAN EMBRYONIC STEM CELLS' VITRO CELL .DEV.BIOL.-ANIMAL vol. 46, 2010, pages 247 - 258, XP055269715
- FURUE, MIHO K. ET AL.: 'Heparin promotes the growth of human embryonic stem cells in a defined serum-free medium' PNAS vol. 105, no. 36, 09 September 2008, pages 13409 - 13414, XP055142731

## Description

### BACKGROUND OF THE INVENTION

### 1. Field of the Invention

The present invention relates to a culture method for pluripotent stem cells and to a kit for culture of pluripotent stem cells used in the culture method. 2. Description of the Related Art

For the purpose of the recovery of functions of damaged tissues, various regenerative medical techniques are being developed. Among these, a large number of techniques relating to totipotent or pluripotent stem cells of primates, particularly, human beings that is ultimately aimed at the regeneration of tissues has been reported. Especially, induced pluripotent stem cells (iPS cells) have an advantage in that these cells lessen ethical issues because they are induced from somatic cells unlike embryonic stem cells.

In a case where the pluripotent stem cells of primates are cultured, the cells need to be kept undifferentiated for a long period of time. For culturing the undifferentiated pluripotent stem cells for a long period of time, generally, feeder cells such as mouse fibroblasts are used.

However, it has been pointed out that the use of heterogeneous animal-derived feeder cells such as mouse fibroblasts leads to a likelihood that foreign substances such as heterogeneous animal-derived antigenic substances may be mixed into the culture solution. Therefore, in a case where the totipotent or pluripotent stem cells are used for medical purposes or for the purposes equivalent to medical purposes, the cells need to be cultured without feeder cells.

In consideration of the circumstances described above, cell-adhesive materials functioning as the feeder cells are being developed. For example, Nature Biotechnology, 2001, Vol. 19, pp. 971-974 discloses that human embryonic stem cells kept undifferentiated are successfully cultured by using matrix gel which is a component extracted from mouse sarcoma as a substituent for feeder cells.

JP2001-17183A discloses a feeder cell-free cellular composition containing growing primordial cells of primates, and discloses, as a preferred embodiment, a cellular composition further containing an extracelluar matrix. JP2010-29186A discloses a cell culture substrate in which a plasma-polymerized cell culture surface is additionally coated with a coating solution containing an extracellular matrix protein at a predetermined concentration and an aqueous solvent. JP2010-29186A describes that the cell culture substrate has excellent adhesiveness helpful to avoid the differentiation of embryonic stem cells.

Biomaterials, 2010, Nov; Vol. 31(32), pp. 8281-8288 and Nature biotechnology, 2010, Vol. 28, No. 6, pp. 606-610 disclose a recombinant peptide or a synthetic peptide having a partial sequence of vitronectin that makes a contribution to long-term culture of embryonic stem cells. Specifically, the above documents disclose a sequence consisting of the 1^{st} to 52^{nd} amino acids of natural vitronectin (see Biomaterials, 2010, Nov; Vol. 31(32), pp. 8281-8288) and a sequence consisting of the 41^{st} to 52^{nd} amino acids of natural vitronectin including an RGD sequence (see Nature biotechnology, 2010, Vol. 28, No. 6, pp. 606-610) respectively. It is known that these peptides make it possible to avoid a likelihood of intermixing of antigenic substances because they are non-biological samples and can be excellently produced in an industrial manner.

Nature Methods, 2011, vol. 8(5), pp.424-429 describes chemically defined conditions for human iPSC derivation and culture.

US 2012/0301962 A1 relates to vitronectin-derived cell culture substrates and methods of using the same for culturing pluripotent stem cells.

Furthermore, regarding a medium for culture of pluripotent stem cells in an undifferentiated state, for example, JP2013-510567A discloses ascorbic acid, basic fibroblast growth factor (bFGF), a serum-free medium which does not contain a heterogeneous component.

### SUMMARY OF THE INVENTION

JP2001-17183A, JP2010-29186A, Nature Biotechnology, 2001, Vol 19, pp. 971-974, Biomaterials, 2010, Nov; Vol. 31(32), pp. 8281-8288, and Nature biotechnology, 2010, Vol. 28, No. 6, pp. 606-610 disclose materials that can replace the feeder cells, and JP2013-510567A discloses a medium that is suitable for culture of pluripotent stem cells. However, there is a demand for a better culture method having excellent cell culture performance for pluripotent stem cells.

Accordingly, objects of the present invention are to provide a culture method for pluripotent stem cells that is excellent in cell culture performance for pluripotent stem cells, particularly, a cell growth ability for pluripotent stem cells, and to provide a kit and a medium for culture of pluripotent stem cells that are used in the culture method.

In order to achieve the aforementioned objects, the inventors of the present invention continued intensive research on the culture method for pluripotent stem cells. As a result, they obtained knowledge that if pluripotent stem cells are cultured using a polypeptide, which includes the amino acid sequence of human vitronectin or a predetermined partial amino acid sequence of human vitronectin, and a medium containing an ascorbic acid derivative in a predetermined amount, the cell culture performance, particularly, the cell growth ability for the pluripotent stem cells becomes excellent. Based on the knowledge, the inventors accomplished the present invention.

That is, a culture method for pluripotent stem cells of the present invention includes obtaining a polypeptide-coated culture surface by applying a polypeptide onto a cell culture surface of a support and culturing pluripotent stem cells by seeding the pluripotent stem cells onto the polypeptide-coated culture surface by using a medium in which the content of an ascorbic acid derivative is equal to or greater than 1.5 mmol/L (mM) and equal to or less than 100 mmol/L (mM), in which the polypeptide is (a) a polypeptide having an amino acid sequence represented by SEQ ID NO: 1, (b) a polypeptide having an amino acid sequence, which shares identity of equal to or higher than 80% with the amino acid sequence represented by SEQ ID NO: 1, and having culture performance for pluripotent stem cells, or (c) a polypeptide having an amino acid sequence, which is formed by the deletion, substitution, or addition of one amino acid or several amino acids (preferably 1 to 5 amino acids) in SEQ ID NO: 1, and having culture performance for pluripotent stem cells.

Furthermore, another culture method for pluripotent stem cells of the present invention is a method including obtaining a polypeptide-coated culture surface by applying a polypeptide onto a cell culture surface of a support and culturing pluripotent stem cells by seeding the pluripotent stem cells onto the polypeptide-coated cell culture surface by using a medium in which the content of an ascorbic acid derivative is equal to or greater than 1.5 mmol/L (mM) and equal to or less than 100 mmol/L (mM), in which the polypeptide is (d) a polypeptide consisting of 40 to 450 amino acid residues and including (1) a first region including at least one amino acid sequence selected from the group consisting of an amino acid sequence represented by CSYYQSC (SEQ ID NO: 2) and an amino acid sequence represented by RGD and (2) a second region including (2-i) an amino acid sequence which is represented by PRPSLAKKQRFRHRNRKGYRSQRGHSRGRNQN (SEQ ID NO: 3), (2-ii) an amino acid sequence which shares identity of equal to or higher than 80% with the amino acid sequence represented by SEQ ID NO: 3 and exhibits adsorbability with respect to the cell culture surface of the support, or (2-iii) an amino acid sequence which is formed by the addition, substitution, or deletion of one amino acid residue or several amino acid residues (preferably 1 to 5 amino acid residues) in the amino acid sequence represented by SEQ ID NO: 3 and exhibits adsorbability with respect to the cell culture surface of the support.

A kit for culture of pluripotent stem cells of the present invention is a kit including (a) a polypeptide having an amino acid sequence represented by SEQ ID NO: 1, (b) a polypeptide having an amino acid sequence, which shares identity of equal to or higher than 80% with the amino acid sequence represented by SEQ ID NO: 1, and having culture performance for pluripotent stem cells, or (c) a polypeptide having an amino acid sequence, which is formed by the deletion, substitution, or addition of one amino acid or several amino acids (preferably 1 to 5 amino acids) in SEQ ID NQ: 1, and having cell culture performance for pluripotent stem cells and a medium in which the content of an ascorbic acid derivative is equal to or greater than 1.5 mmol/L (mM) and equal to or less than 100 mmol/L (mM).

Furthermore, another kit for culture of pluripotent stem cells of the present invention is a kit including a polypeptide and a medium in which the content of an ascorbic acid derivative is equal to or greater than 1.5 mmol/L (mM) and equal to or less than 100 mmol/L (mM), in which the polypeptide is (d) a polypeptide consisting of 40 to 450 amino acid residues and including (1) a first region including at least one amino acid sequence selected from the group consisting of an amino acid sequence represented by CSYYQSC (SEQ ID NO: 2) and an amino acid sequence represented by RGD and (2) a second region including (2-i) an amino acid sequence which is represented by PRPSLAKKQRFRHRNRKGYRSQRGHSRGRNQN (SEQ ID NO: 3), (2-ii) an amino acid sequence which shares identity of equal to or higher than 80% with the amino acid sequence represented by SEQ ID NO: 3 and exhibits adsorbability with respect to a cell culture surface of a support, or (2-iii) an amino acid sequence which is formed by the deletion, substitution, or addition of one amino acid residue or several amino acid residues (preferably I to 5 amino acid residues) in the amino acid sequence represented by SEQ ID NO: 3 and exhibits adsorbability with respect to the cell culture surface of the support.

The medium of the present disclosure is a medium in which the content of an ascorbic acid derivative is equal to or greater than 1.5 mmol/L (mM).

According to the present invention, it is possible to provide a culture method for pluripotent stem cells that is excellent in cell culture performance for pluripotent stem cells, particularly, a cell growth ability for pluripotent stem cells and to provide a kit and a medium for culture of pluripotent stem cells that are used in the culture method.

### BRIEF DESCRIPTION OF THE DRAWINGS

Fig. 1 is a graph showing the results of a test performed to check how well each polypeptide according to the present invention is adsorbed onto the surface of a culture plate.
Fig. 2 is a graph showing growth curves of iPS cells using each polypeptide according to the present invention.
Fig. 3A shows a morphic image (left side) and a magnified image (right side) of an iPS cell colony cultured on each polypeptide according to the present invention.
Fig. 3B shows a morphic image (left side) and a magnified image (right side) of an iPS cell colony cultured on each polypeptide according to the present invention.
Fig. 3C shows a morphic image (left side) and a magnified image (right side) of an iPS cell colony cultured on each polypeptide according to the present invention.
Fig. 3D shows a morphic image (left side) and a magnified image (right side) of an iPS cell colony cultured on each polypeptide according to the present invention.
Fig. 3E shows a morphic image (left side) and a magnified image (right side) of an iPS cell colony cultured on each polypeptide according to the present invention.
Fig. 4A shows an image of iPS cells stained with DAPI (left side) and an image of iPS cells stained with NANOG (right side) that are captured in a case where the iPS cells are cultured on each polypeptide according to the present invention.
Fig. 4B shows an image of iPS cells stained with DAPI (left side) and an image of iPS cells stained with NANOG (right side) that are captured in a case where the iPS cells are cultured on each polypeptide according to the present invention.
Fig. 4C shows an image of iPS cells stained with DAPI (left side) and an image of iPS cells stained with NANOG (right side) that are captured in a case where the iPS cells are cultured on each polypeptide according to the present invention.
Fig. 4D shows an image of iPS cells stained with DAPI (left side) and an image of iPS cells stained with NANOG (right side) that are captured in a case where the iPS cells are cultured on each polypeptide according to the present invention.
Fig. 4E shows an image of iPS cells stained with DAPI (left side) and an image of iPS cells stained with NANOG (right side) that are captured in a case where the iPS cells are cultured on each polypeptide according to the present invention.

### DESCRIPTION OF THE PREFERRED EMBODIMENTS

Hereinafter, embodiments of the present invention will be specifically described.

### <Polypeptide>

The polypeptide according to the present invention is (a) a polypeptide having an amino acid sequence represented by SEQ ID NO: 1, (b) a polypeptide having an amino acid sequence, which shares identity of equal to or higher than 80% with the amino acid sequence represented by SEQ ID NO: 1, and having culture performance for pluripotent stem cells, or (c) a polypeptide having an amino acid sequence, which is formed by the deletion, substitution, or addition of one amino acid or several amino acids in SEQ ID NO: 1, and having culture performance for pluripotent stem cells.
SEQ ID NO: 1:

Herein, "having culture performance for pluripotent stem cells" means the polypeptide has a growth activity for pluripotent stem cells. Whether or not the polypeptide has the growth activity can be evaluated in the following method, for example. Onto a cell culture surface onto which the polypeptide is adsorbed, pluripotent stem cells are seeded at a cell density of 250 cells/well, the pluripotent stem cells are cultured for 8 days, and then nonadhesive cells are washed off with PBS. At this time, whether or not the number of adhesive cells present is equal to or greater than 2,500 cells/well (10 times the number of seeded cells) is checked to evaluate the growth activity.

Herein, the number of adhesive cells can be quantified by a method in which the activity of alkaline phosphatase expressed by the pluripotent stem cells is quantified, an MTT test, or the like.

The full-length polypeptide represented by SEQ ID NO: 1 constituted with 459 amino acid residues is vitronectin. In the present invention, vitronectin means human vitronectin. It has been confirmed that natural vitronectin is a sugar protein having a sugar chain in a portion of the sequence thereof.

Hereinafter, in the resent specification, the polypeptide according to the present invention will be referred to as a "polypeptide for culture" in some cases.

In the present specification, the term "step" includes not only an independent step, but also a step that cannot be clearly distinguished from other steps as long as the intended object thereof is achieved.

In the present specification, a range of numerical values represented by using "to" means a range which includes numerical values listed before and after "to" as a minimum value and a maximum value respectively.

In the present specification, in a case where there is a plurality of substances corresponding to each component in a composition, unless otherwise specified, the amount of each component in the composition means the total amount of the plurality of substances present in the composition.

In the specification of the present application, "one amino acid or several amino acids" means "1 to 5 amino acids".

In the present specification, "homogeneous" means a human being, and "heterogeneous" means an animal other than a human being.

In the present specification, an amino acid residue in an amino acid sequence is designated by one letter (for example, "G" for a glycine residue) or by three letters (for example, "Gly" for a glycine residue) in some cases as widely known in the field of the related art.

In the present invention, unless otherwise specified, "%" relating to an amino acid sequence of a polypeptide is based on the number of amino acid (or imino acid) residues.

In the present specification, the expression such as "the corresponding amino acid residue" used for a specific amino acid residue in an amino acid sequence means an amino acid residue in an amino acid sequence that is in the same position as a specific amino acid residue in another amino acid sequence as a standard when sequence alignment are performed on two or more contrasting amino acid sequences by a method known in the field of the related art in consideration of insertion, deletion, and substitution so as to maximize the number of amino acid residues identical to each other.

In the present specification, "identity" relating to an amino acid sequence can refer to a value calculated by using a BLAST package (see Ausubel et al., 1999 Short Protocols in Molecular Biology, 4th Ed - Chapter 18). For example, to share identity of equal to or higher than 80% with SEQ ID NO: 1 means that a value of Max. Identities in BLAST is equal to or greater than 80.

The polypeptide (b) is preferably a polypeptide having an amino acid sequence, which shares identity of equal to or higher than 90% (that is, 90% to 100%) with the amino acid sequence represented by SEQ ID NO: 1, and having culture performance for pluripotent stem cells, and more preferably a polypeptide having an amino acid sequence, which shares identity of equal to or higher than 95% (that is, 95% to 100%) with the amino acid sequence represented by SEQ ID NO: 1, and having culture performance for pluripotent stem cells.

The polypeptide (c) is preferably a polypeptide having an amino acid sequence, which is formed by the deletion, substitution, or addition of 1 to 5 amino acids in SEQ ID NO: 1, and having culture performance for pluripotent stem cells.

The polypeptide according to the present invention is preferably (a1) a polypeptide including an amino acid sequence represented by SEQ ID NO: 1, (b1) a polypeptide including an amino acid sequence, which shares identity of equal to or higher than 80% (that is, 80% to 100%) with the amino acid sequence represented by SEQ ID NO: 1, and having culture performance for pluripotent stem cells, or (c1) a polypeptide including an amino acid sequence, which is formed by the deletion, substitution, or addition of one amino acid or several amino acids in SEQ ID NO: 1, and having culture performance for pluripotent stem cells.

Herein, the polypeptide (b1) is preferably a polypeptide including an amino acid sequence, which shares identity of equal to or higher than 90% (that is, 90% to 100%) with the amino acid sequence represented by SEQ ID NO: 1, and having culture performance for pluripotent stem cells, and more preferably a polypeptide including an amino acid sequence, which shares identity of equal to or higher than 95% (that is, 95% to 100%) with the amino acid sequence represented by SEQ ID NO: 1, and having culture performance for pluripotent stem cells.

In addition, the polypeptide (c1) is preferably a polypeptide including an amino acid sequence, which is formed by the deletion, substitution, or addition of one amino acid or several amino acids and preferably 1 to 5 amino acids in SEQ ID NO: 1, and having culture performance for pluripotent stem cells.

For example, as the polypeptide according to the present invention, Vitronectin-XF (manufactured by Primorigen Biosciences) can be preferably used.

The polypeptide according to the present invention is preferably a polypeptide having an amino acid sequence formed by partially or completely deleting at least one of an amino acid sequence, which consists of the 1^{st} to 44^{th} amino acid residues in the amino acid sequence of human vitronectin, and an amino acid sequence, which consists of the 379^{th} to 449^{th} amino acid residues in the amino acid sequence of human vitronectin, from the amino acid sequence (SEQ ID NO: 1) of human vitronectin. The polypeptide according to the present invention is more preferably a polypeptide having an amino acid sequence formed by partially or completely deleting an amino acid sequence, which consists of the 1^{st} to 44^{th} amino acid residues in the amino acid sequence of human vitronectin, and an amino acid sequence, which consists of the 379^{th} to 449^{th} amino acid residues in the amino acid sequence of human vitronectin, from the amino acid sequence of human vitronectin. The polypeptide according to the present invention is even more preferably a polypeptide having an amino acid sequence formed by partially deleting an amino acid sequence, which consists of the 1^{st} to 44^{th} amino acid residues in the amino acid sequence of human vitronectin, and partially deleting an amino acid sequence, which consists of the 379^{th} to 449^{th} amino acid residues in the amino acid sequence of human vitronectin, from the amino acid sequence of human vitronectin.

Furthermore, the polypeptide according to the present invention is preferably "a polypeptide having an amino acid sequence which consists of the 62^{nd} to 478^{th} amino acid residues in the amino acid sequence of human vitronectin", "a polypeptide having an amino acid sequence, which shares identity of equal to or higher than 80% (that is, 80% to 100%) with the amino acid sequence consisting of the 62^{nd} to 478^{th} amino acid residues in the amino acid sequence of human vitronectin, and having culture performance for pluripotent stem cells", or "a polypeptide having an amino acid sequence, which is formed by the deletion, substitution, or addition of one amino acid or several amino acids in the amino acid sequence consisting of the 62^{nd} to 478^{th} amino acid residues in the amino acid sequence of human vitronectin, and having culture performance for pluripotent stem cells". The polypeptide according to the present invention is more preferably "a polypeptide consisting of an amino acid sequence which consists of the 62^{nd} to 478^{th} amino acid residues in the amino acid sequence of human vitronectin". For example, as the polypeptide according to the present invention, VTN-N (manufactured by Life Technologies) can be preferably used.

Another polypeptide according to the present invention is the following polypeptide (d).

(d) A polypeptide consisting of 40 to 450 amino acid residues and including (1) a first region including at least one amino acid sequence selected from the group consisting of an amino acid sequence represented by CSYYQSC (SEQ ID NO: 2) and an amino acid sequence represented by RGD and (2) a second region including (2-i) an amino acid sequence which is represented by PRPSLAKKQRFRHRNRKGYRSQRGHSRGRNQN (SEQ ID NO: 3), (2-ii) an amino acid sequence which shares identity of equal to or higher than 80% with an amino acid sequence represented by SEQ ID NO: 3 and exhibits adsorbability with respect to a cell culture surface of a support, or (2-iii) an amino acid sequence which is formed by the deletion, substitution, or addition of one amino acid residue or several amino acid residues in the amino acid sequence represented by SEQ ID NO: 3 and exhibits adsorbability with respect to the cell culture surface of the support.

In order to have the culture performance for pluripotent stem cells, the polypeptide for culture needs to exhibit "cell adhesiveness with respect to pluripotent stem cells" and "adsorbability with respect to a cell culture surface of a support". Herein, the support is a site of a culture vessel that has a surface to which the polypeptide according to the present invention is applied at the time of performing cell culture by using the culture method of the present invention.

In the polypeptide (d) according to the present invention, the first region including a predetermined amino acid sequence has excellent cell adhesiveness. Therefore, the polypeptide (d) enables cells, particularly, pluripotent stem cells to grow excellently. The polypeptide (d) according to the present invention having such an amino acid sequence enables pluripotent stem cells to grow for a long period of time while maintaining the undifferentiated state of the cells.

In addition, the second region including a predetermined sequence makes a contribution to the adsorbability with respect to the cell culture surface of the support. The polypeptide (d) according to the present invention having such an amino acid sequence exhibits excellent adhesiveness with respect to the cell culture surface of the support. Further, since the second region is included in the polypeptide together with the first region, the pluripotent stem cells can be grown for a long period of time while maintaining the undifferentiated state of the cells, without being exfoliated from the cell culture surface of the support for the duration of culture. Furthermore, the polypeptide (d) according to the present invention enables the pluripotent stem cells which are being cultured in the undifferentiated state to grow while inhibiting exfoliation of the cells from the cell culture surface of the support, and can improve the handleability in the culture operation.

As a result, it is possible to obtain a polypeptide which accelerates the growth of pluripotent stem cells in the undifferentiated state, does not require a treatment for fixing the polypeptide to the cell culture surface of the support through chemical bonding, and can be industrially produced.

In addition, the polypeptide (d) according to the present invention having the first and second regions can eliminate the risk of the intermixing of an antigenic substance and an infection source unlike the natural vitronectin, and can retain the performance equivalent to the performance of the natural human vitronectin, that is, the adhesiveness with respect to the pluripotent stem cells, the cell growth properties, and the undifferentiated state maintainability.

Furthermore, the pluripotent stem cells cultured in the presence of the polypeptide (d) according to the present invention having the first and second regions (preferably in the absence of a heterogeneous animal-derived component or the like) can substantially completely eliminate or significantly reduce a likelihood that a foreign substance such as an antigenic substance derived from a sample or the like may be mixed into the cells. Therefore, when the pluripotent stem cells cultured by the aforementioned culture method are used for medical purposes or for purposes equivalent to medical purposes, sufficient safety can be ensured.

Furthermore, according to the culture method using the polypeptide (d) of the present invention having the first and second regions, pluripotent stem cells can be cultured at lower cost by a simple operation. Therefore, the culture method can make a great contribution when being used for the medical purposes and in the field of research.

The first region includes at least one amino acid sequence selected from the group consisting of an amino acid sequence represented by SEQ ID NO: 2 and an RGD sequence.

The amino acid sequence represented by SEQ ID NO: 2 corresponds to seven amino acid residues consisting of the 25^{th} to 31^{st} amino acids residues in the amino acid sequence of vitronectin. Furthermore, the RGD sequence is a cell adhesive motif which corresponds to three amino acid residues consisting of the 45^{th} to 47^{th} amino acid residues in the amino acid sequence of vitronectin. All of these amino acid sequences are sequences positioned relatively on the N-terminal side of natural vitronectin. Presumably, these amino acid sequences may exhibit adhesiveness with respect to the undifferentiated pluripotent stem cells and thus enable the pluripotent stem cells kept undifferentiated to grow. Therefore, compared to a polypeptide including none of these amino acid sequences, a polypeptide including all of these amino acid sequences is excellent in the cell adhesiveness and has a better cell growth ability.

Two cysteine residues in the amino acid sequence represented by SEQ ID NO: 2 may be cross-linked with each other. In this way, a high-order structure is formed in the amino acid sequence represented by SEQ ID NO: 2, and the adhesiveness with respect to the pluripotent stem cells tend to be improved.

Here, "enabling the pluripotent stem cells to grow in an undifferentiated state" means that the pluripotent stem cells retains differentiation potency for the duration of culture. Whether or not the pluripotent stem cells are in an undifferentiated state can be evaluated by a known method. For example, it can be evaluated by the methods known to those in the related art, such as expression of molecular markers (measuring the expression of SSEA-4 and/or Oct-4 by means of flow cytometry, immunostaining by using Oct-4 and/or NANOG, and the like), checking the pluripotent differentiation by in-vitro experiment, and checking the formation of teratoma resulting from the transplantation of the cells into an immunodeficient mouse. Whether or not the pluripotent stem cells are growing should be checked through a common method by means of visual observation using various microscopes, by means of a technique using a test for reactivity such as ALP activity, flow cytometery, or the like, or by means of other techniques. In the present invention, the duration for which the pluripotent stem cells are cultured in a state of retaining the differentiation potency can be set to be, for example, one month, although the duration varies with the culture conditions and the state of the pluripotent stem cells.

The first region in the polypeptide for culture should include any one of the amino acid sequences selected from the group consisting of the amino acid sequence represented by SEQ ID NO: 2 and the RGD sequence. From the viewpoint of the cell adhesiveness and the cell growth properties, the first region in the polypeptide for culture preferably includes both the amino acid sequence represented by SEQ ID NO: 1 and the RGD sequence.

The first region may have an amino acid sequence other than the amino acid sequence represented by SEQ ID NO: 2 and the RGD sequence. From the viewpoint of the cell adhesiveness and the cell growth properties of the first region, examples of such an amino acid sequence include (1a) an amino acid sequence consisting of the 1^{st} to 24^{th} amino acid residues of the amino acid sequence of human vitronectin represented by SEQ ID NO: 1, (1b) an amino acid sequence consisting of the 48^{th} to 55^{th} amino acid residues in the amino acid sequence of human vitronectin represented by SEQ ID NO: 1, (1c) an amino acid sequence consisting of the 32^{nd} to 44^{th} amino acid residues in the amino acid sequence of human vitronectin represented by SEQ ID NO: 1, and a combination of these. Each of the amino acid sequences (1a) to (1c) may have an amino acid sequence in which one amino acid or several amino acids and preferably 1 to 5 amino acids are deleted, substituted, or added, within a range that does not impair the cell adhesiveness and the cell growth properties of the first region. Furthermore, each of the amino acid sequences (1a) to (1c) may have an amino acid sequence which shares identity of equal to or higher than 80% (that is, 80% to 100%), preferably equal to or higher than 90% (that is, 90% to 100%), and even more preferably equal to or higher than 95% (that is, 95% to 100%) with each of the amino acid sequences (1a) to (1c).

The first region may include at least one amino acid sequence selected from the group consisting of the amino acid sequences (1a) to (1c), in addition to the amino acid sequence represented by SEQ ID NO: 2 and the RGD sequence. From the viewpoint of the cell adhesiveness and the cell growth properties, it is preferable that the first region includes both the amino acid sequence represented by SEQ ID NO: 2 and the RGD sequence and further includes an amino acid sequence which consists of the 1^{st} to 55^{th} amino acid residues of the amino acid sequence represented by SEQ ID NO: 1, an amino acid sequence similar to the aforementioned amino acid sequence, or a partial amino acid sequence thereof. It is also preferable that the first region includes both the amino acid sequence represented by SEQ ID NO: 2 and the RGD sequence and further includes an amino acid sequence consisting of the 25^{th} to 47^{th} amino acid residues of the amino acid sequence represented by SEQ ID NO: 1, an amino acid sequence similar to the aforementioned amino acid sequence, or a partial amino acid sequence thereof.

Examples of amino acid sequences preferable for the first region include (1-i) an amino acid sequence which consists of the 1^{st} to 55^{th} amino acid residues in the amino acid sequence represented by SEQ ID NO: 1, (1-ii) an amino acid sequence which shares identity of equal to or higher than 80% (that is, 80% to 100%), preferably equal to or higher than 90% (that is, 90% to 100%), and more preferably equal to or higher than 95% (that is, 95% to 100%) with an amino acid sequence consisting of the amino acid sequence (1-i) and exhibits cell adhesion ability with respect to pluripotent stem cells, and (1-iii) an amino acid sequence which consists of an amino acid sequence formed by the deletion, substitution, or addition of one amino acid or several amino acids and preferably 1 to 5 amino acids in the amino acid sequence (1-i) and exhibits cell adhesion ability with respect to pluripotent stem cells.

From the viewpoint of the cell adhesiveness and the growth properties, the first region can consist of 3 to 60 amino acid residues and preferably can consist of 10 to 55 amino acid residues.

The second region may include an amino acid sequence consisting of 32 amino acid residues represented by SEQ ID NO: 3. From the viewpoint of ease of purifying the polypeptide for culture, the second region is preferably a polypeptide including the amino acid sequence represented by SEQ ID NO: 3. The amino acid sequence represented by SEQ ID NO: 3 is included in a portion of a hemopexin-like domain II positioned on the C-terminal side of the natural vitronectin and corresponds to a heparin binding domain consitituted with the 342^{nd} to 373^{rd} amino acid residues of the amino acid sequence represented by SEQ ID NO: 1. Hereinafter, the amino acid sequence represented by SEQ ID NO: 3 will be referred to as a heparin binding domain in some cases.

Presumably, the polypeptide (d) for culture may exhibit adsorbability with respect to a cell culture surface of a support because the polypeptide (d) has the heparin binding domain. For this reason, the polypeptide (d) for culture enables the undifferentiated pluripotent stem cells to grow for a long period of time while maintaining the undifferentiated state.

Furthermore, because the polypeptide (d) for culture includes the heparin binding domain, the hydrophilicity of the polypeptide for culture is ensured, and the hydrophobic aggregation of the polypeptide tends to be inhibited. As a result, it is easy to purify the polypeptide for culture, and the production efficiency can be improved.

Herein, "exhibiting adsorbability with respect to a cell culture surface of a support" means that the amino acid sequence can be physically adsorbed onto the cell culture surface of a target culture vessel (hereinafter, simply referred to as a "culture surface" in some cases) without chemically reacting with the culture surface. Whether or not the polypeptide exhibits adsorbability with respect to the cell culture surface of the support can be evaluated by the following method, for example. In the method, a solution containing the polypeptide is added in an amount of 200 pmol/cm² to a plasma-treated culture vessel made of polystyrene, and the culture vessel is left to stand for 2 hours at 37°C and then washed twice with a phosphate buffer solution. Thereafter, whether or not the amount of the polypeptide remaining on the surface of the culture dish is equal to or greater than 10 pmol/cm² is checked to evaluate the adsorbability of the polypeptide.

The amount of the polypeptide remaining on the surface of the culture dish can be measured by an Enzyme-Linked Immunosorbent Assay (ELISA) method in which the amount of the polypeptide binding to antibodies recognizing the polypeptide is determined or by a method in which the adsorbed polypeptide is hydrolyzed and the amount of the generated amino acid is determined by HPLC or the like.

The heparin binding domain may share identity of equal to or higher than 80% (that is, 80% to 100%) with the amino acid sequence represented by SEQ ID NO: 3. The identity may be preferably equal to or higher than 90% (that is, 90% to 100%), and more preferably equal to or higher than 95% (that is, 95% to 100%). Furthermore, the heparin binding domain may be an amino acid sequence which enables the pluripotent stem cells to grow in an undifferentiated state and exhibits adsorbability with respect to the cell culture surface of the support.

The heparin binding domain may include an amino acid sequence formed by the deletion, substitution, or addition of one amino acid residue or several amino acid residues and preferably 1 to 5 amino acid residues in the amino acid sequence represented by SEQ ID NO: 3. Furthermore, the heparin binding domain may be an amino acid sequence which exhibits adsorbability with respect to the cell culture surface of the support.

The polypeptide (d) for culture should include the first and second regions, and the relative position thereof is not particularly limited. In the polypeptide (d) for culture, the first region is preferably positioned on the N-terminal side of the second region.

The polypeptide (d) for culture having the first and second regions consists of 40 to 450 amino acid residues. If the number of the amino acid residues is equal to or greater than 40, the cell adhesiveness, the cell growth properties, or the adsorbability with respect to the cell culture surface of the support becomes excellent. In contrast, if the number of the amino acid residues is equal to or less than 450, the cell adhesiveness, the cell growth properties, and the adsorbability with respect to the cell culture surface of the support are further exhibited, and the protein molecules can be inhibited from being agglomerated, cross-linked, or aggregated. From the viewpoint of making it difficult for the protein molecules to be aggregated, the number of amino acid residues constituting the polypeptide (d) for culture is preferably equal to or greater than 80, more preferably equal to or greater than 90, and even more preferably equal to or greater than 100. Furthermore, the number of amino acid residues constituting the polypeptide (d) for culture is preferably equal to or less than 400, more preferably equal to or less than 250, even more preferably equal to or less than 170, and still more preferably equal to or less than 150. Any of the aforementioned upper limits may be combined with any of the aforementioned lower limits. For example, the polypeptide (d) for culture preferably consists of 40 to 400 amino acid residues, more preferably consists of 80 to 250 amino acid residues, even more preferably consists of 80 to 150 amino acid residues, and still more preferably consists of 100 to 150 amino acid residues.

From the viewpoint of preventing the hydrophobic aggregation, it is preferable that the polypeptide (d) for culture has a GRAVY value of -2.0 to -0.95. The GRAVY value (Kyte J., Doolittle R. F. (1982), J. Mol. Biol, 157: 105-132) represents the total average of a degree of hydrophobicity of the polypeptide. The greater the GRAVY value, the higher the degree of hydrophobicity. If the GRAVY value is equal to or less than -0.95, the occurrence of the hydrophobic aggregation tends to be easily inhibited. In contrast, if the GRAVY value is equal to or greater than -2.0, the polypeptide tend to be easily adsorbed onto the cell culture surface of the support, the undifferentiated cells tend to grow easily, and the adsorbability and the cell growth properties tend to be improved as the GRAVY value increases. In view of accomplishing both the inhibition of the aggregation and the adsorbability or the cell growth properties, the GRAVY value of the polypeptide is more preferably -1.70 to -0.975, and even more preferably -1.60 to -1.10. The smaller the number of the amino acid residues, the more the aggregation tends to occur. Therefore, in a case of a polypeptide consisting of 80 to 170 amino acid residues, in view of accomplishing both the inhibition of the aggregation and the adsorbability or the cell growth properties, the GRAVY value is preferably -1.70 to -0.975 and more preferably -1.60 to -1.10.

The GRAVY value can be adjusted by increasing or decreasing the proportion of a hydrophobic amino acid (for example, Trp, Tyr, Phe, Leu, Ile, Val, or Met) in the sequence or by increasing or decreasing the number of amino acid residues in the sequence.

It is preferable that the polypeptide (d) for culture further has an amino acid sequence other than the first and second regions, in addition to the first and second regions. From the viewpoint of sufficiently exhibiting the cell adhesiveness and the adsorbability with respect to the cell culture surface of the support, the polypeptide for culture preferably further includes the amino acid sequence represented by SEQ ID NO: 1, that is, a partial sequence of the amino acid sequence of human vitronectin. In this way, the polypeptide for culture can obtain properties close to the properties of the human vitronectin, for example, excellent adhesiveness and growth properties for the pluripotent stem cells.

From the viewpoint of the cell adhesiveness and the cell growth properties of the polypeptide for culture, the adsorbability with respect to the cell culture surface of the support, or the inhibition of aggregation, the partial amino acid sequence of the human vitronectin that may be include in the polypeptide (d) for culture preferably includes at least one region selected from the group consisting of the following third and fourth regions.
(3) A third region including an amino acid sequence selected from an amino acid sequence, which consists of the 56^{th} to 341^{st} amino acid residues in the amino acid sequence represented by SEQ ID NO: 1, and a partial amino acid sequence thereof; and
(4) a fourth region including an amino acid sequence selected from an amino acid sequence, which consists of the 374^{th} to 459^{th} amino acid residues in the amino acid sequence represented by SEQ ID NO: 1, and a partial amino acid sequence thereof.

As the third region, it is possible to select (3a) an amino acid sequence, which consists of the 56^{th} to 341^{st} amino acid residues in the amino acid sequence represented by SEQ ID NO: 1, or a partial amino acid sequence thereof, (3b) an amino acid sequence, which consists of the 269^{th} to 341^{st} amino acid residues in the amino acid sequence represented by SEQ ID NO: 1, or a partial amino acid sequence thereof, (3c) an amino acid sequence, which consists of the 274^{th} to 341^{st} amino acid residues in the amino acid sequence represented by SEQ ID NO: 1, or a partial amino acid sequence thereof, or (3d) an amino acid sequence, which consists of the 294^{th} to 341^{st} amino acid residues in the amino acid sequence represented by SEQ ID NO: 1, or a partial amino acid sequence thereof, because the above amino acid sequences tend to inhibit the hydrophobic aggregation at the time of preparing the polypeptide. With the amino acid sequences (3a) to (3d), the hydrophobic aggregation tends to be able to be mitigated by reducing the number of amino acid residues. It is particularly preferable to select the amino acid sequence (3d) because the hydrophobic aggregation tends to be able to be more reliably inhibited.

It is preferable that the polypeptide (d) further includes the third region consisting of one of the following amino acid sequences (3a-i) to (3a-iii).
(3a-i) An amino acid sequence, which consists of the 56^{th} to 341^{st} amino acid residues in the amino acid sequence represented by SEQ ID NO: 1, or a partial amino acid sequence thereof;
(3a-ii) an amino acid sequence which shares identity of equal to or higher than 80% (that is, 80% to 100%), preferably equal to or higher than 90% (that is, 90% to 100%), and more preferably equal to or higher than 95% (that is, 95% to 100%) with the amino acid sequence (3a-i) or a partial amino acid sequence thereof; and
(3a-iii) an amino acid sequence which is formed by the deletion, substitution, or addition of one amino acid or several amino acids and preferably 1 to 5 amino acids in the amino acid sequence (3a-i) or a partial amino acid sequence thereof.

It is also preferable that the polypeptide (d) further include a third region consisting of one of the following amino acid sequences (3b-i) to (3b-iii).
(3b-i) An amino acid sequence, which consists of the 269^{th} to 341^{st} amino acid residues in the amino acid sequence represented by SEQ ID NO: 1, or a partial amino acid sequence thereof;
(3b-ii) an amino acid sequence which shares identity of equal to or higher than 80% (that is, 80% to 100%), preferably equal to or higher than 90% (that is, 90% to 100%), and more preferably equal to or higher than 95% (that is, 95% to 100%) with the amino acid sequence (3b-i) or a partial amino acid sequence thereof; and
(3b-iii) an amino acid sequence which is formed by the deletion, substitution, or addition of one amino acid or several amino acids and preferably, 1 to 5 amino acids in the amino acid sequence (3b-i) or a partial amino acid sequence thereof.

From the viewpoint of the adsorbability with respect to the culture dish, for the fourth region, it is possible to select an amino acid sequence, which consists of the 374^{th} to 459^{th} amino acid residues in the amino acid sequence represented by SEQ ID NO: 1, or a partial amino acid sequence thereof, to select an amino acid sequence which consists of the 374^{th} to 409^{th} amino acid residues in the amino acid sequence represented by SEQ ID NO: 1, or a partial amino acid sequence thereof, or to select an amino acid sequence, which consists of the 374^{th} to 379^{th} amino acid residues in the amino acid sequence represented by SEQ ID NO: 1, or a partial amino acid sequence thereof.

Among these, an amino acid sequence, which consists of the 374^{th} to 379^{th} amino acid residues in the amino acid sequence represented by SEQ ID NO: 1, or a partial amino acid sequence thereof is preferable, because such an amino acid sequence makes it possible to obtain the adsorbability with respect to the culture dish and makes it easy to inhibit the hydrophobic aggregation at the time of preparing the polypeptide. By reducing the number of amino acids selected, the hydrophobic aggregation tends to be mitigated.

It is preferable that the polypeptide (d) further includes a fourth region consisting of one of the following amino acid sequences (4a-i) to (4a-iii).
(4a-i) An amino acid sequence, which consists of the 374^{th} to 459^{th} amino acid residues in the amino acid sequence represented by SEQ ID NO: 1, or a partial amino acid sequence thereof;
(4a-ii) an amino acid sequence which shares identity of equal to or higher than 80% (that is, 80% to 100%), preferably equal to or higher than 90% (that is, 90% to 100%), and more preferably equal to or higher than 95% (that is, 95% to 100%) with the amino acid sequence (4a-i) or a partial amino acid sequence thereof; and
(4a-iii) an amino acid sequence which is formed by the deletion, substitution, or addition of one amino acid or several amino acids, preferably, 1 to 5 amino acids in the amino acid sequence (4a-i) or a partial amino acid sequence thereof.

It is also preferable that the polypeptide (d) further includes a fourth region including one of the one of the following amino acid sequences (4b-i) to (4b-iii).
(4b-i) An amino acid sequence, which consists of the 374^{th} to 409^{th} amino acid residues in the amino acid sequence represented by SEQ ID NO: 1, or a partial amino acid sequence thereof;
(4b-ii) an amino acid sequence which shares identity of equal to or higher than 80% (that is, 80% to 100%), preferably equal to or higher than 90% (that is, 90% to 100%), and more preferably equal to or higher than 95% (that is, 95% to 100%) with the amino acid sequence (4b-i) or a partial amino acid sequence thereof; and
(4b-iii) an amino acid sequence which is formed by the deletion, substitution, or addition of one amino acid or several amino acids, preferably, 1 to 5 amino acids in the amino acid sequence (4b-i) or a partial amino acid sequence thereof.

In addition, it is also preferable that the polypeptide (d) further include the fourth region including one of the following amino acid sequences (4c-i) to (4c-iii).
(4c-i) An amino acid sequence, which consists of the 374^{th} to 379^{th} amino acid residues in the amino acid sequence represented by SEQ ID NO: 1, or a partial amino acid sequence thereof;
(4c-ii) an amino acid sequence which shares identity of equal to or higher than 80% (that is, 80% to 100%), preferably equal to or higher than 90% (that is, 90% to 100%), and more preferably equal to or higher than 95% (that is, 95% to 100%) with the amino acid sequence (4c-i) or a partial amino acid sequence thereof; and
(4c-iii) an amino acid sequence which is formed by the deletion, substitution, or addition of one amino acid or several amino acids, preferably, 1 to 5 amino acids in the amino acid sequence (4c-i) or a partial amino acid sequence thereof.

The partial amino acid sequence of the amino acid sequence constituting the third and fourth regions means an amino acid sequence constituted with three or more consecutive amino acid residues among a predetermined range of amino acid residues. The number of amino acid residues of the partial amino acid sequence should be selected within a range that does not exceed the aforementioned total number of amino acid residues of the polypeptide (d) for culture.

By including the third region, the polypeptide (d) for culture tends to obtain an advantage of improving the adsorbability with respect to the culture dish. Furthermore, by including the fourth region, the polypeptide for culture tends to obtain an advantage of further improving the adsorbability with respect to the culture dish. The polypeptide for culture may have one of the third and fourth regions.

The GRAVY value of the polypeptide (d) for culture is preferably adjusted by increasing or decreasing the number of amino acid residues in the amino acid sequences constituting the third and fourth regions or by the substitution, deletion, addition, or the like of the amino acid residues, because then the GRAVY value can be easily adjusted. Particularly, the GRAVY value of the polypeptide (d) for culture is more preferably adjusted by adjusting the length of the amino acid sequence constituting the third region.

Among the amino acid residues constituting the amino acid sequence represented by SEQ ID NO: 1, the 56^{th} to 131^{st} amino acid residues, the 56^{th} to 268^{th} amino acid residues, the 269^{th} to 273^{rd} amino acid residue, or the 50^{th} to 293^{rd} amino acid residues may not be included in the polypeptide (d) for culture. Presumably, an amino acid sequence consisting of the above amino acid residues may not make a contribution to the performance of the polypeptide (d) for culture with respect to the pluripotent stem cells. Therefore, a sequence suitable for the adsorption of the polypeptide onto the culture dish is selected.

In a case where the third region includes an amino acid residue corresponding to a cysteine residue of the sequence represented by SEQ ID NO: 1, the third region may have an amino acid residue other than the cysteine residue in the position of the cysteine residue. It is preferable that the third region has an amino acid residue other than the cysteine residue because then intramolecular cross-linking or intermolecular cross-linking caused by the cysteine residue can be prevented. The amino acid residue substituting the cysteine reisdue is not particularly limited, and preferred examples thereof include a serine residue, an alanine residue, a glycine residue, and the like. Among these, a serine residue or an alanine residue are preferable because these have a structure similar to that of cysteine.

The polypeptide for culture may have any additional amino acid residues other than the aforementioned amino acid residues within a range that does not impair the cell adhesiveness and the adsorbability with respect to the cell culture surface of the support. Examples of the sequence consisting of any other amino acid residues described above include an additional sequence added for easily preparing the polypeptide for culture by a recombination technique. Examples of the additional sequence include a methionine residue on the N-terminal side, a GPLG sequence on the N-terminal side, a tag sequence (for example, glutathione S-transferase (GST), a FLAG tag, or a His tag), a linker sequence (for example, GGGS, GGGGS, or GGGGGS) which can be added so as to be positioned between the respective regions, and the like.

The polypeptide for culture can be manufactured by an amino acid synthesis technique or a gene recombination technique known to those in the related art.

Specifically, in a case where the polypeptide for culture of the present invention is obtained by the gene recombination technique, first, a gene encoding a target amino acid sequence is obtained, and the obtained gene is incorporated into an expression vector, thereby preparing a recombinant expression vector. Thereafter, by introducing the obtained recombinant expression vector into an appropriate host, a transformant is prepared. By culturing the obtained transformant in an appropriate medium, an intended polypeptide is produced. Therefore, by collecting the intended polypeptide from the culture by a common method, the polypeptide according to the present invention can be obtained.

From the viewpoint of the cell growth properties and the ability to grow the undifferentiated pluripotent stem cells in the undifferentiated state, and the like, the polypeptide for culture is preferably a polypeptide (A) which consists of 80 to 450 amino acid residues and includes (1) a first region consisting of an amino acid sequence consisting of the 25^{th} to 47^{th} amino acid residues of the amino acid sequence represented by SEQ ID NO: 1, (2) a second region, which consists of an amino acid sequence consisting of the 342^{nd} to 373^{rd} amino acid residues of the amino acid sequence represented by SEQ ID NO: 1, and at least one region selected from the group consisting of the following third and fourth regions: (3) a third region consisting of an amino acid sequence, which consists of the 269^{th} to 341^{st} amino acid residues of the amino acid sequence represented by SEQ ID NO: 1, or a partial amino acid sequence thereof, and (4) a fourth region consisting of an amino acid sequence, which consists of the 374^{th} to 459^{th} amino acid residues of the amino acid sequence represented by SEQ ID NO: 1, or a partial amino acid sequence thereof.

Furthermore, from the viewpoint of the cell growth properties, the ability to grow the undifferentiated pluripotent stem cells in the undifferentiated state, and the like, the polypeptide for culture is preferably a polypeptide (B) which consists of 100 to 450 amino acid residues and includes (1) a first region consisting of an amino acid sequence (including the amino acid sequence represented by SEQ ID NO: 2 and the RGD sequence) consisting of the 1^{st} to 55^{th} amino acid residues of the amino acid sequence represented by SEQ ID NO: 1, (2) a second region (heparin binding domain) consisting of an amino acid sequence, which consists of the 342^{nd} to 373^{rd} amino acid residues of the amino acid sequence represented by SEQ ID NO: 3, and at least one region selected from the group consisting of the following third and fourth regions: (3) a third region consisting of an amino acid sequence, which consists of the 269^{th} to 341^{st} amino acid residues of the amino acid sequence represented by SEQ ID NO: 1, or a partial amino acid sequence thereof, and (4) a fourth region consisting of an amino acid sequence, which consists of the 374^{th} to 459^{th} amino acid residues of the amino acid sequence represented by SEQ ID NO: 1, and a partial amino acid sequence thereof.

The polypeptide (A) or (B) is preferably a polypeptide having a GRAVY value of -2.0 to -0.95.

The polypeptide (A) preferably consists of 80 to 250 amino acid residues.

The polypeptide (A) is more preferably a polypeptide which has a GRAVY value of -2.0 to -0.95 and consists of 80 to 250 amino acid residues.

The polypeptide (A) is even more preferably a polypeptide which has a GRAVY value of -1.70 to -0.975 and consists of 80 to 250 amino acid residues.

The polypeptide (A) or (B) preferably consists of 100 to 250 amino acid residues.

The polypeptide (A) or (B) is more preferably a polypeptide which has a GRAVY value of -2.0 to -0.95 and consists of 100 to 250 amino acid residues.

The polypeptide (A) or (B) is even more preferably a polypeptide which has a GRAVY value of -1.70 to -0.975 and consists of 100 to 250 amino acid residues.

The polypeptide (A) or (B) is still more preferably a polypeptide which has a GRAVY value of -1.70 to -0.975 and consists of 100 to 170 amino acid residues.

Examples of the polypeptide for culture are shown below, but the present invention is not limited thereto.

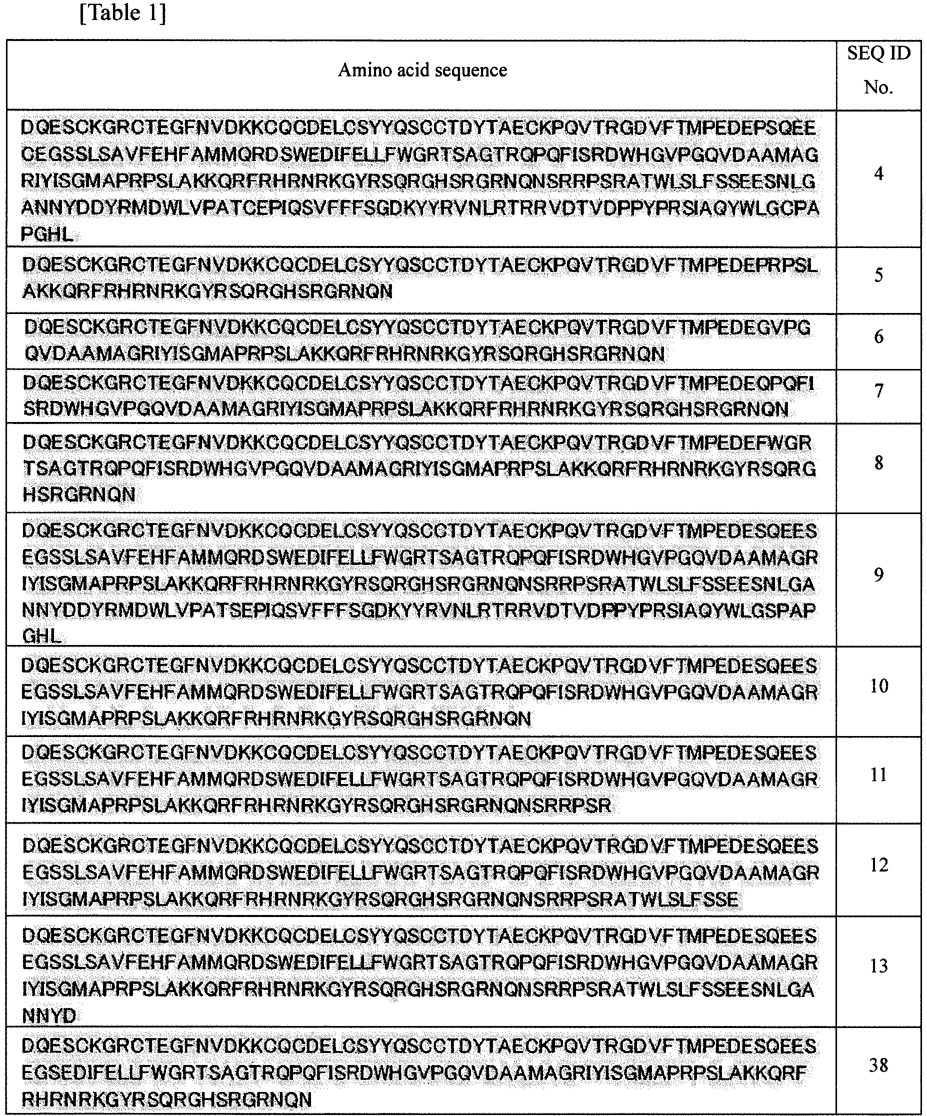

Examples of polypeptides preferred as the polypeptide according to the present invention include (d1) a polypeptide having an amino acid sequence which is represented by one of SEQ ID NO: 4 to SEQ ID NO: 23, SEQ ID NO: 38, and SEQ ID NO: 39, (d2) a polypeptide having an amino acid sequence, which shares identity of equal to or higher than 80% (that is, 80% to 100%), more preferably equal to or higher than 90% (that is, 90% to 100%), and even more preferably equal to or higher than 95% (that is, 95% to 100%) with the amino acid sequence represented by one of SEQ ID NO: 4 to SEQ ID NO: 23, SEQ ID NO: 38, and SEQ ID NO: 39, and having culture performance for pluripotent stem cells, and (d3) a polypeptide having an amino acid sequence, which is formed by the deletion, substitution, or addition of one amino acid or several amino acids, preferably, 1 to 5 amino acids in the amino acid sequence represented by one of SEQ ID NO: 4 to SEQ ID NO: 23, SEQ ID NO: 38, and SEQ ID NO: 39, and having culture performance for pluripotent stem cells.

The polypeptide according to the present invention is more preferably (d4) a polypeptide consisting of an amino acid sequence which is represented by one of SEQ ID NO: 4 to SEQ ID NO: 23, SEQ ID NO: 38, and SEQ ID NO: 39, (d5) a polypeptide consisting of an amino acid sequence, which shares identity of equal to or higher than 80% (that is, 80% to 100%), more preferably equal to or higher than 90% (that is, 90% to 100%), and even more preferably equal to or higher than 95% (that is, 95% to 100%) with the amino acid sequence represented by one of SEQ ID NO: 4 to SEQ ID NO: 23, SEQ ID NO: 38, and SEQ ID NO: 39, and having culture performance for pluripotent stem cells, or (d6) a polypeptide consisting of an amino acid sequence, which is formed by the deletion, substitution, or addition of one amino acid or several amino acids, preferably, 1 to 5 amino acids in the amino acid sequence represented by one of SEQ ID NO: 4 to SEQ ID NO: 23, SEQ ID NO: 38, and SEQ ID NO: 39, and having culture performance for pluripotent stem cells.

### <Culture method for pluripotent stem cells>

The culture method for pluripotent stem cells of the present invention includes obtaining a cell culture surface coated with the polypeptide for culture by applying the polypeptide according to the present invention to a cell culture surface of a support (hereinafter, referred to as a culture surface preparation step), and culturing pluripotent stem cells by seeding the pluripotent stem cells onto the culture surface coated with the polypeptide for culture by using a medium in which the content of an ascorbic acid derivative is equal to or greater than 1.5 mmol/L (mM) and equal to or less than 100 mmol/L (mM) (hereinafter, referred to as a culture step).

In the culture method of the present invention, for the culture of pluripotent stem cells, a polypeptide having an amino acid sequence of vitronetin or an amino acid sequence derived from vitronectin is combined with a medium in which the content of an ascorbic acid derivative is equal to or greater, than 1.5 mmol/L (mM) and equal to or less than 100 mmol/L (mM). In this way, the pluripotent stem cells can more efficiently grow.

The pluripotent stem cells according to the present invention are pluripotent stem cells of an animal that belongs to primates. Specifically, the pluripotent stem cells include embryonic stem cells (ES cells), induced pluripotent stem cells (iPS cells), somatic stem cells.. As the pluripotent stem cells, one kind of these cells may be used singly, or if necessary, two or more kinds thereof may be used by being mixed together. The iPS cells include the cells described in Nature, 2007, July 19; Vol. 448, pp. 313-317; Cell, 2006, August 25; Vol. 126(4), pp. 663-676 and cells similar to the above cells.

Examples of the pluripotent stem cells preferably used in the present invention include iPS cells.

Examples of the animal that belongs to primates include a human being, a monkey, a gorilla, and the like. The animal that belongs to primates is preferably a human being congeneric with the polypeptide for culture. As long as a component or a substance used in the present invention is a component or a substance derived from an animal that belongs to primates, it can be preferably used in the present invention as a component or a substance derived from a homogeneous animal.

In the medium for use in the present invention, the content of an ascorbic acid derivative is equal to or greater than 1.5 mmol/L (mM) and equal to or less than 100 mmol/L (mM). In a case where the medium contains two or more kinds of ascorbic acid derivatives, the aforementioned content of the ascorbic acid refers to the total content of the entire ascorbic acid derivatives contained in the medium. The medium for use in the present invention can be preferably used as a medium for culture of pluripotent stem cells.

The ascorbic acid derivative is not particularly limited. However, the ascorbic acid derivative is preferably one kind of ascorbic acid derivative or two or more kinds of ascorbic acid derivatives selected from the group consisting of ascorbic acid, magnesium ascorbyl phosphate, sodium ascorbyl phosphate, aminopropyl ascorbyl phosphate, disodium ascorbyl sulfate, and ascorbyl 2-phosphoric acid ester, and more preferably magnesium ascorbyl phosphate.

The content of the ascorbic acid derivative in the medium for use in the present invention is equal to or greater than 1.5 mmol/L, preferably equal to or greater than 1.6 mmol/L, more preferably equal to or greater than 3.0 mmol/L, even more preferably equal to or greater than 3.5 mmol/L, and still more preferably equal to or greater than 3.7 mmol/L. The upper limit of the content is not particularly limited. However, the upper limit of the content of the ascorbic acid derivative is preferably equal to or less than 100 mmol/L, more preferably equal to or less than 80 mmol/L, and even more preferably equal to or less than 50 mmol/L.

Any of the values of the upper limit may be combined with any of the values of the lower limit. For example, the content of the ascorbic acid derivative is preferably 1.5 mmol/L to 100 mmol/L, more preferably 1.6 mmol/L to 80 mmol/L, even more preferably 3.0 mmol/L to 80 mmol/L, and still more preferably 3.5 mmol/L to 50 mmol/L.

In addition to the ascorbic acid derivative, various components that can be generally added for culture of pluripotent stem cells may be added to the medium for use in the present invention. Such components can be appropriately selected according to the type of cells to be cultured. For example, as the components other than the ascorbic acid derivative, glucose, fetal bovine serum (FBS), human serum, or antibiotics (penicillin, streptomycin, and the like) may be added. In a case where serum is added to the medium, the concentration thereof can be appropriately changed according to the state of culture at that time. However, generally, the concentration of serum can be 10% (v/v).

The medium for use in the present invention is preferably a medium containing, as the components other than the ascorbic acid derivative, water, a salt (an inorganic salt such as sodium, potassium, magnesium, or calcium and an organic salt such as sodium pyruvate), an amino acid (an essential amino acid and a nonessential amino acid), vitamin (L-ascorbic acid, riboflavin, biotin, cyanocobalamin, or the like), a trace elements (selenium, iron, zinc, copper, or the like), a carbon source (D-glucose or the like), FGF (basic fibroblast growth factor FGF-2 or the like), TGF-β, insulin, and trensferrin. From the viewpoint of efficiently growing the pluripotent stem cells in an undifferentiated state, a medium containing FGF is preferable, and a medium containing FGF and TGF-β is more preferable.

The medium for use in the present invention can also be prepared by using a known medium such as a commercially available medium and adding an ascorbic acid derivative thereto such that the content of the ascorbic acid derivative according to the present invention is achieved. Examples of commercially available media that can be used for preparing the medium for use in the present invention include Essential 8, DMEM, MEM, F 12, DME, RPMI 1640, MCDB 104 and 199, MCDB 153, L 15, SkBM, a Basal medium, and the like. Among these, Essential 8 is preferable.

In the culture method of the present invention, it is preferable that the pluripotent stem cells are cultured in the absence of a heterogeneous animal-derived component. In this way, a likelihood of the intermixing of a heterogeneous animal-derived foreign substance can be eliminated with high accuracy. Examples of the culture of pluripotent stem cells in the absence of a heterogeneous cell-derived component include the culture using a medium not containing a heterogeneous animal-derived component, the culture not using a heterogeneous animal-derived feeder cells, and the like.

Furthermore, in the culture method of the present invention, it is preferable that the pluripotent stem cells are cultured in the absence of a heterogeneous animal-derived component and a serum component. In this way, the intermixing of a heterogeneous animal-derived component can be more reliably prevented.

The medium for use in the present invention may also be prepared by using, as a medium not containing the heterogeneous anima-derived component, a medium mixture composed of a hypoosmotic medium containing at least one kind of medium component such as a nonessential amino acid, glutamic acid, β-mercaptoethanol, FGF-2, TGF-β, insulin, or transferrin, and adding an ascorbic acid derivative thereto such that the content of an ascorbic acid derivative of the present invention is achieved. Specifically, the medium for use in the present invention may be prepared by using a medium such as TeSR2 (Stemcell Technologies Inc) and adding an ascorbic acid derivative thereto such that the content of an ascorbic acid derivative of the present invention is achieved. However, the present invention is not limited to the above method.

The cells are cultured in an incubator under general culture conditions, for example, a temperature of 37°C and a CO₂ concentration of 5% (v/v).

In the culture method and subculture method for pluripotent stem cells, the medium for use in the present invention may be prepared by using a general medium, which is used for retaining pluripotent stem cells, and adding an ascorbic acid derivative thereto such that the content of an ascorbic acid derivative of the present invention is achieved. Specifically, for example, the medium for use in the present invention may be prepared by using mTeSR, TeSR2 (Stemcell Technologies Inc), or the like and adding an ascorbic acid derivative thereto such that the content of an ascorbic acid of the present invention is achieved. The pluripotent stem cells are seeded into the medium by a common method. Herein, it is not necessary to use the same medium for a series of passages, and as long as the pluripotent stem cells can be kept undifferentiated, different media may be used.

In the culture surface preparation step, the culture surface coated with the polypeptide for culture is prepared by applying a coating solution, which contains the polypeptide for culture in a predetermined amount, to a culture surface of a support. In this way, the culture surface can be coated with the polypeptide for culture.

The content of the polypeptide for culture in the coating solution varies with the type and the size of the culture surface to be coated. However, from the viewpoint of the adsorbability with respect to the culture surface, the content of the polypeptide for culture is preferably 1 pmol/cm² to 1,000 pmol/cm², and more preferably 100 pmol/cm² to 300 pmol/cm². An aqueous medium used for preparing the coating solution is not particularly limited, and examples thereof include a phosphate buffer solution, a tris-buffer solution, ultrapure water, and the like.

In the coating operation, after being applied, the coating solution is held as it is for a certain period of time, for example, for about 30 minutes to 24 hours. In this way, the culture surface can be coated with the polypeptide for culture without the need to perform a special treatment.

The culture step includes culturing pluripotent stem cells by seeding the pluripotent stem cells onto the culture surface coated with the polypeptide for culture.

The seeding density and the culture conditions of the pluripotent stem cells are not particularly limited, and generally used conditions can be used as they are. For example, the cells may be seeded at a seeding density of about 1 × 10³ cells/cm² to 1 × 10⁵ cells/cm² and cultured under the aforementioned culture and subculture conditions. Furthermore, a cell mass with a diameter of 10 µm to 100 µm may be seeded at a seeding density of about 1 cell/cm² to 5 cells/cm² and cultured under the aforementioned culture and subculture conditions.

In this way, it is possible to excellently grow the pluripotent stem cells with excellent handleability on the polypeptide. Furthermore, in a case where the polypeptide (d) is used, it is possible to excellently grow the pluripotent stem cells while maintaining the undifferentiated state.

### <Culture vessel>

In the present invention, a culture vessel means a vessel having a support which has a surface used for cell culture. As the support, those known as a support for cell culture in the related art can be used as they are. Examples of the support may include plastic (for example, polystyrene, an acrylonitrile-butadiene-styrene resin, a polycarbonate resin, and a polyester resin), glass, filter with fine pores (for example, cellulose, nylon, glass fiber, polyester, and polycarbonate), a material for a bioreactor (may include hollow fiber tubes or microcarrier beads) used in batch cell culture, continuous cell culture, or genetic engineering (for example, a bioreactor), polyethylene terephthalate, Teflon (registered trademark), ceramic and polymer materials relating thereto, and the like.

In addition, the aforementioned support may be a support of which the culture surface is coated with a plasma-polymerized thin film.

The shape of the support is not particularly limited, and the support may have any shape as long as it is applicable to the culture of pluripotent stem cells. Examples of vessels with such a shape include a multi-well plate (for example, a 6-well plate, a 12-well plate, a 24-well plate, and a 96-well plate), a culture dish (for example, a petri dish), a tube, a culture flask, a roller bottle, a flask for shake culture, and the like.

The culture vessel according to the present disclosure includes a support having a cell culture surface and the polypeptide for culture disposed on the cell culture surface of the support.

The culture vessel of the present disclosure has the culture surface including the aforementioned polypeptide for culture according to the present invention. Therefore, the polypeptide for culture is excellently adsorbed onto the culture surface, and in a case where pluripotent stem cells are seeded onto the polypeptide for culture, the pluripotent stem cells with excellent handleability can grow in a state of being kept undifferentiated.

Herein, the culture surface in the culture vessel means a surface to which cells can adhere at the time of seeding and growing the cells.

The culture vessel according to the present disclosure can be manufactured by a manufacturing method including preparing a vessel with a support having a cell culture surface (hereinafter, referred to as a "preparation step") and performing an adsorption treatment on the cell culture surface by applying the polypeptide for culture thereto (hereinafter, referred to as a "adsorption treatment step"). In this way, the culture vessel according to the present disclosure can be obtained in a simple manner.

In the preparation step, the culture vessel with the support having the culture surface is prepared. In a case where the support has a plasma-polymerized thin film on the culture surface, the preparation step may include a step of forming the plasma-polymerized thin film on the support. As the method for forming the plasma-polymerized thin film, a common method may be used as it is.

The adsorption treatment step includes applying the polypeptide for culture according to the present invention to the culture surface and holding the culture surface as it is. In the adsorption treatment step, the polypeptide for culture should be adsorbed onto the culture surface by preparing an adsorbent solution containing the polypeptide for culture in a predetermined amount, applying the adsorbent solution to the culture surface, and holding the culture surface as it is for a predetermined time.

In the adsorption treatment step, the details described in the step of preparing the culture surface coated with the polypeptide for culture in the culture method can be applied as they are.

### <Kit>

The kit for culture of pluripotent stem cells of the present invention is a kit including (a) a polypeptide having an amino acid sequence represented by SEQ ID NO: 1, (b) a polypeptide having an amino acid sequence, which shares identity of equal to or higher than 80% with the polypeptide represented by SEQ ID NO: 1, and having culture performance for pluripotent stem cells, or (c) a polypeptide having an amino acid sequence, which is formed by the deletion, substitution, or addition of one amino acid or several amino acids (preferably 1 to 5 amino acids) in SEQ ID NO: 1, and having culture performance for pluripotent stem cells and a medium in which the content of an ascorbic acid derivative is equal to or greater than 1.5 mmol/L (mM) and equal to or less than 100 mmol/L (mM).

Herein, the preferred ranges of all of the polypeptides (a) to (c), the type of the ascorbic acid derivative, the content of the ascorbic acid derivative, and other components of the medium are as described above.

Furthermore, another kit for culture of pluripotent stem cells of the present invention is a kit including a polypeptide and a medium in which the content of an ascorbic acid derivative is equal to or greater than 1.5 mmol/L (mM) and equal to or less than 100 mmol/L (mM), in which the polypeptide is (d) a polypeptide consisting of 40 to 450 amino acid residues and including (1) a first region including at least one amino acid sequence selected from the group consisting of an amino acid sequence represented by CSYYQSC (SEQ ID NO: 2) and an amino acid sequence represented by RGD and (2) a second region including (2-i) an amino acid sequence which is represented by PRPSLAKKQRFRHRNRKGYRSQRGHSRGRNQN (SEQ ID NO: 3), (2-ii) an amino acid sequence which shares identity of equal to or higher than 80% with the amino acid sequence represented by SEQ ID NO: 3 and exhibits adsorbability with respect to a cell culture surface of a support, or (2-iii) an amino acid sequence which is formed by the deletion, substitution, or addition of one amino acid residue or several amino acid residues (preferably 1 to 5 amino acid residues) in the amino acid sequence represented by SEQ ID NO: 3 and exhibits adsorbability with respect to the cell culture surface of the support.

Herein, the preferred ranges of all of the polypeptide (d), the type of the ascorbic acid derivative, the content of the ascorbic acid derivative, and other components of the medium are as described above.

The medium of the present disclosure is a medium in which the content of an ascorbic acid derivative is equal to or greater than 1.5 mmol/L (mM).

Herein, the preferred ranges of all of the type of the ascorbic acid derivative, the content of the ascorbic acid derivative, and other components of the medium are as described above.

### Examples

Hereinafter, the present invention will be specifically described based on examples, but the present invention is not limited thereto. Herein, unless otherwise specified, "%" is based on mass.

### [Reference example 1]

### <Preparation of polypeptide>

By a common method using PCR, gene sequences encoding each of the polypeptides RCP-1 to RCP-17 having the amino acid sequences shown in Tables 2 and 3 were amplified. Herein, RCP-11 corresponds to the sequence of natural human vitronectin. In Tables 2 and 3, the column of "NOTE" shows the position in the amino acid sequence (SEQ ID NO: 1) of natural human vitronectin corresponding to the amino acid sequence of each of the polypeptides. Here, in some cases, the amino acid sequence of each of the polypeptides include an amino acid sequence which is formed by the addition, deletion, or substitution occurring in the amino acid sequence of the natural human vitronectin within a range corresponding described in the tables. The amino acid sequences of RCP-1 to RCP-10 and RCP-17 are the same as each other, except that methionine is on the N-terminal in each of the amino acid sequences represented by SEQ ID NO: 4 to SEQ ID NO: 13 and SEQ ID NO: 38.

For RCP-1 to RCP-10 and RCP-17, target genes were inserted into pET-28b(+), which was cleaved in advance by being treated with Ncol (TAKARA BIO INC.), by using an InFusion Advantage PCR Cloning Kit (Clontech Laboratories, Inc), thereby constructing the respective expression vectors. For RCP-11 to RCP-16, target genes were inserted into pGEX-6P-1 (GE Healthcare Corporation), which was cleaved in advance by being treated with BamHI (TAKARA BIO INC.), in the same manner as described above, thereby constructing the respective expression vectors. The sequences of the expression vectors were checked by sequence analysis.

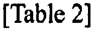

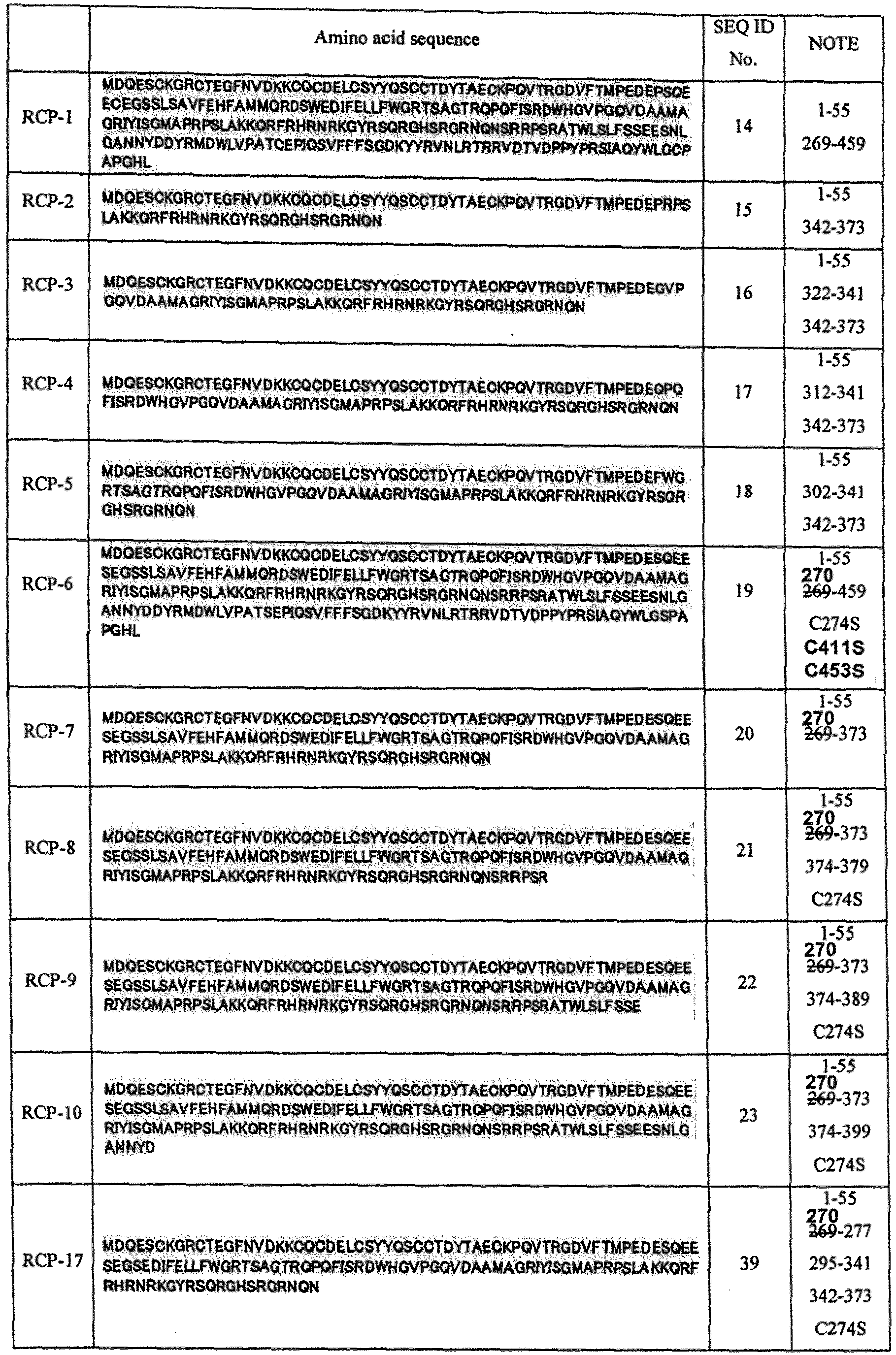

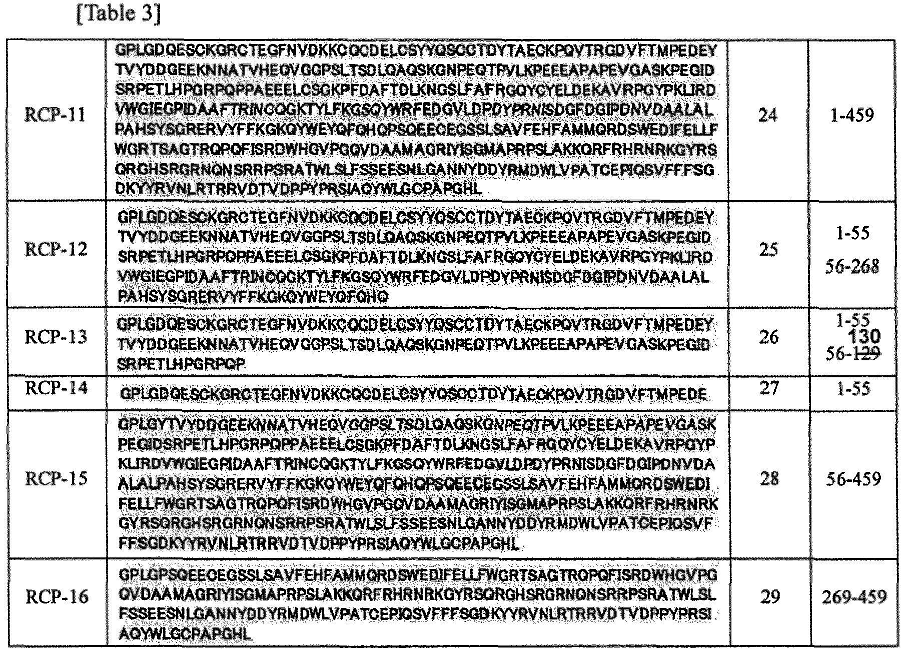

The prepared expression vectors of RCP-1 to RCP-10 and RCP-17 were transformed into BL21(DE3)pLysS (Novagen) by a common method, applied to a kanamycin-containing LB plate, and incubated for 16 hours at 37°C. By a colony direct PCR method, the state where the vectors were introduced into the cells was checked. Thereafter, 1 mM IPTG (Wako Pure Chemical Industries, Ltd.) was added thereto, and the cells were cultured by being shaken for 5 hours at 37°C, thereby inducing the expression of the polypeptides.

The bacterial cells were collected through a centrifugal treatment and resuspended in a washing buffer (20 mM Tris, 150 mM NaCl, pH 7.6). Through sonication, the bacterial cells were fragmented and then subjected to centrifugation for 30 minutes at 4°C and 15,000 rpm, and an insoluble fraction was collected. The bacterial cells were washed with a washing buffer containing 0.5% by mass of Triton ×100, then resuspended in a low-concentration urea buffer (Low Urea Buffer: 20 mM Tris, 150 mM NaCl, 2 M urea, pH 7.6), and subjected to a sonication treatment. Through a centrifugation treatment, an insoluble fraction was collected, a high-concentration urea buffer (High Urea Buffer: 20 mM Tris, 150 mM NaCl, 8 M urea, pH 7.6) was then added thereto, and the insoluble fraction was solubilized through a sonification treatment.

The solution obtained by the method described above that contained a target peptide was purified by using AKTA Explorer 100 (GE Healthcare Corporation) and HiTrap Heparin HP 5 ml (GE Healthcare Corporation). By performing stepwise elution using a high-concentration urea buffer as a binding buffer and a high-salt concentration adjusting buffer (20 mM Tris, 1 M NaCl, 8 M urea, pH 7.6) as an elution buffer, the target polypeptide was purified.

The expression vectors of RCP-11 to RCP-16 prepared as above were transformed into BL21 (Novagen) by a common method, applied to an ampicillin-containing LB plate, and incubated for 16 hours at 37°C. By a colony direct PCR method, the state where the vectors were introduced into the cells was checked. Thereafter, 100 µM isopropyl-β-D-thiogalactopyranoside (IPTG) was added thereto, and the cells were cultured by being shaken for 24 hours at 20°C, thereby inducing the expression of the polypeptides.

The bacterial cells were collected, resuspended in a B-PER (registered trademark) Bacterial Protein Extraction Reagent in Phosphate Buffer (Thermo Fisher Scientific Inc.), and then fragmented through sonication. By performing centrifugation for 30 minutes at 4°C and 15,000 rpm, an insoluble fraction was removed, and the supernatant was purified by using AKTA Explorer 100 and GSTrap HP 5 ml ×2 (GE Healthcare Corporation). The eluted fraction was desalted by using Hiprep 26/10 Desalting (GE Healthcare Corporation). Furthermore, a protease (PreScission Protease) for cleaving a GST fusion protein was added thereto in a solution amount of 1/2,000, and the resultant was incubated for 24 hours at 4°C, thereby cleaving the GST tag. The resultant was purified again by using GSTrap HP 5 ml ×2, and the cleaved GST tag was removed by being adsorbed onto a column. The fraction passing through the column was dialyzed using Slide-A-Lizer (3.5 K MWCO.: Thermo Fisher Scientific Inc., the same device will be used hereinafter) and substituted with PBS.

The polypeptide of RCP-1 obtained as above was subjected to electrophoresis by using Ready Gel (12.5%, Bio-Rad Laboratories, Inc.) and stained with a GelCode™ Blue Stain Reagent (Thermo Scientific). As a result, a single band could be confirmed at a site corresponding to a molecular weight of 28.3 kDa expected from the amino acid sequence. The same results were obtained from other polypeptides.

For RCP-1 to RCP-10 and RCP-17, each of the purified polypeptide solutions was dialyzed using Slide-A-Lizer (3.5 K MWCO.). Basically, by using a dialysis buffer (PBS, 1.5 M NaCl, 0.5 M L-arginine, 1 mM EDTA, pH 7.4) as an outer dialysate, urea was removed by stepwise dialysis. The concentration of the end-product of dialysis was calculated from the absorbence at 280 nm by using NanoDrop (Thermo Fisher Scientific Inc.). Table 4 shows whether or not aggregation occurred after dialysis.

Furthermore, the indices of hydrophobicity determined for each of the amino acids were summed up, the obtained value was divided by the number of the amino acids, and the outcome was determined as the GRAVY value (see Kyte J., Doolittle R. F. (1982), J. Mol. Biol, 157: 105-132). The GRAVY value is an index of the hydrophilicity and hydrophobicity of a polypeptide calculated from the degree of hydrophobicity of the amino acids contained in each polypeptide. The greater the GRAVY value, the more the polypeptide is hydrophobic, and the smaller the GRAVY value, the more the polypeptide is hydrophilic. The results are shown in Table 4.

Whether or not aggregation was occurred was evaluated based on the scale of G, A, and B as shown below. The results are summarized in Table 4.
G: The formation of an aggregate was not observed.
A: The formation of particles having a particle size of about 100 nm was observed.
B: The formation of an aggregation of particles having a particle size of equal to or greater than 1 mm was visually observed.

**[Table 4]**

| | GRAVY | Number of amino acids | Aggregation |
|---|---|---|---|
| RCP-1 | -0.835 | 247 | A |
| RCP-2 | -1.516 | 88 | G |
| RCP-3 | -1.124 | 108 | G |
| RCP-4 | -1.150 | 118 | G |
| RCP-5 | -1.124 | 128 | G |
| RCP-6 | -0.875 | 246 | A |
| RCP-7 | -0.979 | 160 | A |
| RCP-8 | -1.045 | 166 | A |
| RCP-9 | -0.958 | 176 | B |
| RCP-10 | -0.971 | 186 | B |
| RCP-17 | -1.072 | 143 | A |

From Table 4, it is understood that although each of RCP-2 to RCP-5, RCP-7, RCP-8, and RCP-17 is a polypeptide consisting of about 80 to 170 amino acid residues, and aggregation easily occurs in the polypeptide, the occurrence of aggregation is inhibited because the GRAVY value thereof is within a range of -1.70 to -0.975.

### [Reference example 2]

### <Evaluation of adsorbability with respect to culture dish>

Each of the polypeptides obtained as above was diluted with a predetermined buffer such that they could be added to wells at a predetermined final concentration of 0 pmol/cm² to 200 pmol/cm². Thereafter, each of the polypeptides was added in an amount of 64 µL to a plasma-treated 96-well plate made of polystyrene (Tissue Culture-Treated, Falcon). Each of the polypeptides was allowed to adsorb onto the plate by being incubated for 2 hours at 37°C, and then the wells were washed twice with PBS, thereby obtaining surfaces coated with each of the polypeptides of RCP-1 to RCP-16.

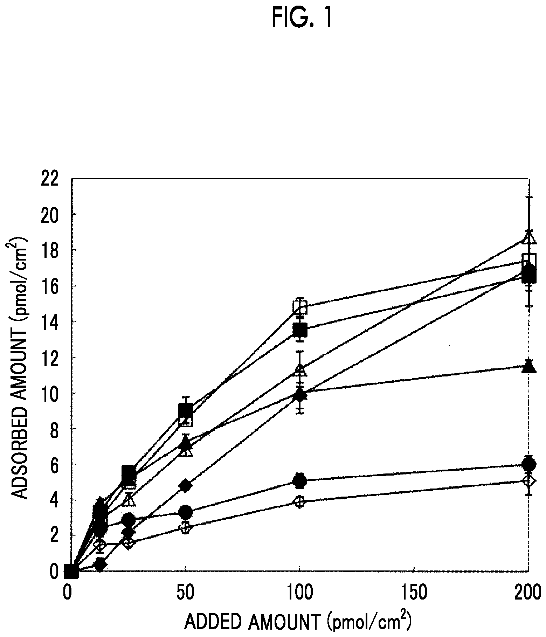

Among the surfaces coated with each of the polypeptides obtained as above, the surfaces coated with RCP-1 and RCP-11 to RCP-16 were applied with 64 µL of a borate buffer and 64 µL of 1 N NaOH, followed by incubation for 24 hours at 80°C and 100% humidity. After the resultant was air-cooled, 75 µL of a borate buffer was added to each well, and 50 µL of a reaction solution obtained by mixing OPA (o-phthalaldehyde: Wako Pure Chemical Industries, Ltd./methanol solution (160 mg/ml)) with N-acetyl-L-cysteine (NAC: Wako Pure Chemical Industries, Ltd.)/borate buffer solution (2 mg/ml) at a ratio of 1:100 (mass ratio) was further added thereto. After incubation for 30 minutes at 40°C, the fluorescence intensity thereof was measured by using an Envision Multilabel Counter (PerkinElmer Inc.) (excitation 355 nm/fluorescence 486 nm). A calibration curve was separately prepared from each of the polypeptide solutions so as to calculate the amount of the polypeptide adsorbed. The results are shown in Fig. 1. In Fig. 1, a black rhombus indicates RCP-1, a black square indicates RCP-11, a black triangle indicates RCP-12, a black circle indicates RCP-13, a white rhombus indicates RCP-14, a white square indicates RCP-15, and a white triangle indicates RCP-16.

From Fig. 1, it is understood that among the polypeptides used in the test, the polypeptides of RCP-1, RCP-15, and RCP-16 including PRPSLAKKQRFRHRNRKGYRSQRGHSRGRNQN (SEQ ID NO: 3 [the 342^{nd} to 373^{rd} amino acid residues in SEQ ID NO: 1]) exhibits excellent adsorbability with respect to the plate that is equivalent to the adsorbability of RCP-11 having the sequence of human vitronectin. It is also understood that, in contrast, the amount of RCP-13 and RCP-14 not including PRPSLAKKQRFRHRNRKGYRSQRGHSRGRNQN adsorbed onto the plate is small and about 1/4 of the amount of the polypeptide including the aforementioned sequence adsorbed onto the plate, and thus RCP-13 and RCP-14 are inappropriate as materials to be adsorbed onto the culture dish.

### [Reference example 3]

### <Cell adhesiveness evaluation 1>

The cell adhesiveness of human iPS cells ("Tic": Cell No. JCRB 1331: from National Institute of Biomedical Innovation. [567-0085, 7-6-8 Asagi Saito Ibaraki-City Osaka]) with respect to the aforementioned polypeptides was evaluated in the following manner.

As feeder cells for retaining the human iPS cells, EmbryoMax (registered trademark) (early mouse embryonic fibroblasts: resistant to hygromycin, treated with mitomycin C, derived from C57/BL6, third passage) (Millipore Corporation) was used. By using DMEM (Invitrogen) and a 10% (v/v) fetal bovine serum medium, the feeder cells were cultured for 24 hours and attached to a T25 flask (Corning Incorporated). As a medium for the human iPS cells, the one obtained by adding FGF-2 (Sigma-Aldrich Co, LLC.) to a medium composed as shown in Table 5 at a final concentration of 10 ng/ml was used.

**[Table 5]**

| Composition | Manufacturer | Amount |
|---|---|---|
| KO-DMEM/F12 | Invitrogen | 400 ml |
| Non-Essential Amino Acid Solution | | 4 ml |
| L-Glutamine | | 5 ml |
| Knock Out Serum Replacement | | 100 ml |
| 2-mercaptoethanol 55 mM | Wako Pure Chemical Industries, Ltd. | 0.925 ml |
| Total amount: about 500 ml | | |

By using the aforementioned medium, the human iPS cells were retained and cultured in a 5% (v/v, the same unit will be used hereinafter) CO₂ incubator at 37°C. Except for the day after the seeding of the iPS cells, the medium was replaced every day. The subculture operation was performed by exfoliating the cells by using Dispase II (neutral protease Grade II, Roche) and separating the cells in an appropriate size by a pipetting operation.

The iPS cells cultured as described above were treated with TrypLE Select (Invitrogen) for 5 minutes at 37°C and separated into a single cell. After being subjected to centrifugation for 2 minutes at 300 rpm, the cells were collected and suspended in TeSR2 (a medium not containing a heterogeneous animal-derived component and a serum component, STEMCELL Technologies.) containing Y-27362 ((R)-(+)-trans-N-(4-pyridyl)-4-(1-aminoethyl)-cyclohexanecarboxamide·2HCl·H₂O, an Rho binding kinase inhibitor, Wako Pure Chemical Industries, Ltd.) at a final concentration of 10 µM.

Samples 1 to 17 were prepared to which each of RCP-1 to RCP-10, RCP-17, RCP-11, RCP-15, RCP-16, and control including human vitronectin (extracted from human serum, BD Biosciences) and recombinant laminin (rLaminin-5: Oriental Yeast Co., ltd. and Human Recombinant Laminin-511: BIOLAMINA AB) was added at the concentration shown in Table 6. The samples were added to the respective wells of a 96-well plate and adsorbed onto the plate by being held for 2 hours at 37°C. Into the respective wells of the 96-well plate treated with the peptides, iPS cells were seeded at a cell density of 30,000 cells/well. After the cells were cultured for 24 hours, the nonadhesive cells were washed off with PBS, and only the adhesive cells were immobilized by using 4% paraformaldehyde (Wako Pure Chemical Industries, Ltd.). By using an Attophos (registered trademark) AP Fluorescent Substrate System (Promega Corporation), the ALP activity was calculated, and from the calibration curve, the number of undifferentiated iPS cells having the ALP activity was calculated. The results are shown in Table 6. In Table 6, the cell adhesion rate is expressed as a relative value calculated by regarding the cell adhesion rate obtained from the sample 15 using natural vitronectin as being 100. n = 3.

**[Table 6]**

| | Type of peptide | Added amount | Cell adhesion rate (%) |
|---|---|---|---|
| Sample 1 | RCP-1 | 200 pmol/cm² | 109.3 ±5.3 |
| Sample 2 | RCP-2 | 20 µg/cm² | 98.7 ± 6.2 |
| Sample 3 | RCP-3 | 20 µg/cm² | 106.1 ± 4.5 |
| Sample 4 | RCP-4 | 20 µg/cm² | 100.1 ± 4.5 |
| Sample 5 | RCP-5 | 10 µg/cm² | 94.1 ± 6.5 |
| Sample 6 | RCP-6 | 20 µg/cm² | 92.8 ± 4.4 |
| Sample 7 | RCP-7 | 5 µg/cm² | 88.6 ± 8.1 |
| Sample 8 | RCP-8 | 5 µg/cm² | 89.2 ± 1.4 |
| Sample 9 | RCP-9 | 20 µg/cm² | 95.5 ± 10.2 |
| Sample 10 | RCP-10 | 20 µg/cm² | 93.0 ± 7.8 |
| Sample 11 | RCP-17 | 5 µg/cm² | 95.9 ± 4.6 |
| Sample 12 | RCP-11 | 200 pmol/cm² | 93.5 ± 7.9 |
| Sample 13 | RCP-15 | 200 pmol/cm² | 13.2 ± 3.4 |
| Sample 14 | RCP-16 | 200 pmol/cm² | 9.4 ± 2.9 |
| Sample 15 | Natural vitronectin | 130 pmol/cm² | 100 ± 5.5 |
| Sample 16 | rLaminin-5 | 3.2 µg/cm² | 155.7 |
| Sample 17 | Laminin-511 | 5.0 µg/cm² | 142.0 |

As shown in Table 6, RCP-1 to RCP-10, PCR-17, and RCP-11, which had the 1^{st} to 55^{th} amino acids of the sequence represented by SEQ ID NO: 1, and natural human vitronectin were excellent in the cell adhesion rate of the iPS cells. Particularly, the cell adhesion rate was higher in RCP-1 to RCP-10 and PCR-17, which did not include a portion of the 56^{th} to 268^{th} amino acids of the sequence represented by SEQ ID NO: 1 or included none of the above amino acids, than in RCP-11 having the same amino acid sequence as the natural human vitronectin. Therefore, it is understood that a sequence important for the cell adhesion is present in the 1^{st} to 55^{th} amino acids of the sequence represented by SEQ ID NO: 1.

### [Reference example 4]

### <Cell adhesiveness evaluation 2>

The polypeptides shown in Table 7 were synthesized by an Fmoc solid-phase synthesis method. A surface onto which natural vitronectin was adsorbed at a concentration of 130 pmol/cm² was prepared, and then a cell suspension to which 100 µM of the aforementioned synthetic polypeptides were added was seeded into wells at a ratio of 30,000 cells/well. The number of adhesive cells 24 hours after seeding was calculated in the same manner as in <Cell adhesiveness evaluation 1>, and the results are shown in Table 7. In Table 7, the cell adhesion rate is expressed as a relative value calculated by regarding the cell adhesion rate obtained by using a culture solution not containing the synthetic polypeptides as being 100%. n = 3.

**[Table 7]**

| | Sequence of synthetic polypeptide | Cell adhesion rate (%) | SEQ ID No |
|---|---|---|---|
| Peptide-1 | DQESCKGRCTEGFNVDKKCQ | 91.8 ± 1.2 | 30 |
| Peptide-2 | KGRCTEGFNVDKKCQCDELC | 92.7 ± 19.6 | 31 |
| Peptide-3 | EGFNVDKKCQCDELCSYYQS | 102.5 ± 4.2 | 32 |
| Peptide-4 | DKKCQCDELCSYYQSCCTDY | 63.8 ± 11.6 | 33 |
| Peptide-5 | CCTDYTAECKPQVTRGDVFT | 70.5 ± 7.1 | 34 |
| Peptide-6 | TAECKPQVTRGDVFTMPEDE | 52.7 ± 10.3 | 35 |
| Peptide-7 | CCTDYTAECKPQVTRGEVFT | 86.7 ± 7.1 | 36 |
| Peptide-8 | TAECKPQVTRGEVFTMPEDE | 83.8 ± 14.8 | 37 |

From Table 7, it is understood that while the adhesion of cells to the natural vitronectin is significantly hindered by the addition of Peptides-4, 5, and 6 including CSYYQSC or RGD, the adhesion of cells is not hindered by the addition of Peptides-1, 2, and 3 not including CSYYQSC and RGD and Peptides-7 and 8 obtained by substituting the RGD sequence of Peptides-5 and 6 with RGE. Accordingly, it is understood that the polypeptide exhibits cell adhesion ability, in a case where the polypeptide includes at least one of CSYYQSC and RGD.

### [Reference example 5]

### <Growth evaluation>

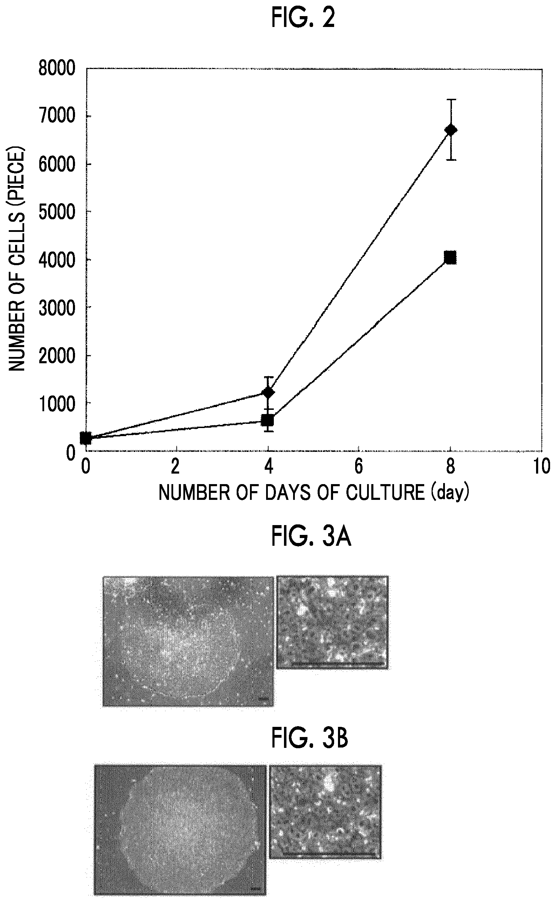

The iPS cells collected in the same manner as in <Cell adhesiveness evaluation 1> were seeded into a 96-well plate, onto which RCP-1, RCP-11, and natural human vitronectin were adsorbed, at a ratio of 250 cells/well and cultured for 8 days in a 5% CO₂ incubator at 37°C. The number of adhesive cells after different days of culture was measured in the same manner as in <Cell adhesiveness evaluation 1>, thereby obtaining growth curves. Fig. 2 shows the growth curves. In Fig. 2, a black rhombus indicates a case using RCP-1, and a black square indicates a case using RCP-11.

By adding RCP-1 to RCP-10, RCP-17, and Human Recombinant Laminin-511 as control at the concentration shown in Table 8, samples 1 to 12 were prepared. The samples were seeded into a 96-well plate, onto which each of the polypeptides was adsorbed in the same manner as in <Cell adhesiveness evaluation 1>, at a ratio of 5,000 cells/well and cultured for 3 days in a CO₂ incubator at 37°C. The number of cells after 3 days was calculated in the same manner as in <Cell adhesiveness evaluation 1>. The results are shown in Table 8.

**[Table 8]**

| | Type of peptide | Added amount | Number of cells after 3 days (%) |
|---|---|---|---|
| Sample 1 | RCP-1 | 80 µg/cm² | 100.0 |
| Sample 2 | RCP-2 | 20 µg/cm² | 81.7 |
| Sample 3 | RCP-3 | 20 µg/cm² | 109.7 |
| Sample 4 | RCP-4 | 20 µg/cm² | 120.7 |
| Sample 5 | RCP-5 | 10 µg/cm² | 121.2 |
| Sample 6 | RCP-6 | 20 µg/cm² | 70.5 |
| Sample 7 | RCP-7 | 5 µg/cm² | 89.9 |
| Sample 8 | RCP-8 | 5 µg/cm² | 171.0 |
| Sample 9 | RCP-9 | 20 µg/cm² | 105.8 |
| Sample 10 | RCP-10 | 20 µg/cm² | 101.9 |
| Sample 11 | RCP-17 | 5 µg/cm² | 153.5 |
| Sample 12 | Laminin-511 | 1.28 µg/cm² | 56.8 |

As shown in Fig. 2, RCP-1 showed higher cell growth properties compared to RCP-11 having the amino acid sequence of the natural vitronectin, and on Day 8 of culture, the number of cells in the case using RCP-11 was about 1/3 of the number of cells in the case where RCP-1 was used. From the increase and decrease in the obtained number of cells, the doubling time was calculated. As a result, it was confirmed that it takes 46.4 ± 2.1 hours for the number of cells to be doubled in a case where RCP-1 is used, and 67.7 ± 2.1 hours in a case where RCP-11 is used.

From Table 8, it is understood that all of RCP-1 to RCP-10 and RCP-17 show a higher cell growth rate compared to laminin which is the extracellular matrix just like vitronectin. Furthermore, it is understood that even if the 274^{th} cysteine residue in SEQ ID NO: 1 was substituted with a serine residue, the same high cell growth rate as described above is obtained.

From the results shown in Fig. 2 and Table 8, it is understood that, surprisingly, RCP-1 to RCP-10 and RCP-17, which include a sequence effective for the cell growth and the adsorption onto the culture dish but do not include a sequence corresponding to a portion or the entirety of the 56^{th} to 268^{th} amino acids of natural human vitronectin, have a growth ability higher than that of RCP-11 having the same sequence as the human vitronectin and Laminin-511 as a comparative example.

Furthermore, from Table 8, it is understood that all of RCP-1 to RCP-10 and RCP-17 including both the sequences of CSYYQSC and RDG and the sequence of PRPSLAKKQRFRHRNRKGYRSQRGHSRGRNQN show high cell growth properties.

### [Reference example 6]

### <Cell adhesiveness evaluation 3>

The cell adhesiveness was evaluated in the same manner as in <Cell adhesiveness evaluation 1>, except that RCP-1 was used after the concentration thereof was adjusted to 125 pmol/cm² to 1,000 pmol/cm² by using PBS. The results are shown in Table 9. In Table 9, the cell adhesion rate is expressed as relative value calculated by regarding a cell adhesion rate with respect to the culture vessel, onto which natural vitronectin is adsorbed at a concentration of 130 pmol/cm², as being 100. n = 3.

**[Table 9]**

| Type of peptide | Added amount | Cell adhesion rate (%) |
|---|---|---|
| RCP-1 | 1000 pmol/cm² | 98.5 ± 17.2 |
| RCP-1 | 500 pmol/cm² | 108.8 ± 23.0 |
| RCP-1 | 250 pmol/cm² | 89.2 ± 10.6 |
| RCP-1 | 125 pmol/cm² | 90.3 ± 25.3 |
| Natural vitronectin | 130 pmol/cm² | 100 ± 5.5 |

As shown in Table 9, in a case where the amount of RCP-1 added was equal to or greater than 125 pmol/cm², the adhesiveness of iPS cells with respect to RCP-1 was equivalent to the cell adhesion rate of the natural vitronection.

### [Reference example 7]

### <Evaluation of maintenance of undifferentiated state>

iPS cells collected in the same manner as in <Cell adhesiveness evaluation 1> were suspended in TeSR2. The iPS cells were seeded into 6-well plate (Tissue culture-treated, Falcon), onto which each of the samples 1, 2, 5, 6, and 7 used in <Cell adhesiveness evaluation 1> was adsorbed in the same manner as in <Cell adhesiveness evaluation 1>, and cultured in a CO₂ incubator at 37°C. Except for the day after the seeding, the medium was replaced every day. In the same method as described above, the cells were subcultured every six days. Figs. 3A to 3E show forms of the iPS cells cultured on each of the samples.

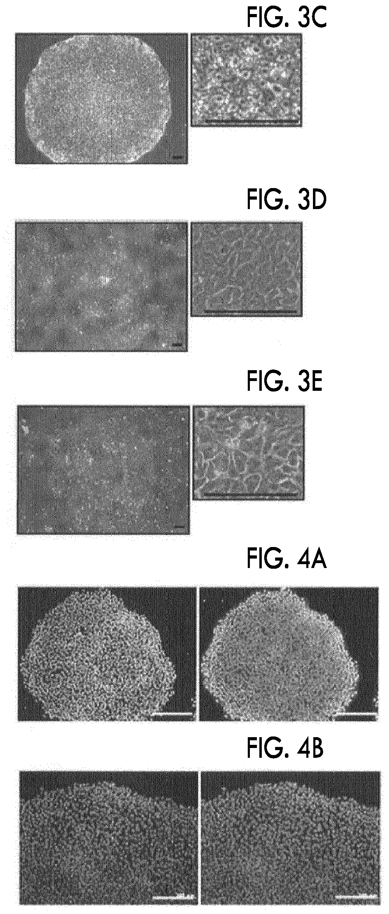

After being cultured for 1 month under the conditions described above, the cells were immobilized using 4% paraformaldehyde and treated with 1% Triton-X/PBS so as to enhance the membrane permeability. After the cells were subjected to a blocking treatment using an Image IT Signal Enhancer (Invitrogen), an anti-human NANOG antibody (AF 1997, R&D Systems, Inc.), an Alexa Fluor 555 binding rabbit anti-goat IgG antibody (Invitrogen), and DAPI (Dojindo Molecular Technologies, Inc.) were added thereto for labeling, and the cells were imaged using a fluorescence microscope. Figs. 4A to 4E show the fluorescence microscopic images.

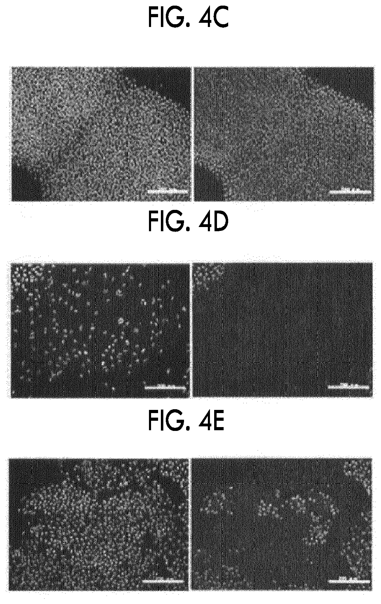

Figs. 3A and 4A show the iPS cells cultured on RCP-1; Figs. 3B and 4B show the iPS cells cultured on RCP-11; Figs. 3C and 4C show the iPS cells cultured on natural human vitronectin; Figs. 3D and 4D show the iPS cells cultured on rLaminin-5; and Figs. 3E and 4E show the iPS cells cultured on rLaminin-511. The scale bar in the images indicates 100 µm. In Figs. 3A to 3E, the images on the left side are full images of the colony, and the images on the right side are magnified images. In Figs. 4A to 4E, the images on the left side are images of the cells stained with DAPI, and the images on the right side are images of the cells stained with an anti-NANOG antibody. The scale bar in Figs. 3 and 4 indicates 200 µm.

As shown in Figs. 3A to 3E, the iPS cells, which were cultured on RCP-1, RCP-11, and the natural human vitronectin including a sequence effective for the cell growth and the adsorption onto the culture dish, had a form specific to undifferentiated cells which have a homogeneous colony and posses nuclei at a high ratio. Furthermore, as shown in Figs. 4A to 4E, the iPS cells, which were cultured on RCP-1, RCP-11, and the natural human vitronectin having a cell growth domain and an adsorption domain, strongly expressed NANOG in the entirety of the colony, and accordingly, it was understood that the undifferentiated state is excellently maintained.

From the evaluation results of Reference examples 1 to 7, it was understood that the polypeptide, which includes either the sequence CSYYQSC or the sequence RGD and the sequence of PRPSLAKKQRFRHRNRKGYRSQRGHSRGRNQN and consists of 40 to 450 amino acid residues, is excellent in the adsorbability with respect to the cell culture surface of the support. Furthermore, it was understood that under the condition of co-culture with iPS cells, such a polypeptide is equivalent to RCP-11 having a sequence equivalent to that of the natural vitronectin and human vitronection, in terms of the cell adhesiveness of the iPS cell and the maintenance of the undifferentiated state, and is better than RCP-11 in terms of the growth properties of the iPS cells. It was also understood that all of RCP-1 to RCP-10 and RCP-17 are excellent in terms of the cell adhesiveness of the iPS cells and the maintenance of the undifferentiated state. Such excellent results in terms of the aforementioned abilities were not obtained from other polypeptides or the recombinant laminin as a comparative example.

Therefore, according to the polypeptide (d) of the present invention, it is possible to provide a polypeptide, which enables pluripotent stem cells to grow in an undifferentiated state and is excellent in the adsorbability with respect to the cell culture surface, and a culture method and a culture vessel for pluripotent stem cells using the polypeptide.

### [Examples]

### <Growth evaluation>

The growth of cells was evaluated in the same manner as in Reference example 5.

Magnesium ascorbyl phosphate was added to a medium·Essential 8 (manufactured by Life Technologies), and RCP-17 and Human Recombinant Laminin-511 as control were added thereto such that the concentration of each of the polypeptides became as shown in Table 10, thereby preparing samples 1 to 15. The samples were adsorbed onto a 96-well plate, and the iPS cells collected in the same manner as in <Cell adhesiveness evaluation 1> were seeded into the plate at a ratio of 1 × 10⁴ cells/well and cultured for 3 days in a 5% CO₂ incubator at 37°C. The number of cells after 3 days was measured in the same manner as in <Cell adhesiveness evaluation 1>. Furthermore, by using VTN-N (manufactured by Life Technologies) and Vitronectin-XF (manufactured by Primorigen Biosciences), the growth of the cells was evaluated in the same manner.

The results obtained from RCP-17 are shown in Table 10, the results obtained from Human Recombinant Laminin-511 are shown in Table 11, the results obtained from VTN-N (manufactured by Life Technologies) are shown in Table 12, and the results obtained from Vitronectin-XF (manufactured by Primorigen Biosciences) are shown in Table 13. In Tables 10 and 11, the number of cells is expressed as a relative value calculated by regarding the number of cells at the concentration of magnesium ascorbyl phosphate of 0.8 mmol/L as being 100. n = 3.

**[Table 10]**

| Sample No. | Type of peptide | Added amount | Concentration of magnesium ascorbyl phosphate | Number of cells after 3 days (%) | Note |
|---|---|---|---|---|---|
| 1 | RCP-17 | 100 µg/cm³ | 0.2 mmol/L | 114 | Comparative example |
| 2 | RCP-17 | 100 µg/cm³ | 0.5 mmol/L | 98 | Comparative example |
| 3 | RCP-17 | 100 µg/cm³ | 0.8 mmol/L | 100 | Comparative example |
| 4 | RCP-17 | 100 µg/cm³ | 1.6 mmol/L | 145 | Present invention |
| 5 | RCP-17 | 100 µg/cm³ | 3.7 mmol/L | 219 | Present invention |
| 6 | RCP-17 | 100 µg/cm³ | 9.1 mmol/L | 212 | Present invention |
| 7 | RCP-17 | 100 µg/cm³ | 18.1 mmol/L | 204 | Present invention |
| 8 | RCP-17 | 100 µg/cm³ | 36.0 mmol/L | 230 | Present invention |

**[Table 11]**

| Sample No. | Type of peptide | Added amount | Concentration of magnesium ascorbyl phosphate | Number of cells after 3 days (%) | Note |
|---|---|---|---|---|---|
| 9 | Laminin-511 | 20 µg/cm³ | 0.2 mmol/L | 61 | Comparative example |
| 10 | Laminin-511 | 20 µg/cm³ | 0.5 mmol/L | 79 | Comparative example |
| 11 | Laminin-511 | 20 µg/cm³ | 0.8 mmol/L | 100 | Comparative example |
| 12 | Laminin-511 | 20 µg/cm³ | 1.6 mmol/L | 90 | Comparative example |
| 13 | Laminin-511 | 20 µg/cm³ | 3.7 mmol/L | 114 | Comparative example |
| 14 | Laminin-511 | 20 µg/cm³ | 9.1 mmol/L | 100 | Comparative example |
| 15 | Laminin-511 | 20 µg/cm³ | 18.1 mmol/L | 112 | Comparative example |

**[Table 12]**

| Sample No. | Type of peptide | Added amount | Concentration of magnesium ascorbyl phosphate | Number of cells after 3 days (%) | Note |
|---|---|---|---|---|---|
| 16 | VTN-N | 20 µg/cm³ | 0.2 mmol/L | 100 | Comparative example |
| 17 | VTN-N | 20 µg/cm³ | 1.6 mmol/L | 121 | Example |
| 18 | VTN-N | 20 µg/cm³ | 3.7 mmol/L | 124 | Example |
| 19 | VTN-N | 20 µg/cm³ | 9.1 mmol/L | 132 | Example |
| 20 | VTN-N | 20 µg/cm³ | 18.1 mmol/L | 135 | Example |
| 21 | VTN-N | 20 µg/cm³ | 36.0 mmol/L | 149 | Example |

**[Table 13]**

| Sample No. | Type of peptide | Added amount | Final concentration of magnesium ascorbyl phosphate | Number of cells after 3 days (%) | Note |
|---|---|---|---|---|---|
| 22 | Vitronectin-XF | 10 µg/cm³ | 0.2 mmol/L | 100 | Comparative example |
| 23 | Vitronectin- XF | 10 µg/cm³ | 1.6 mmol/L | 151 | Example |
| 24 | Vitronectin-XF | 10 µg/cm³ | 3.7 mmol/L | 153 | Example |
| 25 | Vitronectin-XF | 10 µg/cm³ | 9.1 mmol/L | 150 | Example |
| 26 | Vitronectin-XF | 10 µg/cm³ | 18.1 mmol/L | 155 | Example |
| 27 | Vitronectin-XF | 10 µg/cm³ | 36.0 mmol/L | 185 | Example |

From the results shown in Table 10, it was confirmed that in a case where the cells are cultured using RCP-17, the number of growing cells increases in Samples 4 to 8 depending on the concentration of magnesium ascorbyl phosphate, and there is a significant difference in the number of growing cells between the samples. In contrast, from the results shown in Table 11, it was confirmed that in a case where Human Recombinant Laminin-511 is used, the number of growing cells does not depend on the concentration of magnesium ascorbyl phosphate, and there is no significant difference in the number of growing cells between any two samples among the samples 9 to 15. From the results shown in Table 12, it was confirmed that in a case where VTN-N is used, the number of growing cells increases in the samples 17 to 21 depending on the concentration of magnesium ascorbyl phosphate, and there is a significant difference in the number of growing cells between the samples. From the results shown in Table 13, it was confirmed that in a case where Vitronectin-XF is used, the number of growing cells increases in the samples 23 to 27 depending on the concentration of magnesium ascorbyl phosphate, and there is a significant difference in the number of growing cells between the samples.

## Claims

1. A culture method for pluripotent stem cells, comprising:
obtaining a polypeptide-coated culture surface by applying a polypeptide to a cell culture surface of a support; and
culturing pluripotent stem cells by seeding the pluripotent stem cells onto the polypeptide-coated culture surface by using a medium in which the content of an ascorbic acid derivative is equal to or greater than 1.5 mmol/L (mM) and equal to or less than 100 mmol/L (mM),
wherein the polypeptide is (a) a polypeptide having an amino acid sequence represented by SEQ ID NO: 1, (b) a polypeptide having an amino acid sequence, which shares identity of equal to or higher than 80% with the amino acid sequence represented by SEQ ID NO: 1, and having culture performance for pluripotent stem cells, or (c) a polypeptide having an amino acid sequence, which is formed by the deletion, substitution, or addition of one amino acid or several amino acids in SEQ ID NO: 1, and having culture performance for pluripotent stem cells.

2. The culture method for pluripotent stem cells according to claim 1,
wherein the pluripotent stem cells are cultured by seeding the pluripotent stem cells onto the polypeptide-coated culture surface by using a medium in which the content of an ascorbic acid derivative is equal to or greater than 1.6 mmol/L (mM), and/or
wherein the polypeptide is (b) a polypeptide having an amino acid sequence, which shares identity of equal to or higher than 90% with the amino acid sequence represented by SEQ ID NO: 1, and having culture performance for pluripotent stem cells.

3. A culture method for pluripotent stem cells, comprising:
obtaining a polypeptide-coated culture surface by applying a polypeptide to a cell culture surface of a support; and
culturing pluripotent stem cells by seeding the pluripotent stem cells onto the polypeptide-coated culture surface by using a medium in which the content of an ascorbic acid derivative is equal to or greater than 1.5 mmol/L (mM) and equal to or less than 100 mmol/L (mM),
wherein the polypeptide is (d) a polypeptide consisting of 40 to 450 amino acid residues, and including (1) a first region including at least one amino acid sequence selected from the group consisting of an amino acid sequence represented by CSYYQSC (SEQ ID NO: 2) and an amino acid sequence represented by RGD and (2) a second region including (2-i) an amino acid sequence which is represented by PRPSLAKKQRFRHRNRKGYRSQRGHSRGRNQN (SEQ ID NO: 3), (2-ii) an amino acid sequence which shares identity of equal to or higher than 80% with the amino acid sequence represented by SEQ ID NO: 3 and exhibits adsorbability with respect to the cell culture surface of the support, or (2-iii) an amino acid sequence which is formed by the deletion, substitution, or addition of one amino acid residue or several amino acid residues in the amino acid sequence represented by SEQ ID NO: 3 and exhibits adsorbability with respect to the cell culture surface of the support.

4. The culture method for pluripotent stem cells according to claim 3,
wherein the pluripotent stem cells are cultured by seeding the pluripotent stem cells onto the polypeptide-coated culture surface by using a medium in which the content of an ascorbic acid derivative is equal to or greater than 1.6 mmol/L (mM), and/or
wherein the polypeptide is (d) a polypeptide consisting of 40 to 400 amino acid residues, preferably 40 to 250 amino acid residues.

5. The culture method for pluripotent stem cells according to claim 3 or 4,
wherein the first region of the polypeptide (d) includes:
(1-i) both the amino acid sequence represented by CSYYQSC (SEQ ID NO: 2) and the amino acid sequence represented by RGD; or
(1-ii) an amino acid sequence consisting of the 25^{th} to 47^{th} amino acid residues of an amino acid sequence represented by SEQ ID NO: 1,
or wherein the first region of the polypeptide (d) is:
(1-iii) an amino acid sequence which consists of the 1^{st} to 55^{th} amino acid residues in the amino acid sequence represented by SEQ ID NO: 1;
(1-iv) an amino acid sequence which consists of an amino acid sequence sharing identity of equal to or higher than 80% with an amino acid sequence consisting of the amino acid sequence (1-iii) and exhibits a cell adhesion ability with respect to pluripotent stem cells; or
(1-v) an amino acid sequence which consists of an amino acid sequence formed by the deletion, substitution, or addition of one amino acid or several amino acids in the amino acid sequence (1-iii) and exhibits a cell adhesion ability with respect to pluripotent stem cells.

6. The culture method for pluripotent stem cells according to any one of claims 3 to 5,
wherein the polypeptide (d) further includes a third region consisting of one amino acid sequence selected from the group consisting of the following amino acid sequences (3a-i) to (3a-iii):
(3a-i) an amino acid sequence, which consists of the 56^{th} to 341^{st} amino acid residues in the amino acid sequence represented by SEQ ID NO: 1, or a partial amino acid sequence thereof, or an amino acid sequence, which consists of the 269^{th} to 341^{st} amino acid residues in the amino acid sequence represented by SEQ ID NO: 1, or a partial amino acid sequence thereof,
(3a-ii) an amino acid sequence which shares identity of equal to or higher than 80% with the amino acid sequence (3a-i) or a partial amino acid sequence thereof, and
(3a-iii) an amino acid sequence which is formed by the deletion, substitution, or addition of one amino acid or several amino acids in the amino acid sequence (3a-i) or a partial amino acid sequence thereof.

7. The culture method for pluripotent, stem cells according to any one of claims 3 to 6,
wherein the polypeptide (d) further includes a fourth region consisting of one amino acid sequence selected from the group consisting of the following amino acid sequences (4a-i) to (4a-iii):
(4a-i) an amino acid sequence, which consists of the 374^{th} to 459^{th} amino acid residues in the amino acid sequence represented by SEQ ID NO: 1, or a partial amino acid sequence thereof,
(4a-ii) an amino acid sequence which shares identity of equal to or higher than 80% with the amino acid sequence (4a-i) or a partial amino acid sequence thereof, and
(4a-iii) an amino acid sequence which is formed by the deletion, substitution, or addition of one amino acid or several amino acids in the amino acid sequence (4a-i) or a partial amino acid sequence thereof.

8. The culture method for pluripotent stem cells according to claim 3 or 4,
wherein the polypeptide (d) consists of 80 to 450 amino acid residues and includes (1) a first region consisting of an amino acid sequence consisting of the 25^{th} to 47^{th} amino acid residues of the amino acid sequence represented by SEQ ID NO: 1, (2) a second region consisting of an amino acid sequence consisting of the 342^{nd} to 373^{rd} amino acid residues of the amino acid sequence represented by SEQ ID NO: 1, and at least one region selected from the group consisting of the following third and fourth regions:
(3) a third region consisting of an amino acid sequence, which consists of the 269^{th} to 341^{st} amino acid residues of the amino acid sequence represented by SEQ ID NO: 1, or a partial amino acid sequence thereof, and
(4) a fourth region consisting of an amino acid sequence, which consists of the 374^{th} to 459^{th} amino acid residues of the amino acid sequence represented by SEQ ID NO: 1, or a partial amino acid sequence thereof, or
wherein the polypeptide (d) consists of 100 to 450 amino acid residues and includes (1) a first region consisting of an amino acid sequence consisting of the 1^{st} to 55^{th} amino acid residues of the amino acid sequence represented by SEQ ID NO: 1, (2) a second region consisting of an amino acid sequence consisting of the 342^{nd} to 373^{rd} amino acid residues of the amino acid sequence represented by SEQ ID NO: 1, and at least one region selected from the group consisting of the following third and fourth regions:
(3) a third region consisting of an amino acid sequence, which consists of the 269^{th} to 341^{st} amino acid residues of the amino acid sequence represented by SEQ ID NO: 1, or a partial amino acid sequence thereof, and
(4) a fourth region consisting of an amino acid sequence, which consists of the 374^{th} to 459^{th} amino acid residues of the amino acid sequence represented by SEQ ID NO: 1, or a partial amino acid sequence thereof.

9. The culture method for pluripotent stem cells according to claim 6,
wherein the third region has an amino acid residue other than a cysteine residue, preferably a serine residue, an alanine residue, or a glycine residue, in a position corresponding to a cysteine residue of the amino acid sequence represented by SEQ ID NO: 1.

10. The culture method for pluripotent stem cells according to any one of claims 3 to 9,
wherein the first region of the polypeptide (d) is positioned on the N-terminal side of the second region.

11. The culture method for pluripotent stem cells according to any one of claims 3 to 10,
wherein two cysteine residues in the amino acid sequence of the polypeptide (d) that is represented by SEQ ID NO: 2 are cross-linked with each other.

12. The culture method for pluripotent stem cells according to claim 3 or 4,
wherein the polypeptide (d) is
(d1) a polypeptide having an amino acid sequence represented by one of SEQ ID NO: 4 to SEQ ID NO: 23, SEQ ID NO: 38, and SEQ ID NO: 39,
(d2) a polypeptide having an amino acid sequence, which shares identity of equal to or higher than 80% with the amino acid sequence represented by one of SEQ ID NO: 4 to SEQ ID NO: 23, SEQ ID NO: 38, and SEQ ID NO: 39, and having culture performance for pluripotent stem cells, or
(d3) a polypeptide having an amino acid sequence, which is formed by the deletion, substitution, or addition of one amino acid or several amino acids in the amino acid sequence represented by one of SEQ ID NO: 4 to SEQ ID NO: 23, SEQ ID NO: 38, and SEQ ID NO: 39, and having culture performance for pluripotent stem cells, or
wherein the polypeptide (d) is
(d4) a polypeptide consisting of an amino acid sequence represented by one of SEQ ID NO: 4 to 23, SEQ ID NO: 38, and SEQ ID NO: 39,
(d5) a polypeptide consisting of an amino acid sequence, which shares identity of equal to or higher than 80% with the amino acid sequence represented by one of SEQ ID NO: 4 to SEQ ID NO: 23, SEQ ID NO: 38, and SEQ ID NO: 39, and having culture performance for pluripotent stem cells, or
(d6) a polypeptide consisting of an amino acid sequence, which is formed by the deletion, substitution, or addition of one amino acid or several amino acids in the amino acid sequence represented by one of SEQ ID NO: 4 to SEQ ID NO: 23, SEQ ID NO: 38, and SEQ ID NO: 39, and having culture performance for pluripotent stem cells.

13. The culture method for pluripotent stem cells according to any one of claims 1 to 12,
wherein the pluripotent stem cells are selected from the group consisting of embryonic stem cells, induced pluripotent stem cells, and somatic stem cells, preferably induced pluripotent stem cells.

14. The culture method for pluripotent stem cells according to any one of claims 1 to 13,
wherein the content of the ascorbic acid derivative is equal to or less than 3.5 mmol/L (mM).

15. The culture method for pluripotent stem cells according to any one of claims 1 to 14,
wherein the ascorbic acid derivative is one or two or more ascorbic acid derivatives selected from the group consisting of ascorbic acid, magnesium ascorbyl phosphate, sodium ascorbyl phosphate, aminopropyl ascorbyl phosphate, disodium ascorbyl sulfate, and ascorbyl 2-phosphoric acid ester, preferably magnesium ascorbyl phosphate.

16. A kit for culture of pluripotent stem cells, comprising:
a polypeptide; and
a medium in which the content of an ascorbic acid derivative is equal to or greater than 1.5 mmol/L (mM) and equal to or less than 100 mmol/L (mM),
wherein the polypeptide is
(a) a polypeptide having an amino acid sequence represented by SEQ ID NO: 1,
(b) a polypeptide having an amino acid sequence, which shares identity of equal to or higher than 80% with the amino acid sequence represented by SEQ ID NO: 1, and having culture performance for pluripotent stem cells, or
(c) a polypeptide having an amino acid sequence, which is formed by the deletion, substitution, or addition of one amino acid or several amino acids in SEQ ID NO: 1, and having culture performance for pluripotent stem cells.

17. A kit for culture of pluripotent stem cells, comprising:
a polypeptide; and
a medium in which the content of an ascorbic acid derivative is equal to or greater than 1.5 mmol/L (mM) and equal to or less than 100 mmol/L (mM),
wherein the polypeptide is (d) a polypeptide consisting of 40 to 450 amino acid residues and including
(1) a first region including at least one amino acid sequence selected from the group consisting of an amino acid sequence represented by CSYYQSC (SEQ ID NO: 2) and an amino acid sequence represented by RGD and
(2) a second region including (2-i) an amino acid sequence which is represented by PRPSLAKKQRFRHRNRKGYRSQRGHSRGRNQN (SEQ ID NO: 3), (2-ii) an amino acid sequence which shares identity of equal to or higher than 80% with the amino acid sequence represented by SEQ ID NO: 3 and exhibits adsorbability with respect to a cell culture surface of a support, or (2-iii) an amino acid sequence which is formed by the deletion, substitution, or addition of one amino acid residue or several amino acid residues in the amino acid sequence represented by SEQ ID NO: 3 and exhibits adsorbability with respect to the cell culture surface of the support.

## Patentansprüche

1. Kulturverfahren für pluripotente Stammzellen, umfassend:
das Erhalten einer mit Polypeptid beschichteten Kulturoberfläche durch das Applizieren eines Polypeptids auf eine Zellkulturoberfläche eines Trägers; und
das Kultivieren von pluripotenten Stammzellen durch das Aussähen der pluripotenten Stammzellen auf die mit Polypeptid beschichtete Kulturoberfläche unter Verwendung eines Mediums, in dem der Gehalt eines Ascorbinsäurederivats gleich oder größer als 1,5 mmol/L (mM) und gleich oder geringer als 100 mmol/L (mM) ist,
wobei das Polypeptid (a) ein Polypeptid mit einer durch SEQ ID NO: 1 dargestellten Aminosäuresequenz ist, (b) ein Polypeptid mit einer Aminosäuresequenz, die eine Identität von gleich oder mehr als 80% zu der durch SEQ ID NO: 1 dargestellten Aminosäuresequenz aufweist, und mit Kulturleistung (culture performance) für pluripotente Stammzellen ist, oder (c) ein Polypeptid mit einer Aminosäuresequenz, die durch die Deletion, Substitution oder Addition einer Aminosäure oder mehrerer Aminosäuren in SEQ ID NO: 1 gebildet wird, und mit Kulturleistung (culture performance) für pluripotente Stammzellen ist.

2. Kulturverfahren für pluripotente Stammzellen gemäß Anspruch 1,
wobei die pluripotenten Stammzellen durch das Aussähen der pluripotenten Stammzellen auf die mit Polypeptid beschichtete Kulturoberfläche unter Verwendung eines Mediums, in dem der Gehalt eines Ascorbinsäurederivats gleich oder größer als 1,6 mmol/L (mM) ist, kultiviert werden, und/oder
wobei das Polypeptid (b) ein Polypeptid mit einer Aminosäuresequenz, die eine Identität von gleich oder mehr als 90% zu der durch SEQ ID NO: 1 dargestellten Aminosäuresequenz aufweist, und mit Kulturleistung (culture performance) für pluripotente Stammzellen ist.

3. Kulturverfahren für pluripotente Stammzellen, umfassend:
das Erhalten einer mit Polypeptid beschichteten Kulturoberfläche durch das Applizieren eines Polypeptids auf eine Zellkulturoberfläche eines Trägers; und
das Kultivieren von pluripotenten Stammzellen durch das Aussähen der pluripotenten Stammzellen auf die mit Polypeptid beschichtete Kulturoberfläche unter Verwendung eines Mediums, in dem der Gehalt eines Ascorbinsäurederivats gleich oder größer als 1,5 mmol/L (mM) und gleich oder geringer als 100 mmol/L (mM) ist,
wobei das Polypeptid (d) ein Polypeptid ist, das aus 40 bis 450 Aminosäureresten besteht, und einschließt (1) eine erste Region, die mindestens eine Aminosäuresequenz einschließt, die ausgewählt ist aus der Gruppe bestehend aus einer durch CSYYQSC (SEQ ID NO: 2) dargestellten Aminosäuresequenz und einer durch RGD dargestellten Aminosäuresequenz, und (2) eine zweite Region, die (2-i) eine Aminosäuresequenz, die durch PRPSLAKKQRFRHRNRKGYRSQRGHSRGRNQN (SEQ ID NO: 3) dargestellt ist, (2-ii) eine Aminosäuresequenz, die eine Identität von gleich oder mehr als 80% zu der durch SEQ ID NO: 3 dargestellten Aminosäuresequenz aufweist, und Adsorptionsfähigkeit in Bezug auf die Zellkulturoberfläche des Trägers zeigt, oder (2-iii) eine Aminosäuresequenz, die durch die Deletion, Substitution oder Addition eines Aminosäurerests oder mehrerer Aminosäurereste in der durch SEQ ID NO: 3 dargestellten Aminosäuresequenz gebildet wird, und Adsorptionsfähigkeit in Bezug auf die Zellkulturoberfläche des Trägers zeigt, einschließt.

4. Kulturverfahren für pluripotente Stammzellen gemäß Anspruch 3,
wobei die pluripotenten Stammzellen durch das Aussähen der pluripotenten Stammzellen auf die mit Polypeptid beschichtete Kulturoberfläche unter Verwendung eines Mediums, in dem der Gehalt eines Ascorbinsäurederivats gleich oder größer als 1,6 mmol/L (mM) ist, kultiviert werden, und/oder
wobei das Polypeptid (d) ein Polypeptid ist, das aus 40 bis 400 Aminosäureresten besteht, bevorzugt 40 bis 250 Aminosäureresten.

5. Kulturverfahren für pluripotente Stammzellen gemäß Anspruch 3 oder 4,
wobei die erste Region des Polypeptids (d):
(1-i) sowohl die durch CSYYQSC (SEQ ID NO: 2) dargestellte Aminosäuresequenz, als auch die durch RGD dargestellte Aminosäuresequenz einschließt; oder
(1-ii) eine Aminosäuresequenz einschließt, die aus dem 25sten bis 47sten Aminosäurerest einer durch SEQ ID NO: 1 dargestellten Aminosäuresequenz besteht,
oder wobei die erste Region des Polypeptids (d):
(1-iii) eine Aminosäuresequenz ist, die aus dem ersten bis 55sten Aminosäurerest in der durch SEQ ID NO: 1 dargestellten Aminosäuresequenz besteht;
(1-iv) eine Aminosäuresequenz ist, die aus einer Aminosäuresequenz besteht, die eine Identität von gleich oder mehr als 80% zu einer Aminosäuresequenz aufweist, die aus der Aminosäuresequenz (1-iii) besteht, und eine Zelladhäsionsfähigkeit in Bezug auf pluripotente Stammzellen zeigt; oder
(1-v) eine Aminosäuresequenz ist, die aus einer Aminosäuresequenz besteht, die durch die Deletion, Substitution oder Addition einer Aminosäure oder mehrerer Aminosäuren in der Aminosäuresequenz (1-iii) gebildet wird, und eine Zelladhäsionsfähigkeit in Bezug auf pluripotente Stammzellen zeigt.

6. Kulturverfahren für pluripotente Stammzellen gemäß irgendeinem der Ansprüche 3 bis 5,
wobei das Polypeptid (d) weiter eine dritte Region einschließt, die aus einer Aminosäuresequenz besteht, die ausgewählt ist aus der Gruppe bestehend aus den folgenden Aminosäuresequenzen (3a-i) bis (3a-iii):
(3a-i) einer Aminosäuresequenz, die aus dem 56sten bis 341sten Aminosäurerest in der durch SEQ ID NO: 1 dargestellten Aminosäuresequenz besteht, oder einer partiellen Aminosäuresequenz davon, oder einer Aminosäuresequenz, die aus dem 269sten bis 341sten Aminosäurerest in der durch SEQ ID NO: 1 dargestellten Aminosäuresequenz besteht, oder einer partiellen Aminosäuresequenz davon,
(3a-ii) einer Aminosäuresequenz, die eine Identität von gleich oder mehr als 80% zu der Aminosäuresequenz (3a-i) aufweist, oder einer partiellen Aminosäuresequenz davon, und
(3-iii) einer Aminosäuresequenz, die durch die Deletion, Substitution oder Addition einer Aminosäure oder mehrerer Aminosäuren in der Aminosäuresequenz (3a-i) gebildet wird, oder einer partiellen Aminosäuresequenz davon.

7. Kulturverfahren für pluripotente Stammzellen gemäß irgendeinem der Ansprüche 3 bis 6,
wobei das Polypeptid (d) weiter eine vierte Region einschließt, die aus einer Aminosäuresequenz besteht, die ausgewählt ist aus der Gruppe bestehend aus den folgenden Aminosäuresequenzen (4a-i) bis (4a-iii):
(4a-i) einer Aminosäuresequenz, die aus dem 374sten bis 459sten Aminosäurerest in der durch SEQ ID NO: 1 dargestellten Aminosäuresequenz besteht, oder einer partiellen Aminosäuresequenz davon,
(4a-ii) einer Aminosäuresequenz, die eine Identität von gleich oder mehr als 80% zu der Aminosäuresequenz (4a-i) aufweist, oder einer partiellen Aminosäuresequenz davon, und
(4-iii) einer Aminosäuresequenz, die durch die Deletion, Substitution oder Addition einer Aminosäure oder mehrerer Aminosäuren in der Aminosäuresequenz (4a-i) gebildet wird, oder einer partiellen Aminosäuresequenz davon.

8. Kulturverfahren für pluripotente Stammzellen gemäß Anspruch 3 oder 4, wobei das Polypeptid (d) aus 80 bis 450 Aminosäureresten besteht und (1) eine erste Region, die aus einer Aminosäuresequenz besteht, die aus dem 25sten bis 47sten Aminosäurerest der durch SEQ ID NO: 1 dargestellten Aminosäuresequenz besteht, (2) eine zweite Region, die aus einer Aminosäuresequenz besteht, die aus dem 342sten bis 373sten Aminosäurerest der durch SEQ ID NO: 1 dargestellten Aminosäuresequenz besteht, und mindestens eine Region einschließt, die ausgewählt ist aus der Gruppe bestehend aus der folgenden dritten und vierten Region:
(3) einer dritten Region, die aus einer Aminosäuresequenz besteht, die aus dem 269sten bis 341sten Aminosäurerest der durch SEQ ID NO: 1 dargestellten Aminosäuresequenz besteht, oder einer partiellen Aminosäuresequenz davon, und
(4) einer vierten Region, die aus einer Aminosäuresequenz besteht, die aus dem 374sten bis 459sten Aminosäurerest der durch SEQ ID NO: 1 dargestellten Aminosäuresequenz besteht, oder einer partiellen Aminosäuresequenz davon, oder
wobei das Polypeptid (d) aus 100 bis 450 Aminosäureresten besteht und (1) eine erste Region, die aus einer Aminosäuresequenz besteht, die aus dem ersten bis 55sten Aminosäurerest der durch SEQ ID NO: 1 dargestellten Aminosäuresequenz besteht, (2) eine zweite Region, die aus einer Aminosäuresequenz besteht, die aus dem 342sten bis 373sten Aminosäurerest der durch SEQ ID NO: 1 dargestellten Aminosäuresequenz besteht, und mindestens eine Region einschließt, die ausgewählt ist aus der Gruppe bestehend aus der folgenden dritten und vierten Region:
(3) einer dritten Region, die aus einer Aminosäuresequenz besteht, die aus dem 269sten bis 341sten Aminosäurerest der durch SEQ ID NO: 1 dargestellten Aminosäuresequenz besteht, oder einer partiellen Aminosäuresequenz davon, und
(4) einer vierten Region, die aus einer Aminosäuresequenz besteht, die aus dem 374sten bis 459sten Aminosäurerest der durch SEQ ID NO: 1 dargestellten Aminosäuresequenz besteht, oder einer partiellen Aminosäuresequenz davon.

9. Kulturverfahren für pluripotente Stammzellen gemäß Anspruch 6,
wobei die dritte Region einen anderen Aminosäurerest als einen Cysteinrest, bevorzugt einen Serinrest, einen Alaninrest oder einen Glycinrest, in einer Position hat, die einem Cysteinrest der durch SEQ ID NO: 1 dargestellten Aminosäuresequenz entspricht.

10. Kulturverfahren für pluripotente Stammzellen gemäß irgendeinem der Ansprüche 3 bis 9,
wobei die erste Region des Polypeptids (d) auf der N-terminalen Seite der zweiten Region positioniert ist.

11. Kulturverfahren für pluripotente Stammzellen gemäß irgendeinem der Ansprüche 3 bis 10,
wobei zwei Cysteinreste in der Aminosäuresequenz des Polypeptids (d), das durch SEQ ID NO: 2 dargestellt ist, miteinander quervernetzt (cross-linked) sind.

12. Kulturverfahren für pluripotente Stammzellen gemäß Anspruch 3 oder 4,
wobei das Polypeptid (d)
(d1) ein Polypeptid mit einer Aminosäuresequenz ist, die durch eine von SEQ ID NO: 4 bis SEQ ID NO: 23, SEQ ID NO: 38 und SEQ ID NO: 39 dargestellt ist,
(d2) ein Polypeptid mit einer Aminosäuresequenz, die eine Identität von gleich oder mehr als 80% zu der Aminosäuresequenz aufweist, die durch eine von SEQ ID NO: 4 bis SEQ ID NO: 23, SEQ ID NO: 38 und SEQ ID NO: 39 dargestellt ist, und mit Kulturleistung (culture performance) für pluripotente Stammzellen ist, oder
(d3) ein Polypeptid mit einer Aminosäuresequenz, die durch die Deletion, Substitution oder Addition einer Aminosäure oder mehrerer Aminosäuren in der Aminosäuresequenz gebildet wird, die durch eine von SEQ ID NO: 4 bis SEQ ID NO: 23, SEQ ID NO: 38 und SEQ ID NO: 39 dargestellt ist, und mit Kulturleistung (culture performance) für pluripotente Stammzellen ist, oder
wobei das Polypeptid (d)
(d4) eine Polypeptid ist, das aus einer Aminosäuresequenz besteht, die durch eine von SEQ ID NO: 4 bis 23, SEQ ID NO: 38 und SEQ ID NO: 39 dargestellt ist,
(d5) ein Polypeptid, das aus einer Aminosäuresequenz besteht, die eine Identität von gleich oder mehr als 80% zu der Aminosäuresequenz aufweist, die durch eine von SEQ ID NO: 4 bis SEQ ID NO: 23, SEQ ID NO: 38 und SEQ ID NO: 39 dargestellt ist, mit Kulturleistung (culture performance) für pluripotente Stammzellen ist, oder
(d6) ein Polypeptid, das aus einer Aminosäuresequenz besteht, die durch die Deletion, Substitution oder Addition einer Aminosäure oder mehrerer Aminosäuren in der Aminosäuresequenz, die durch eine von SEQ ID NO: 4 bis SEQ ID NO: 23, SEQ ID NO: 38 und SEQ ID NO: 39 dargestellt ist, mit Kulturleistung (culture performance) für pluripotente Stammzellen ist.

13. Kulturverfahren für pluripotente Stammzellen gemäß irgendeinem der Ansprüche 1 bis 12,
wobei die pluripotenten Stammzellen ausgewählt sind aus der Gruppe bestehend aus embryonalen Stammzellen, induzierten pluripotenten Stammzellen und somatischen Stammzellen, bevorzugt induzierten pluripotenten Stammzellen.

14. Kulturverfahren für pluripotente Stammzellen gemäß irgendeinem der Ansprüche 1 bis 13,
wobei der Gehalt des Ascorbinsäurederivats gleich oder geringer als 3,5 mmol/L (mM) ist.

15. Kulturverfahren für pluripotente Stammzellen gemäß irgendeinem der Ansprüche 1 bis 14,
wobei das Ascorbinsäurederivat ein oder zwei oder mehrere Ascorbinsäurederivate ist, die ausgewählt sind aus der Gruppe bestehend aus Ascorbinsäure, Magnesiumascorbylphosphat, Natriumascorbylphosphat, Aminopropylascorbylphosphat, Dinatriumascorbylsulfat und Ascorbyl-2-Phophorsäureester, bevorzugt Magnesiumascorbylphosphat.

16. Kit zum Kultivieren pluripotenter Stammzellen, umfassend:
ein Polypeptid; und
ein Medium, in dem der Gehalt eines Ascorbinsäurederivats gleich oder größer als 1,5 mmol/L (mM) und gleich oder geringer als 100 mmol/L (mM) ist,
wobei das Polypeptid
(a) ein Polypeptid mit einer durch SEQ ID NO: 1 dargestellten Aminosäuresequenz ist,
(b) ein Polypeptid mit einer Aminosäuresequenz, die eine Identität von gleich oder mehr als 80% zu der durch SEQ ID NO: 1 dargestellten Aminosäuresequenz aufweist, und mit Kulturleistung (culture performance) für pluripotente Stammzellen ist, oder
(c) ein Polypeptid mit einer Aminosäuresequenz, die durch die Deletion, Substitution oder Addition einer Aminosäure oder mehrerer Aminosäuren in SEQ ID NO: 1 gebildet wird, und mit Kulturleistung (culture performance) für pluripotente Stammzellen ist.

17. Kit zum Kultivieren pluripotenter Stammzellen, umfassend:
ein Polypeptid; und
ein Medium, in dem der Gehalt eines Ascorbinsäurederivats gleich oder größer als 1,5 mmol/L (mM) und gleich oder geringer als 100 mmol/L (mM) ist,
wobei das Polypeptid (d) ein Polypeptid ist, das aus 40 bis 450 Aminosäureresten besteht, und einschließt
(1) eine erste Region, die mindestens eine Aminosäuresequenz einschließt, die ausgewählt ist aus der Gruppe bestehend aus einer durch CSYYQSC (SEQ ID NO: 2) dargestellten Aminosäuresequenz und einer durch RGD dargestellten Aminosäuresequenz, und
(2) eine zweite Region, die (2-i) eine Aminosäuresequenz, die durch PRPSLAKKQRFRHRNRKGYRSQRGHSRGRNQN (SEQ ID NO: 3) dargestellt ist, (2-ii) eine Aminosäuresequenz, die eine Identität von gleich oder mehr als 80% zu der durch SEQ ID NO: 3 dargestellten Aminosäuresequenz aufweist, und Adsorptionsfähigkeit in Bezug auf eine Zellkulturoberfläche eines Trägers zeigt, oder (2-iii) eine Aminosäuresequenz, die durch die Deletion, Substitution oder Addition eines Aminosäurerests oder mehrerer Aminosäurereste in der durch SEQ ID NO: 3 dargestellten Aminosäuresequenz gebildet wird, und Adsorptionsfähigkeit in Bezug auf die Zellkulturoberfläche des Trägers zeigt, einschließt.

## Revendications

1. Procédé de culture pour cellules souches pluripotentes comprenant :
l'obtention d'une surface de culture revêtue d'un polypeptide en appliquant un polypeptide à une surface de culture cellulaire d'un support ; et
la culture de cellules souches pluripotentes par ensemencement des cellules souches pluripotentes sur la surface de culture revêtue d'un polypeptide en utilisant un milieu dans lequel le contenu d'un dérivé d'acide ascorbique est égal ou supérieur à 1,5 mmole/L (mM) et égal ou inférieur à 100 mmoles/L (mM),
dans lequel le polypeptide est (a) un polypeptide ayant une séquence d'acides aminés représentée par la SEQ ID n° 1, (b) un polypeptide ayant une séquence d'acides aminés qui partage une identité égale ou supérieure à 80 % avec la séquence d'acides aminés représentée par la SEQ ID n° 1 et ayant un rendement de culture pour les cellules souches pluripotentes, ou (c) un polypeptide ayant une séquence d'acides aminés qui est formée par la délétion, la substitution ou l'addition d'un acide aminé ou de plusieurs acides aminés dans la SEQ ID n° 1 et ayant un rendement de culture pour les cellules souches pluripotentes.

2. Procédé de culture pour cellules souches pluripotentes selon la revendication 1, dans lequel les cellules souches pluripotentes sont cultivées par ensemencement des cellules souches pluripotentes sur la surface de culture revêtue d'un polypeptide en utilisant un milieu dans lequel le contenu d'un dérivé d'acide ascorbique est égal ou supérieur à 1,6 mmole/L (mM) et/ou
dans lequel le polypeptide est (b) un polypeptide ayant une séquence d'acides aminés qui partage une identité égale ou supérieure à 90 % avec la séquence d'acides aminés représentée par la SEQ ID n° 1 et ayant un rendement de culture pour les cellules souches pluripotentes.

3. Procédé de culture pour cellules souches pluripotentes comprenant :
l'obtention d'une surface de culture revêtue d'un polypeptide en appliquant un polypeptide à une surface de culture cellulaire d'un support ; et
la culture de cellules souches pluripotentes par ensemencement des cellules souches pluripotentes sur la surface de culture revêtue d'un polypeptide en utilisant un milieu dans lequel le contenu d'un dérivé d'acide ascorbique est égal ou supérieur à 1,5 mmole/L (mM) et égal ou inférieur à 100 mmoles/L (mM),
dans lequel le polypeptide est (d) un polypeptide constitué de 40 à 450 résidus d'acides aminés et comprenant (1) une première région comprenant une séquence d'acides aminés choisie dans le groupe constitué d'une séquence d'acides aminés représentée par CSYYQSC (SEQ ID n° 2) et une séquence d'acides aminés représentée par RGD et (2) une deuxième région comprenant (2-i) une séquence d'acides aminés qui est représentée par PRPSLAKKQRFRHRNRKGYRSQRGHSRGRNQN (SEQ ID n° 3), (2-ii) une séquence d'acides aminés qui partage une identité égale ou supérieure à 80 % avec la séquence d'acides aminés représentée par la SEQ ID n° 3 et présente une capacité d'adsorption par rapport à la surface de culture cellulaire du support ou (2-iii) une séquence d'acides aminés qui est formée par la délétion, la substitution ou l'addition d'un résidu d'acide aminé ou de plusieurs résidus d'acides aminés dans la séquence d'acides aminés représentée par la SEQ ID n° 3 et présente une capacité d'adsorption par rapport à la surface de culture cellulaire du support.

4. Procédé de culture de cellules souches pluripotentes selon la revendication 3, dans lequel les cellules souches pluripotentes sont cultivées par ensemencement des cellules souches pluripotentes sur la surface de culture revêtue d'un polypeptide en utilisant un support dans lequel le contenu d'un dérivé d'acide ascorbique est égal ou supérieur à 1,6 mmole/L (mM) et/ou
dans lequel le polypeptide est (d) un polypeptide constitué de 40 à 400 résidus d'acides aminés, de préférence de 40 à 250 résidus d'acides aminés.

5. Procédé de culture pour cellules souches pluripotentes selon la revendication 3 ou 4,
dans lequel la première région du polypeptide (d) comprend :
(1-i) à la fois la séquence d'acides aminés représentée par CSYYQSC (SEQ ID n° 2) et la séquence d'acides aminés représentée par RGD ; ou
(1-ii) une séquence d'acides aminés constituée du 25^{ème} au 47^{ème} résidu d'acides aminés d'une séquence d'acides aminés représentée par la SEQ ID n° 1,
ou dans lequel la première région du polypeptide (d) est :
(1-iii) une séquence d'acides aminés qui est constituée du 1^{er} au 55^{ème} résidu d'acides aminés dans la séquence d'acides aminés représentée par la SEQ ID n° 1 ;
(1-iv) une séquence d'acides aminés qui est constituée d'une séquence d'acides aminés partageant une identité égale ou supérieure à 80 % avec une séquence d'acides aminés constituée de la séquence d'acides aminés (1-iii) et présente une capacité d'adhérence cellulaire par rapport aux cellules souches pluripotentes ; ou
(1-v) une séquence d'acides aminés qui est constituée d'une séquence d'acides aminés formée par la délétion, la substitution ou l'addition d'un acide aminé ou de plusieurs acides aminés dans la séquence d'acides aminés (1-iii) et présente une capacité d'adhérence cellulaire par rapport aux cellules souches pluripotentes.

6. Procédé de culture pour cellules souches pluripotentes selon l'une quelconque des revendications 3 à 5,
dans lequel le polypeptide (d) comprend en outre une troisième région constituée d'une séquence d'acides aminés choisie dans le groupe constitué des séquences d'acides aminés suivantes (3a-i) à (3a-iii) :
(3a-i) une séquence d'acides aminés qui est constituée du 56^{ème} au 341^{ème} résidu d'acides aminés dans la séquence d'acides aminés représentée par la SEQ ID n° 1 ou une séquence d'acides aminés partielle de celle-ci, ou une séquence d'acides aminés qui est constituée du 269^{ème} au 341^{ème} résidu d'acides aminés dans la séquence d'acides aminés représentée par la SEQ ID n° 1 ou une séquence d'acides aminés partielle de celle-ci,
(3a-ii) une séquence d'acides aminés qui partage une identité égale ou supérieure à 80 % avec la séquence d'acides aminés (3a-i) ou une séquence d'acides aminés partielle de celle-ci et
(3a-iii) une séquence d'acides aminés qui est formée par la délétion, la substitution ou l'addition d'un acide aminé ou de plusieurs acides aminés dans la séquence d'acides aminés (3a-i) ou une séquence d'acides aminés partielle de celle-ci.

7. Procédé de culture pour cellules souches pluripotentes selon l'une quelconque des revendications 3 à 6,
dans lequel le polypeptide (d) comprend en outre une quatrième région constituée d'une séquence d'acides aminés choisie dans le groupe constitué des séquences d'acides aminés suivantes (4a-i) à (4a-iii) :
(4a-i) une séquence d'acides aminés qui est constituée du 374^{ème} au 459^{ème} résidu d'acides aminés dans la séquence d'acides aminés représentée par la SEQ ID n° 1 ou une séquence d'acides aminés partielle de celle-ci,
(4a-ii) une séquence d'acides aminés qui partage une identité égale ou supérieure à 80 % avec la séquence d'acides aminés (4a-i) ou une séquence d'acides aminés partielle de celle-ci et
(4a-iii) une séquence d'acides aminés qui est formée par la délétion, la substitution ou l'addition d'un acide aminé ou de plusieurs acides aminés dans la séquence d'acides aminés (4a-i) ou une séquence d'acides aminés partielle de celle-ci.

8. Procédé de culture pour cellules souches pluripotentes selon la revendication 3 ou 4,
dans lequel le polypeptide (d) est constitué de 80 à 450 résidus d'acides aminés et comprend (1) une première région constituée d'une séquence d'acides aminés constituée du 25^{ème} au 47^{ème} résidu d'acides aminés de la séquence d'acides aminés représentée par la SEQ ID n° 1, (2) une deuxième région constituée d'une séquence d'acides aminés constituée du 342^{ème} au 373^{ème} résidu d'acides aminés de la séquence d'acides aminés représentée par la SEQ ID n° 1 et au moins une région choisie dans le groupe constitué de la troisième et de la quatrième région suivantes :
(3) une troisième région constituée d'une séquence d'acides aminés qui est constituée du 269^{ème} au 341^{ème} résidu d'acides aminés de la séquence d'acides aminés représentée par la SEQ ID n° 1 ou d'une séquence d'acides aminés partielle de celle-ci et
(4) une quatrième région constituée d'une séquence d'acides aminés qui est constituée du 374^{ème} au 459^{ème} résidu d'acides aminés de la séquence d'acides aminés représentée par la SEQ ID n° 1 ou d'une séquence d'acides aminés partielle de celle-ci ou
dans lequel le polypeptide (d) est constitué de 100 à 450 résidus d'acides aminés et comprend (1) une première région constituée d'une séquence d'acides aminés constituée du 1^{er} au 55^{ème} résidu d'acides aminés de la séquence d'acides aminés représentée par la SEQ ID n° 1, (2) une deuxième région constituée d'une séquence d'acides aminés constituée du 342^{ème} au 373^{ème} résidu d'acides aminés de la séquence d'acides aminés représentée par la SEQ ID n° 1 et au moins une région choisie dans le groupe constitué des troisième et quatrième régions suivantes :
(3) une troisième région constituée d'une séquence d'acides aminés qui est constituée du 269^{ème} au 341^{ème} résidu d'acides aminés de la séquence d'acides aminés représentée par la SEQ ID n° 1 ou d'une séquence d'acides aminés partielle de celle-ci et
(4) une quatrième région constituée d'une séquence d'acides aminés qui est constituée du 374^{ème} au 459^{ème} résidu d'acides aminés de la séquence d'acides aminés représentée par la SEQ ID n° 1 ou d'une séquence d'acides aminés partielle de celle-ci.

9. Procédé de culture pour cellules souches pluripotentes selon la revendication 6, dans lequel la troisième région a un résidu d'acide aminé autre qu'un résidu de cystéine, de préférence un résidu de sérine, un résidu d'alanine ou un résidu de glycine, dans une position correspondant à un résidu de cystéine de la séquence d'acides aminés représentée par la SEQ ID n° 1.

10. Procédé de culture pour cellules souches pluripotentes selon l'une quelconque des revendications 3 à 9,
dans lequel la première région du polypeptide (d) est positionnée sur le côté N-terminal de la deuxième région.

11. Procédé de culture pour cellules souches pluripotentes selon l'une quelconque des revendications 3 à 10,
dans lequel deux résidus de cystéine de la séquence d'acides aminés du polypeptide (d) qui est représentée par la SEQ ID n° 2 sont réticulés mutuellement.

12. Procédé de culture pour cellules souches pluripotentes selon la revendication 3 ou 4,
dans lequel le polypeptide (d) est :
(d1) un polypeptide ayant une séquence d'acides aminés représentée par l'une de la SEQ ID n° 4 à la SEQ ID n° 23, de la SEQ ID n° 38 et de la SEQ ID n° 39.
(d2) un polypeptide ayant une séquence d'acides aminés qui partage une identité égale ou supérieure à 80 % avec la séquence d'acides aminés représentée par l'une de la SEQ ID n° 4 à la SEQ ID n° 23, de la SEQ ID n° 38 et de la SEQ ID n° 39 et ayant un rendement de culture pour des cellules souches pluripotentes ou
(d3) un polypeptide ayant une séquence d'acides aminés qui est formée par la délétion, la substitution ou l'addition d'un acide aminé ou de plusieurs acides aminés dans la séquence d'acides aminés représentée par l'une de la SEQ ID n° 4 à la SEQ ID n° 23, de la SEQ ID n° 38 et de la SEQ ID n° 39 et ayant un rendement de culture pour les cellules souches pluripotentes, ou
dans lequel le polypeptide (d) est :
(d4) un polypeptide constitué d'une séquence d'acides aminés représentée par l'une de la SEQ ID n° 4 à la SEQ ID n° 23, de la SEQ ID n° 38 et de la SEQ ID n° 39,
(d5) un polypeptide constitué d'une séquence d'acides aminés qui partage une identité égale ou supérieure à 80 % avec la séquence d'acides aminés représentée par l'une de la SEQ ID n° 4 à la SEQ ID n° 23, de la SEQ ID n° 38 et de la SEQ ID n° 39 et ayant un rendement de culture pour les cellules souches pluripotentes, ou
(d6) un polypeptide constitué d'une séquence d'acides aminés qui est formée par la délétion, la substitution ou l'addition d'un acide aminé ou de plusieurs acides aminés dans la séquence d'acides aminés représentée par l'une de la SEQ ID n° 4 à la SEQ ID n° 23, de la SEQ ID n° 38 et de la SEQ ID n° 39 et ayant un rendement de culture pour les cellules souches pluripotentes.

13. Procédé de culture pour cellules souches pluripotentes selon l'une quelconque des revendications 1 à 12,
dans lequel les cellules souches pluripotentes sont choisies dans le groupe constitué de cellules souches embryonnaires, de cellules souches pluripotentes induites et de cellules souches somatiques, de préférence de cellules souches pluripotentes induites.

14. Procédé de culture pour cellules souches pluripotentes selon l'une quelconque des revendications 1 à 13,
dans lequel le contenu du dérivé d'acide ascorbique est égal ou inférieur à 3,5 mmoles/L (mM).

15. Procédé de culture pour cellules souches pluripotentes selon l'une quelconque des revendications 1 à 14,
dans lequel le dérivé d'acide ascorbique est constitué d'un ou deux ou de plus de dérivés d'acide ascorbique choisis dans le groupe constitué de l'acide ascorbique, de l'ascorbylphosphate de magnésium, de l'ascorbylphosphate de sodium, de l'ascorbylphosphate d'aminopropyle, de l'ascorbylsulfate disodique et de l'ester d'ascorbyle d'acide 2-phosphorique, de préférence de l'ascorbylphosphate de magnésium.

16. Kit de culture de cellules souches pluripotentes, comprenant :
un polypeptide ; et
un milieu dans lequel le contenu d'un dérivé d'acide ascorbique est égal ou supérieur à 1,5 mmole/L (mM) et égal ou inférieur à 100 mmoles/L (mM),
dans lequel le polypeptide est :
(a) un polypeptide ayant une séquence d'acides aminés représentée par la SEQ ID n° 1,
(b) un polypeptide ayant une séquence d'acides aminés qui partage une identité égale ou supérieure à 80 % avec la séquence d'acides aminés représentée par la SEQ ID n° 1 et ayant un rendement de culture pour les cellules souches pluripotentes, ou
(c) un polypeptide ayant une séquence d'acides aminés qui est formée par la délétion, la substitution ou l'addition d'un acide aminé ou de plusieurs acides aminés dans la SEQ ID n° 1 et ayant un rendement de culture pour les cellules souches pluripotentes.

17. Kit de culture de cellules souches pluripotentes comprenant :
un polypeptide ; et
un milieu dans lequel le contenu d'un dérivé d'acide ascorbique est égal ou supérieur à 1,5 mmole/L (mM) et égal ou inférieur à 100 mmoles/L (mM),
dans lequel le polypeptide est (d) un polypeptide constitué de 40 à 450 résidus d'acides aminés et comprenant :
(1) une première région comprenant au moins une séquence d'acides aminés choisie dans le groupe constitué d'une séquence d'acides aminés représentée par CSYYQSC (SEQ ID n° 2) et d'une séquence d'acides aminés représentée par RGD et
(2) une deuxième région comprenant (2-i) une séquence d'acides aminés qui est représentée par PRPSLAKKQRFRHRNRKGYRSQRGHSRGRNQN (SEQ ID n° 3), (2-ii) une séquence d'acides aminés qui partage une identité égale ou supérieure à 80 % avec la séquence d'acides aminés représentée par la SEQ ID n° 3 et présente une capacité d'adsorption par rapport à une surface de culture cellulaire d'un support ou (2-iii) une séquence d'acides aminés qui est formée par la délétion, la substitution ou l'addition d'un résidu d'acide aminé ou de plusieurs résidus d'acides aminés dans la séquence d'acides aminés représentée par la SEQ ID n° 3 et présente une capacité d'adsorption par rapport à la surface de culture cellulaire du support.


### SEQUENCE LISTING

<110> FUJIFILM CORPORATION
<120> Method for culturing Pluripotent Stem Cells, and Kit for culturing Pluripotent stem cells and Culture medium therefor
<130> FS-F06112-00
<150> JP2013-187441
   <151> 2013-09-10
<160> 39
<170> PatentIn version 3.5
<210> 1
   <211> 459
   <212> PRT
   <213> Homo sapiens
<400> 1
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Tyr Thr Val Tyr Asp Asp Gly Glu Glu
       50                  55                  60
   Lys Asn Asn Ala Thr Val His Glu Gln Val Gly Gly Pro Ser Leu Thr
   65                  70                  75                  80
   Ser Asp Leu Gln Ala Gln Ser Lys Gly Asn Pro Glu Gln Thr Pro Val
                   85                  90                  95
   Leu Lys Pro Glu Glu Glu Ala Pro Ala Pro Glu Val Gly Ala Ser Lys
               100                 105                 110
   Pro Glu Gly Ile Asp Ser Arg Pro Glu Thr Leu His Pro Gly Arg Pro
           115                 120                 125
   Gln Pro Pro Ala Glu Glu Glu Leu Cys Ser Gly Lys Pro Phe Asp Ala
       130                 135                 140
   Phe Thr Asp Leu Lys Asn Gly Ser Leu Phe Ala Phe Arg Gly Gln Tyr
   145                 150                 155                 160
   Cys Tyr Glu Leu Asp Glu Lys Ala Val Arg Pro Gly Tyr Pro Lys Leu
                   165                 170                 175
   Ile Arg Asp Val Trp Gly Ile Glu Gly Pro Ile Asp Ala Ala Phe Thr
               180                 185                 190
   Arg Ile Asn Cys Gln Gly Lys Thr Tyr Leu Phe Lys Gly Ser Gln Tyr
           195                 200                 205
   Trp Arg Phe Glu Asp Gly Val Leu Asp Pro Asp Tyr Pro Arg Asn Ile
       210                 215                 220
   Ser Asp Gly Phe Asp Gly Ile Pro Asp Asn Val Asp Ala Ala Leu Ala
   225                 230                 235                 240
   Leu Pro Ala His Ser Tyr Ser Gly Arg Glu Arg Val Tyr Phe Phe Lys
                   245                 250                 255
   Gly Lys Gln Tyr Trp Glu Tyr Gln Phe Gln His Gln Pro Ser Gln Glu
               260                 265                 270
   Glu Cys Glu Gly Ser Ser Leu Ser Ala Val Phe Glu His Phe Ala Met
           275                 280                 285
   Met Gln Arg Asp Ser Trp Glu Asp Ile Phe Glu Leu Leu Phe Trp Gly
       290                 295                 300
   Arg Thr Ser Ala Gly Thr Arg Gln Pro Gln Phe Ile Ser Arg Asp Trp
   305                 310                 315                 320
   His Gly Val Pro Gly Gln Val Asp Ala Ala Met Ala Gly Arg Ile Tyr
                   325                 330                 335
   Ile Ser Gly Met Ala Pro Arg Pro Ser Leu Ala Lys Lys Gln Arg Phe
               340                 345                 350
   Arg His Arg Asn Arg Lys Gly Tyr Arg Ser Gln Arg Gly His Ser Arg
           355                 360                 365
   Gly Arg Asn Gln Asn Ser Arg Arg Pro Ser Arg Ala Thr Trp Leu Ser
       370                 375                 380
   Leu Phe Ser Ser Glu Glu Ser Asn Leu Gly Ala Asn Asn Tyr Asp Asp
   385                 390                 395                 400
   Tyr Arg Met Asp Trp Leu Val Pro Ala Thr Cys Glu Pro Ile Gln Ser
                   405                 410                 415
   Val Phe Phe Phe Ser Gly Asp Lys Tyr Tyr Arg Val Asn Leu Arg Thr
               420                 425                 430
   Arg Arg Val Asp Thr Val Asp Pro Pro Tyr Pro Arg Ser Ile Ala Gln
           435                 440                 445
   Tyr Trp Leu Gly Cys Pro Ala Pro Gly His Leu
       450                 455
<210> 2
   <211> 7
   <212> PRT
   <213> Artificial Sequence
<220>
   <223> CSYYQSC
<400> 2
   Cys Ser Tyr Tyr Gln Ser Cys
   1               5
<210> 3
   <211> 32
   <212> PRT
   <213> Artificial Sequence
<220>
   <223> Heparin Binding Domain
<400> 3
   Pro Arg Pro Ser Leu Ala Lys Lys Gln Arg Phe Arg His Arg Asn Arg
   1               5                   10                  15
   Lys Gly Tyr Arg Ser Gln Arg Gly His Ser Arg Gly Arg Asn Gln Asn
               20                  25                  30
<210> 4
   <211> 246
   <212> PRT
   <213> Artificial Sequence
<220>
   <223> No.4
<400> 4
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Pro Ser Gln Glu Glu Cys Glu Gly Ser
       50                  55                  60
   Ser Leu Ser Ala Val Phe Glu His Phe Ala Met Met Gln Arg Asp Ser
   65                  70                  75                  80
   Trp Glu Asp Ile Phe Glu Leu Leu Phe Trp Gly Arg Thr Ser Ala Gly
                   85                  90                  95
   Thr Arg Gln Pro Gln Phe Ile Ser Arg Asp Trp His Gly Val Pro Gly
               100                 105                 110
   Gln Val Asp Ala Ala Met Ala Gly Arg Ile Tyr Ile Ser Gly Met Ala
           115                 120                 125
   Pro Arg Pro Ser Leu Ala Lys Lys Gln Arg Phe Arg His Arg Asn Arg
       130                 135                 140
   Lys Gly Tyr Arg Ser Gln Arg Gly His Ser Arg Gly Arg Asn Gln Asn
   145                 150                 155                 160
   Ser Arg Arg Pro Ser Arg Ala Thr Trp Leu Ser Leu Phe Ser Ser Glu
                   165                 170                 175
   Glu Ser Asn Leu Gly Ala Asn Asn Tyr Asp Asp Tyr Arg Met Asp Trp
               180                 185                 190
   Leu Val Pro Ala Thr Cys Glu Pro Ile Gln Ser Val Phe Phe Phe Ser
           195                 200                 205
   Gly Asp Lys Tyr Tyr Arg Val Asn Leu Arg Thr Arg Arg Val Asp Thr
       210                 215                 220
   Val Asp Pro Pro Tyr Pro Arg Ser Ile Ala Gln Tyr Trp Leu Gly Cys
   225                 230                 235                 240
   Pro Ala Pro Gly His Leu
                   245
<210> 5
   <211> 87
   <212> PRT
   <213> Artificial Sequence
<220>
   <223> No.5
<400> 5
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Pro Arg Pro Ser Leu Ala Lys Lys Gln
       50                  55                  60
   Arg Phe Arg His Arg Asn Arg Lys Gly Tyr Arg Ser Gln Arg Gly His
   65                  70                  75                  80
   Ser Arg Gly Arg Asn Gln Asn
                   85
<210> 6
   <211> 107
   <212> PRT
   <213> Artificial Sequence
<220>
   <223> No.6
<400> 6
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Gly Val Pro Gly Gln Val Asp Ala Ala
       50                  55                  60
   Met Ala Gly Arg Ile Tyr Ile Ser Gly Met Ala Pro Arg Pro Ser Leu
   65                  70                  75                  80
   Ala Lys Lys Gln Arg Phe Arg His Arg Asn Arg Lys Gly Tyr Arg Ser
                   85                  90                  95
   Gln Arg Gly His Ser Arg Gly Arg Asn Gln Asn
               100                 105
<210> 7
   <211> 117
   <212> PRT
   <213> Artificial Sequence
<220>
   <223> No.7
<400> 7
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Gln Pro Gln Phe Ile Ser Arg Asp Trp
       50                  55                  60
   His Gly Val Pro Gly Gln Val Asp Ala Ala Met Ala Gly Arg Ile Tyr
   65                  70                  75                  80
   Ile Ser Gly Met Ala Pro Arg Pro Ser Leu Ala Lys Lys Gln Arg Phe
                   85                  90                  95
   Arg His Arg Asn Arg Lys Gly Tyr Arg Ser Gln Arg Gly His Ser Arg
               100                 105                 110
   Gly Arg Asn Gln Asn
           115
<210> 8
   <211> 127
   <212> PRT
   <213> Artificial Sequence
<220>
   <223> No.8
<400> 8
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
               35                  40                  45
   Phe Thr Met Pro Glu Asp Glu Phe Trp Gly Arg Thr Ser Ala Gly Thr
       50                  55                  60
   Arg Gln Pro Gln Phe Ile Ser Arg Asp Trp His Gly Val Pro Gly Gln
   65                  70                  75                  80
   Val Asp Ala Ala Met Ala Gly Arg Ile Tyr Ile Ser Gly Met Ala Pro
                   85                  90                  95
   Arg Pro Ser Leu Ala Lys Lys Gln Arg Phe Arg His Arg Asn Arg Lys
               100                 105                 110
   Gly Tyr Arg Ser Gln Arg Gly His Ser Arg Gly Arg Asn Gln Asn
           115                 120                 125
<210> 9
   <211> 245
   <212> PRT
   <213> Artificial Sequence
<220>
   <223> No.9
<400> 9
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Ser Gln Glu Glu Ser Glu Gly Ser Ser
       50                  55                  60
   Leu Ser Ala Val Phe Glu His Phe Ala Met Met Gln Arg Asp Ser Trp
   65                  70                  75                  80
   Glu Asp Ile Phe Glu Leu Leu Phe Trp Gly Arg Thr Ser Ala Gly Thr
                   85                  90                  95
   Arg Gln Pro Gln Phe Ile Ser Arg Asp Trp His Gly Val Pro Gly Gln
               100                 105                 110
   Val Asp Ala Ala Met Ala Gly Arg Ile Tyr Ile Ser Gly Met Ala Pro
           115                 120                 125
   Arg Pro Ser Leu Ala Lys Lys Gln Arg Phe Arg His Arg Asn Arg Lys
   130 135 140
   Gly Tyr Arg Ser Gln Arg Gly His Ser Arg Gly Arg Asn Gln Asn Ser
   145 150 155 160
   Arg Arg Pro Ser Arg Ala Thr Trp Leu Ser Leu Phe Ser Ser Glu Glu
   165 170 175
   Ser Asn Leu Gly Ala Asn Asn Tyr Asp Asp Tyr Arg Met Asp Trp Leu
   180 185 190
   Val Pro Ala Thr Ser Glu Pro Ile Gln Ser Val Phe Phe Phe Ser Gly
   195 200 205
   Asp Lys Tyr Tyr Arg Val Asn Leu Arg Thr Arg Arg Val Asp Thr Val
   210 215 220
   Asp Pro Pro Tyr Pro Arg Ser Ile Ala Gln Tyr Trp Leu Gly Ser Pro
   225 230 235 240
   Ala Pro Gly His Leu
   245
<210> 10
   <211> 159
   <212> PRT
   <213> Artificial Sequence
<220>
   <223> No.10
<400> 10
   Asp Gln Glu Ser Cys Lys Gly Arg Cys Thr Glu Gly Phe Asn Val Asp
   1 5 10 15
   Lys Lys Cys Gln Cys Asp Glu Leu Cys Ser Tyr Tyr Gln Ser Cys Cys
   20 25 30
   Thr Asp Tyr Thr Ala Glu Cys Lys Pro Gln Val Thr Arg Gly Asp Val
   35 40 45
   Phe Thr Met Pro Glu Asp Glu Ser Gln Glu Glu Ser Glu Gly Ser Ser
   50 55 60
   Leu Ser Ala Val Phe Glu His Phe Ala Met Met Gln Arg Asp Ser Trp
   65 70 75 80
   Glu Asp Ile Phe Glu Leu Leu Phe Trp Gly Arg Thr Ser Ala Gly Thr
                   85                  90                  95
   Arg Gln Pro Gln Phe Ile Ser Arg Asp Trp His Gly Val Pro Gly Gln
               100                 105                 110
   Val Asp Ala Ala Met Ala Gly Arg Ile Tyr Ile Ser Gly Met Ala Pro
           115                 120                 125
   Arg Pro Ser Leu Ala Lys Lys Gln Arg Phe Arg His Arg Asn Arg Lys
       130                 135                 140
   Gly Tyr Arg Ser Gln Arg Gly His Ser Arg Gly Arg Asn Gln Asn
   145                 150                 155
<210> 11
   <211> 165
   <212> PRT
   <213> Artificial Sequence
<220>
   <223> No.11
<400> 11
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Ser Gln Glu Glu Ser Glu Gly Ser Ser
       50                  55                  60
   Leu Ser Ala Val Phe Glu His Phe Ala Met Met Gln Arg Asp Ser Trp
   65                  70                  75                  80
   Glu Asp Ile Phe Glu Leu Leu Phe Trp Gly Arg Thr Ser Ala Gly Thr
                   85                  90                  95
   Arg Gln Pro Gln Phe Ile Ser Arg Asp Trp His Gly Val Pro Gly Gln
               100                 105                 110
   Val Asp Ala Ala Met Ala Gly Arg Ile Tyr Ile Ser Gly Met Ala Pro
           115                 120                 125
   Arg Pro Ser Leu Ala Lys Lys Gln Arg Phe Arg His Arg Asn Arg Lys
       130                 135                 140
   Gly Tyr Arg Ser Gln Arg Gly His Ser Arg Gly Arg Asn Gln Asn Ser
   145                 150                 155                 160
   Arg Arg Pro Ser Arg
                   165
<210> 12
   <211> 175
   <212> PRT
   <213> Artificial Sequence
<220>
   <223> No.12
<400> 12
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Ser Gln Glu Glu Ser Glu Gly Ser Ser
       50                  55                  60
   Leu Ser Ala Val Phe Glu His Phe Ala Met Met Gln Arg Asp Ser Trp
   65                  70                  75                  80
   Glu Asp Ile Phe Glu Leu Leu Phe Trp Gly Arg Thr Ser Ala Gly Thr
                   85                  90                  95
   Arg Gln Pro Gln Phe Ile Ser Arg Asp Trp His Gly Val Pro Gly Gln
               100                 105                 110
   Val Asp Ala Ala Met Ala Gly Arg Ile Tyr Ile Ser Gly Met Ala Pro
           115                 120                 125
   Arg Pro Ser Leu Ala Lys Lys Gln Arg Phe Arg His Arg Asn Arg Lys
       130                 135                 140
   Gly Tyr Arg Ser Gln Arg Gly His Ser Arg Gly Arg Asn Gln Asn Ser
   145                 150                 155                 160
   Arg Arg Pro Ser Arg Ala Thr Trp Leu Ser Leu Phe Ser Ser Glu
             165                 170                 175
<210> 13
   <211> 185
   <212> PRT
   <213> Artificial Sequence
<220>
   <223> No.13
<400> 13
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Ser Gln Glu Glu Ser Glu Gly Ser Ser
       50                  55                  60
   Leu Ser Ala Val Phe Glu His Phe Ala Met Met Gln Arg Asp Ser Trp
   65                  70                  75                  80
   Glu Asp Ile Phe Glu Leu Leu Phe Trp Gly Arg Thr Ser Ala Gly Thr
                   85                  90                  95
   Arg Gln Pro Gln Phe Ile Ser Arg Asp Trp His Gly Val Pro Gly Gln
               100                 105                 110
   Val Asp Ala Ala Met Ala Gly Arg Ile Tyr Ile Ser Gly Met Ala Pro
           115                 120                 125
   Arg Pro Ser Leu Ala Lys Lys Gln Arg Phe Arg His Arg Asn Arg Lys
       130                 135                 140
   Gly Tyr Arg Ser Gln Arg Gly His Ser Arg Gly Arg Asn Gln Asn Ser
   145                 150                 155                 160
   Arg Arg Pro Ser Arg Ala Thr Trp Leu Ser Leu Phe Ser Ser Glu Glu
                   165                 170                 175
   Ser Asn Leu Gly Ala Asn Asn Tyr Asp
               180                 185
<210> 14
   <211> 247
   <212> PRT
   <213> Artificial Sequence
<220>
   <223> RCP-1
<400> 14
   Met Asp Gln Glu Ser Cys Lys Gly Arg Cys Thr Glu Gly Phe Asn Val
   1               5                   10                  15
   Asp Lys Lys Cys Gln Cys Asp Glu Leu Cys Ser Tyr Tyr Gln Ser Cys
               20                  25                  30
   Cys Thr Asp Tyr Thr Ala Glu Cys Lys Pro Gln Val Thr Arg Gly Asp
           35                  40                  45
   Val Phe Thr Met Pro Glu Asp Glu Pro Ser Gln Glu Glu Cys Glu Gly
       50                  55                  60
   Ser Ser Leu Ser Ala Val Phe Glu His Phe Ala Met Met Gln Arg Asp
   65                  70                  75                  80
   Ser Trp Glu Asp Ile Phe Glu Leu Leu Phe Trp Gly Arg Thr Ser Ala
                   85                  90                  95
   Gly Thr Arg Gln Pro Gln Phe Ile Ser Arg Asp Trp His Gly Val Pro
               100                 105                 110
   Gly Gln Val Asp Ala Ala Met Ala Gly Arg Ile Tyr Ile Ser Gly Met
           115                 120                 125
   Ala Pro Arg Pro Ser Leu Ala Lys Lys Gln Arg Phe Arg His Arg Asn
       130                 135                 140
   Arg Lys Gly Tyr Arg Ser Gln Arg Gly His Ser Arg Gly Arg Asn Gln
   145                 150                 155                 160
   Asn Ser Arg Arg Pro Ser Arg Ala Thr Trp Leu Ser Leu Phe Ser Ser
                   165                 170                 175
   Glu Glu Ser Asn Leu Gly Ala Asn Asn Tyr Asp Asp Tyr Arg Met Asp
               180                 185                 190
   Trp Leu Val Pro Ala Thr Cys Glu Pro Ile Gln Ser Val Phe Phe Phe
           195                 200                 205
   Ser Gly Asp Lys Tyr Tyr Arg Val Asn Leu Arg Thr Arg Arg Val Asp
       210                 215                 220
   Thr Val Asp Pro Pro Tyr Pro Arg Ser Ile Ala Gln Tyr Trp Leu Gly
   225                 230                 235                 240
   Cys Pro Ala Pro Gly His Leu
                   245
<210> 15
   <211> 88
   <212> PRT
   <213> Artificial Sequence
<220>
   <223> RCP-2
<400> 15
   Met Asp Gln Glu Ser Cys Lys Gly Arg Cys Thr Glu Gly Phe Asn Val
   1               5                   10                  15
   Asp Lys Lys Cys Gln Cys Asp Glu Leu Cys Ser Tyr Tyr Gln Ser Cys
               20                  25                  30
   Cys Thr Asp Tyr Thr Ala Glu Cys Lys Pro Gln Val Thr Arg Gly Asp
           35                  40                  45
   Val Phe Thr Met Pro Glu Asp Glu Pro Arg Pro Ser Leu Ala Lys Lys
       50                  55                  60
   Gln Arg Phe Arg His Arg Asn Arg Lys Gly Tyr Arg Ser Gln Arg Gly
   65                  70                  75                  80
   His Ser Arg Gly Arg Asn Gln Asn
                   85
<210> 16
   <211> 108
   <212> PRT
   <213> Artificial Sequence
<220>
   <223> RCP-3
<400> 16
   Met Asp Gln Glu Ser Cys Lys Gly Arg Cys Thr Glu Gly Phe Asn Val
   1               5                   10                  15
   Asp Lys Lys Cys Gln Cys Asp Glu Leu Cys Ser Tyr Tyr Gln Ser Cys
               20                  25                  30
   Cys Thr Asp Tyr Thr Ala Glu Cys Lys Pro Gln Val Thr Arg Gly Asp
           35                  40                  45
   Val Phe Thr Met Pro Glu Asp Glu Gly Val Pro Gly Gln Val Asp Ala
       50                  55                  60
   Ala Met Ala Gly Arg Ile Tyr Ile Ser Gly Met Ala Pro Arg Pro Ser
   65                  70                  75                  80
   Leu Ala Lys Lys Gln Arg Phe Arg His Arg Asn Arg Lys Gly Tyr Arg
                   85                  90                  95
   Ser Gln Arg Gly His Ser Arg Gly Arg Asn Gln Asn
               100                 105
<210> 17
   <211> 118
   <212> PRT
   <213> Artificial Sequence
<220>
   <223> RCP-4
<400> 17
   Met Asp Gln Glu Ser Cys Lys Gly Arg Cys Thr Glu Gly Phe Asn Val
   1               5                   10                  15
   Asp Lys Lys Cys Gln Cys Asp Glu Leu Cys Ser Tyr Tyr Gln Ser Cys
               20                  25                  30
   Cys Thr Asp Tyr Thr Ala Glu Cys Lys Pro Gln Val Thr Arg Gly Asp
           35                  40                  45
   Val Phe Thr Met Pro Glu Asp Glu Gln Pro Gln Phe Ile Ser Arg Asp
       50                  55                  60
   Trp His Gly Val Pro Gly Gln Val Asp Ala Ala Met Ala Gly Arg Ile
   65                  70                  75                  80
   Tyr Ile Ser Gly Met Ala Pro Arg Pro Ser Leu Ala Lys Lys Gln Arg
                   85                  90                  95
   Phe Arg His Arg Asn Arg Lys Gly Tyr Arg Ser Gln Arg Gly His Ser
               100                 105                 110
   Arg Gly Arg Asn Gln Asn
           115
<210> 18
   <211> 128
   <212> PRT
   <213> Artificial Sequence
<220>
   <223> RCP-5
<400> 18
   Met Asp Gln Glu Ser Cys Lys Gly Arg Cys Thr Glu Gly Phe Asn Val
   1               5                   10                  15
   Asp Lys Lys Cys Gln Cys Asp Glu Leu Cys Ser Tyr Tyr Gln Ser Cys
               20                  25                  30
   Cys Thr Asp Tyr Thr Ala Glu Cys Lys Pro Gln Val Thr Arg Gly Asp
           35                  40                  45
   Val Phe Thr Met Pro Glu Asp Glu Phe Trp Gly Arg Thr Ser Ala Gly
       50                  55                  60
   Thr Arg Gln Pro Gln Phe Ile Ser Arg Asp Trp His Gly Val Pro Gly
   65                  70                  75                  80
   Gln Val Asp Ala Ala Met Ala Gly Arg Ile Tyr Ile Ser Gly Met Ala
                   85                  90                  95
   Pro Arg Pro Ser Leu Ala Lys Lys Gln Arg Phe Arg His Arg Asn Arg
               100                 105                 110
   Lys Gly Tyr Arg Ser Gln Arg Gly His Ser Arg Gly Arg Asn Gln Asn
           115                 120                 125
<210> 19
   <211> 246
   <212> PRT
   <213> Artificial Sequence
<220>
   <223> RCP-6
<400> 19
   Met Asp Gln Glu Ser Cys Lys Gly Arg Cys Thr Glu Gly Phe Asn Val
   1               5                   10                  15
   Asp Lys Lys Cys Gln Cys Asp Glu Leu Cys Ser Tyr Tyr Gln Ser Cys
               20                  25                  30
   Cys Thr Asp Tyr Thr Ala Glu Cys Lys Pro Gln Val Thr Arg Gly Asp
           35                  40                  45
   Val Phe Thr Met Pro Glu Asp Glu Ser Gln Glu Glu Ser Glu Gly Ser
       50                  55                  60
   Ser Leu Ser Ala Val Phe Glu His Phe Ala Met Met Gln Arg Asp Ser
   65                  70                  75                  80
   Trp Glu Asp Ile Phe Glu Leu Leu Phe Trp Gly Arg Thr Ser Ala Gly
                   85                  90                  95
   Thr Arg Gln Pro Gln Phe Ile Ser Arg Asp Trp His Gly Val Pro Gly
               100                 105                 110
   Gln Val Asp Ala Ala Met Ala Gly Arg Ile Tyr Ile Ser Gly Met Ala
           115                 120                 125
   Pro Arg Pro Ser Leu Ala Lys Lys Gln Arg Phe Arg His Arg Asn Arg
       130                 135                 140
   Lys Gly Tyr Arg Ser Gln Arg Gly His Ser Arg Gly Arg Asn Gln Asn
   145                 150                 155                 160
   Ser Arg Arg Pro Ser Arg Ala Thr Trp Leu Ser Leu Phe Ser Ser Glu
                   165                 170                 175
   Glu Ser Asn Leu Gly Ala Asn Asn Tyr Asp Asp Tyr Arg Met Asp Trp
               180                 185                 190
   Leu Val Pro Ala Thr Ser Glu Pro Ile Gln Ser Val Phe Phe Phe Ser
           195                 200                 205
   Gly Asp Lys Tyr Tyr Arg Val Asn Leu Arg Thr Arg Arg Val Asp Thr
       210                 215                 220
   Val Asp Pro Pro Tyr Pro Arg Ser Ile Ala Gln Tyr Trp Leu Gly Ser
   225                 230                 235                 240
   Pro Ala Pro Gly His Leu
                   245
<210> 20
   <211> 160
   <212> PRT
   <213> Artificial Sequence
<220>
   <223> RCP-7
<400> 20
   Met Asp Gln Glu Ser Cys Lys Gly Arg Cys Thr Glu Gly Phe Asn Val
   1               5                   10                  15
   Asp Lys Lys Cys Gln Cys Asp Glu Leu Cys Ser Tyr Tyr Gln Ser Cys
               20                  25                  30
   Cys Thr Asp Tyr Thr Ala Glu Cys Lys Pro Gln Val Thr Arg Gly Asp
           35                  40                  45
   Val Phe Thr Met Pro Glu Asp Glu Ser Gln Glu Glu Ser Glu Gly Ser
       50                  55                  60
   Ser Leu Ser Ala Val Phe Glu His Phe Ala Met Met Gln Arg Asp Ser
   65                  70                  75                  80
   Trp Glu Asp Ile Phe Glu Leu Leu Phe Trp Gly Arg Thr Ser Ala Gly
                   85                  90                  95
   Thr Arg Gln Pro Gln Phe Ile Ser Arg Asp Trp His Gly Val Pro Gly
               100                 105                 110
   Gln Val Asp Ala Ala Met Ala Gly Arg Ile Tyr Ile Ser Gly Met Ala
           115                 120                 125
   Pro Arg Pro Ser Leu Ala Lys Lys Gln Arg Phe Arg His Arg Asn Arg
       130                 135                 140
   Lys Gly Tyr Arg Ser Gln Arg Gly His Ser Arg Gly Arg Asn Gln Asn
   145                 150                 155                 160
<210> 21
   <211> 166
   <212> PRT
   <213> Artificial Sequence
<220>
   <223> RCP-8
<400> 21
   Met Asp Gln Glu Ser Cys Lys Gly Arg Cys Thr Glu Gly Phe Asn Val
   1               5                   10                  15
   Asp Lys Lys Cys Gln Cys Asp Glu Leu Cys Ser Tyr Tyr Gln Ser Cys
               20                  25                  30
   Cys Thr Asp Tyr Thr Ala Glu Cys Lys Pro Gln Val Thr Arg Gly Asp
           35                  40                  45
   Val Phe Thr Met Pro Glu Asp Glu Ser Gln Glu Glu Ser Glu Gly Ser
       50                  55                  60
   Ser Leu Ser Ala Val Phe Glu His Phe Ala Met Met Gln Arg Asp Ser
   65                  70                  75                  80
   Trp Glu Asp Ile Phe Glu Leu Leu Phe Trp Gly Arg Thr Ser Ala Gly
                   85                  90                  95
   Thr Arg Gln Pro Gln Phe Ile Ser Arg Asp Trp His Gly Val Pro Gly
               100                 105                 110
   Gln Val Asp Ala Ala Met Ala Gly Arg Ile Tyr Ile Ser Gly Met Ala
           115                 120                 125
   Pro Arg Pro Ser Leu Ala Lys Lys Gln Arg Phe Arg His Arg Asn Arg
       130                 135                 140
   Lys Gly Tyr Arg Ser Gln Arg Gly His Ser Arg Gly Arg Asn Gln Asn
   145                 150                 155                 160
   Ser Arg Arg Pro Ser Arg
                   165
<210> 22
   <211> 176
   <212> PRT
   <213> Artificial Sequence
<220>
   <223> RCP-9
<400> 22
   Met Asp Gln Glu Ser Cys Lys Gly Arg Cys Thr Glu Gly Phe Asn Val
   1               5                   10                  15
   Asp Lys Lys Cys Gln Cys Asp Glu Leu Cys Ser Tyr Tyr Gln Ser Cys
               20                  25                  30
   Cys Thr Asp Tyr Thr Ala Glu Cys Lys Pro Gln Val Thr Arg Gly Asp
           35                  40                  45
   Val Phe Thr Met Pro Glu Asp Glu Ser Gln Glu Glu Ser Glu Gly Ser
       50                  55                  60
   Ser Leu Ser Ala Val Phe Glu His Phe Ala Met Met Gln Arg Asp Ser
   65                  70                  75                  80
   Trp Glu Asp Ile Phe Glu Leu Leu Phe Trp Gly Arg Thr Ser Ala Gly
                   85                  90                  95
   Thr Arg Gln Pro Gln Phe Ile Ser Arg Asp Trp His Gly Val Pro Gly
               100                 105                 110
   Gln Val Asp Ala Ala Met Ala Gly Arg Ile Tyr Ile Ser Gly Met Ala
           115                 120                 125
   Pro Arg Pro Ser Leu Ala Lys Lys Gln Arg Phe Arg His Arg Asn Arg
       130                 135                 140
   Lys Gly Tyr Arg Ser Gln Arg Gly His Ser Arg Gly Arg Asn Gln Asn
   145                 150                 155                 160
   Ser Arg Arg Pro Ser Arg Ala Thr Trp Leu Ser Leu Phe Ser Ser Glu
                   165                 170                 175
<210> 23
   <211> 186
   <212> PRT
   <213> Artificial Sequence
<220>
   <223> RCP-10
<400> 23
   Met Asp Gln Glu Ser Cys Lys Gly Arg Cys Thr Glu Gly Phe Asn Val
   1 5 10 15
   Asp Lys Lys Cys Gln Cys Asp Glu Leu Cys Ser Tyr Tyr Gln Ser Cys
   20 25 30
   Cys Thr Asp Tyr Thr Ala Glu Cys Lys Pro Gln Val Thr Arg Gly Asp
   35 40 45
   Val Phe Thr Met Pro Glu Asp Glu Ser Gln Glu Glu Ser Glu Gly Ser
   50 55 60
   Ser Leu Ser Ala Val Phe Glu His Phe Ala Met Met Gln Arg Asp Ser
   65 70 75 80
   Trp Glu Asp Ile Phe Glu Leu Leu Phe Trp Gly Arg Thr Ser Ala Gly
   85 90 95
   Thr Arg Gln Pro Gln Phe Ile Ser Arg Asp Trp His Gly Val Pro Gly
   100 105 110
   Gln Val Asp Ala Ala Met Ala Gly Arg Ile Tyr Ile Ser Gly Met Ala
   115 120 125
   Pro Arg Pro Ser Leu Ala Lys Lys Gln Arg Phe Arg His Arg Asn Arg
   130 135 140
   Lys Gly Tyr Arg Ser Gln Arg Gly His Ser Arg Gly Arg Asn Gln Asn
   145 150 155 160
   Ser Arg Arg Pro Ser Arg Ala Thr Trp Leu Ser Leu Phe Ser Ser Glu
   165 170 175
   Glu Ser Asn Leu Gly Ala Asn Asn Tyr Asp
   180 185
<210> 24
   <211> 463
   <212> PRT
   <213> Artificial Sequence
<220>
   <223> CRP-11
<400> 24
   Gly Pro Leu Gly Asp Gln Glu Ser Cys Lys Gly Arg Cys Thr Glu Gly
   1               5                   10                  15
   Phe Asn Val Asp Lys Lys Cys Gln Cys Asp Glu Leu Cys Ser Tyr Tyr
               20                  25                  30
   Gln Ser Cys Cys Thr Asp Tyr Thr Ala Glu Cys Lys Pro Gln Val Thr
           35                  40                  45
   Arg Gly Asp Val Phe Thr Met Pro Glu Asp Glu Tyr Thr Val Tyr Asp
       50                  55                  60
   Asp Gly Glu Glu Lys Asn Asn Ala Thr Val His Glu Gln Val Gly Gly
   65                  70                  75                  80
   Pro Ser Leu Thr Ser Asp Leu Gln Ala Gln Ser Lys Gly Asn Pro Glu
                   85                  90                  95
   Gln Thr Pro Val Leu Lys Pro Glu Glu Glu Ala Pro Ala Pro Glu Val
               100                 105                 110
   Gly Ala Ser Lys Pro Glu Gly Ile Asp Ser Arg Pro Glu Thr Leu His
           115                 120                 125
   Pro Gly Arg Pro Gln Pro Pro Ala Glu Glu Glu Leu Cys Ser Gly Lys
       130                 135                 140
   Pro Phe Asp Ala Phe Thr Asp Leu Lys Asn Gly Ser Leu Phe Ala Phe
   145                 150                 155                 160
   Arg Gly Gln Tyr Cys Tyr Glu Leu Asp Glu Lys Ala Val Arg Pro Gly
                   165                 170                 175
   Tyr Pro Lys Leu Ile Arg Asp Val Trp Gly Ile Glu Gly Pro Ile Asp
               180                 185                 190
   Ala Ala Phe Thr Arg Ile Asn Cys Gln Gly Lys Thr Tyr Leu Phe Lys
           195                 200                 205
   Gly Ser Gln Tyr Trp Arg Phe Glu Asp Gly Val Leu Asp Pro Asp Tyr
       210                 215                 220
   Pro Arg Asn Ile Ser Asp Gly Phe Asp Gly Ile Pro Asp Asn Val Asp
   225                 230                 235                 240
   Ala Ala Leu Ala Leu Pro Ala His Ser Tyr Ser Gly Arg Glu Arg Val
                   245                 250                 255
   Tyr Phe Phe Lys Gly Lys Gln Tyr Trp Glu Tyr Gln Phe Gln His Gln
               260                 265                 270
   Pro Ser Gln Glu Glu Cys Glu Gly Ser Ser Leu Ser Ala Val Phe Glu
           275                 280                 285
   His Phe Ala Met Met Gln Arg Asp Ser Trp Glu Asp Ile Phe Glu Leu
       290                 295                 300
   Leu Phe Trp Gly Arg Thr Ser Ala Gly Thr Arg Gln Pro Gln Phe Ile
   305                 310                 315                 320
   Ser Arg Asp Trp His Gly Val Pro Gly Gln Val Asp Ala Ala Met Ala
                   325                 330                 335
   Gly Arg Ile Tyr Ile Ser Gly Met Ala Pro Arg Pro Ser Leu Ala Lys
               340                 345                 350
   Lys Gln Arg Phe Arg His Arg Asn Arg Lys Gly Tyr Arg Ser Gln Arg
           355                 360                 365
   Gly His Ser Arg Gly Arg Asn Gln Asn Ser Arg Arg Pro Ser Arg Ala
       370                 375                 380
   Thr Trp Leu Ser Leu Phe Ser Ser Glu Glu Ser Asn Leu Gly Ala Asn
   385                 390                 395                 400
   Asn Tyr Asp Asp Tyr Arg Met Asp Trp Leu Val Pro Ala Thr Cys Glu
                   405                 410                 415
   Pro Ile Gln Ser Val Phe Phe Phe Ser Gly Asp Lys Tyr Tyr Arg Val
   420 425 430
   Asn Leu Arg Thr Arg Arg Val Asp Thr Val Asp Pro Pro Tyr Pro Arg
   435 440 445
   Ser Ile Ala Gln Tyr Trp Leu Gly Cys Pro Ala Pro Gly His Leu
   450 455 460
<210> 25
   <211> 272
   <212> PRT
   <213> Artificial Sequence
<220>
   <223> RCP-12
<400> 25
   Gly Pro Leu Gly Asp Gln Glu Ser Cys Lys Gly Arg Cys Thr Glu Gly
   1 5 10 15
   Phe Asn Val Asp Lys Lys Cys Gln Cys Asp Glu Leu Cys Ser Tyr Tyr
   20 25 30
   Gln Ser Cys Cys Thr Asp Tyr Thr Ala Glu Cys Lys Pro Gln Val Thr
   35 40 45
   Arg Gly Asp Val Phe Thr Met Pro Glu Asp Glu Tyr Thr Val Tyr Asp
   50 55 60
   Asp Gly Glu Glu Lys Asn Asn Ala Thr Val His Glu Gln Val Gly Gly
   65 70 75 80
   Pro Ser Leu Thr Ser Asp Leu Gln Ala Gln Ser Lys Gly Asn Pro Glu
   85 90 95
   Gln Thr Pro Val Leu Lys Pro Glu Glu Glu Ala Pro Ala Pro Glu Val
   100 105 110
   Gly Ala Ser Lys Pro Glu Gly Ile Asp Ser Arg Pro Glu Thr Leu His
   115 120 125
   Pro Gly Arg Pro Gln Pro Pro Ala Glu Glu Glu Leu Cys Ser Gly Lys
   130 135 140
   Pro Phe Asp Ala Phe Thr Asp Leu Lys Asn Gly Ser Leu Phe Ala Phe
   145 150 155 160
   Arg Gly Gln Tyr Cys Tyr Glu Leu Asp Glu Lys Ala Val Arg Pro Gly
                   165                 170                 175
   Tyr Pro Lys Leu Ile Arg Asp Val Trp Gly Ile Glu Gly Pro Ile Asp
               180                 185                 190
   Ala Ala Phe Thr Arg Ile Asn Cys Gln Gly Lys Thr Tyr Leu Phe Lys
           195                 200                 205
   Gly Ser Gln Tyr Trp Arg Phe Glu Asp Gly Val Leu Asp Pro Asp Tyr
       210                 215                 220
   Pro Arg Asn Ile Ser Asp Gly Phe Asp Gly Ile Pro Asp Asn Val Asp
   225                 230                 235                 240
   Ala Ala Leu Ala Leu Pro Ala His Ser Tyr Ser Gly Arg Glu Arg Val
                   245                 250                 255
   Tyr Phe Phe Lys Gly Lys Gln Tyr Trp Glu Tyr Gln Phe Gln His Gln
               260                 265                 270
<210> 26
   <211> 134
   <212> PRT
   <213> Artificial Sequence
<220>
   <223> RCP-13
<400> 26
   Gly Pro Leu Gly Asp Gln Glu Ser Cys Lys Gly Arg Cys Thr Glu Gly
   1               5                   10                  15
   Phe Asn Val Asp Lys Lys Cys Gln Cys Asp Glu Leu Cys Ser Tyr Tyr
               20                  25                  30
   Gln Ser Cys Cys Thr Asp Tyr Thr Ala Glu Cys Lys Pro Gln Val Thr
           35                  40                  45
   Arg Gly Asp Val Phe Thr Met Pro Glu Asp Glu Tyr Thr Val Tyr Asp
       50                  55                  60
   Asp Gly Glu Glu Lys Asn Asn Ala Thr Val His Glu Gln Val Gly Gly
   65                  70                  75                  80
   Pro Ser Leu Thr Ser Asp Leu Gln Ala Gln Ser Lys Gly Asn Pro Glu
                   85                  90                  95
   Gln Thr Pro Val Leu Lys Pro Glu Glu Glu Ala Pro Ala Pro Glu Val
               100                 105                 110
   Gly Ala Ser Lys Pro Glu Gly Ile Asp Ser Arg Pro Glu Thr Leu His
           115                 120                 125
   Pro Gly Arg Pro Gln Pro
       130
<210> 27
   <211> 59
   <212> PRT
   <213> Artificial Sequence
<220>
   <223> RCP-14
<400> 27
   Gly Pro Leu Gly Asp Gln Glu Ser Cys Lys Gly Arg Cys Thr Glu Gly
   1               5                   10                  15
   Phe Asn Val Asp Lys Lys Cys Gln Cys Asp Glu Leu Cys Ser Tyr Tyr
               20                  25                  30
   Gln Ser Cys Cys Thr Asp Tyr Thr Ala Glu Cys Lys Pro Gln Val Thr
           35                  40                  45
   Arg Gly Asp Val Phe Thr Met Pro Glu Asp Glu
       50                  55
<210> 28
   <211> 408
   <212> PRT
   <213> Artificial Sequence
<220>
   <223> RCP-15
<400> 28
   Gly Pro Leu Gly Tyr Thr Val Tyr Asp Asp Gly Glu Glu Lys Asn Asn
   1               5                   10                  15
   Ala Thr Val His Glu Gln Val Gly Gly Pro Ser Leu Thr Ser Asp Leu
               20                  25                  30
   Gln Ala Gln Ser Lys Gly Asn Pro Glu Gln Thr Pro Val Leu Lys Pro
           35                  40                  45
   Glu Glu Glu Ala Pro Ala Pro Glu Val Gly Ala Ser Lys Pro Glu Gly
       50                  55                  60
   Ile Asp Ser Arg Pro Glu Thr Leu His Pro Gly Arg Pro Gln Pro Pro
   65                  70                  75                  80
   Ala Glu Glu Glu Leu Cys Ser Gly Lys Pro Phe Asp Ala Phe Thr Asp
                   85                  90                  95
   Leu Lys Asn Gly Ser Leu Phe Ala Phe Arg Gly Gln Tyr Cys Tyr Glu
               100                 105                 110
   Leu Asp Glu Lys Ala Val Arg Pro Gly Tyr Pro Lys Leu Ile Arg Asp
           115                 120                 125
   Val Trp Gly Ile Glu Gly Pro Ile Asp Ala Ala Phe Thr Arg Ile Asn
       130                 135                 140
   Cys Gln Gly Lys Thr Tyr Leu Phe Lys Gly Ser Gln Tyr Trp Arg Phe
   145                 150                 155                 160
   Glu Asp Gly Val Leu Asp Pro Asp Tyr Pro Arg Asn Ile Ser Asp Gly
                   165                 170                 175
   Phe Asp Gly Ile Pro Asp Asn Val Asp Ala Ala Leu Ala Leu Pro Ala
               180                 185                 190
   His Ser Tyr Ser Gly Arg Glu Arg Val Tyr Phe Phe Lys Gly Lys Gln
           195                 200                 205
   Tyr Trp Glu Tyr Gln Phe Gln His Gln Pro Ser Gln Glu Glu Cys Glu
       210                 215                 220
   Gly Ser Ser Leu Ser Ala Val Phe Glu His Phe Ala Met Met Gln Arg
   225                 230                 235                 240
   Asp Ser Trp Glu Asp Ile Phe Glu Leu Leu Phe Trp Gly Arg Thr Ser
                   245                 250                 255
   Ala Gly Thr Arg Gln Pro Gln Phe Ile Ser Arg Asp Trp His Gly Val
               260                 265                 270
   Pro Gly Gln Val Asp Ala Ala Met Ala Gly Arg Ile Tyr Ile Ser Gly
           275                 280                 285
   Met Ala Pro Arg Pro Ser Leu Ala Lys Lys Gln Arg Phe Arg His Arg
       290                 295                 300
   Asn Arg Lys Gly Tyr Arg Ser Gln Arg Gly His Ser Arg Gly Arg Asn
   305                 310                 315                 320
   Gln Asn Ser Arg Arg Pro Ser Arg Ala Thr Trp Leu Ser Leu Phe Ser
   325 330 335
   Ser Glu Glu Ser Asn Leu Gly Ala Asn Asn Tyr Asp Asp Tyr Arg Met
   340 345 350
   Asp Trp Leu Val Pro Ala Thr Cys Glu Pro Ile Gln Ser Val Phe Phe
   355 360 365
   Phe Ser Gly Asp Lys Tyr Tyr Arg Val Asn Leu Arg Thr Arg Arg Val
   370 375 380
   Asp Thr Val Asp Pro Pro Tyr Pro Arg Ser Ile Ala Gln Tyr Trp Leu
   385 390 395 400
   Gly Cys Pro Ala Pro Gly His Leu
   405
<210> 29
   <211> 195
   <212> PRT
   <213> Artificial Sequence
<220>
   <223> RCP-16
<400> 29
   Gly Pro Leu Gly Pro Ser Gln Glu Glu Cys Glu Gly Ser Ser Leu Ser
   1 5 10 15
   Ala Val Phe Glu His Phe Ala Met Met Gln Arg Asp Ser Trp Glu Asp
   20 25 30
   Ile Phe Glu Leu Leu Phe Trp Gly Arg Thr Ser Ala Gly Thr Arg Gln
   35 40 45
   Pro Gln Phe Ile Ser Arg Asp Trp His Gly Val Pro Gly Gln Val Asp
   50 55 60
   Ala Ala Met Ala Gly Arg Ile Tyr Ile Ser Gly Met Ala Pro Arg Pro
   65 70 75 80
   Ser Leu Ala Lys Lys Gln Arg Phe Arg His Arg Asn Arg Lys Gly Tyr
   85 90 95
   Arg Ser Gln Arg Gly His Ser Arg Gly Arg Asn Gln Asn Ser Arg Arg
   100 105 110
   Pro Ser Arg Ala Thr Trp Leu Ser Leu Phe Ser Ser Glu Glu Ser Asn
   115 120 125
   Leu Gly Ala Asn Asn Tyr Asp Asp Tyr Arg Met Asp Trp Leu Val Pro
   130 135 140
   Ala Thr Cys Glu Pro Ile Gln Ser Val Phe Phe Phe Ser Gly Asp Lys
   145 150 155 160
   Tyr Tyr Arg Val Asn Leu Arg Thr Arg Arg Val Asp Thr Val Asp Pro
   165 170 175
   Pro Tyr Pro Arg Ser Ile Ala Gln Tyr Trp Leu Gly Cys Pro Ala Pro
   180 185 190
   Gly His Leu
   195
<210> 30
   <211> 20
   <212> PRT
   <213> Artificial Sequence
<220>
   <223> Peptide-1
<400> 30
   Asp Gln Glu Ser Cys Lys Gly Arg Cys Thr Glu Gly Phe Asn Val Asp
   1 5 10 15
   Lys Lys Cys Gln
   20
<210> 31
   <211> 20
   <212> PRT
   <213> Artificial Sequence
<220>
   <223> Peptide-2
<400> 31
   Lys Gly Arg Cys Thr Glu Gly Phe Asn Val Asp Lys Lys Cys Gln Cys
   1 5 10 15
   Asp Glu Leu Cys
   20
<210> 32
   <211> 20
   <212> PRT
   <213> Artificial Sequence
<220>
   <223> Peptide-3
<400> 32
   Glu Gly Phe Asn Val Asp Lys Lys Cys Gln Cys Asp Glu Leu Cys Ser
   1               5                   10                  15
   Tyr Tyr Gln Ser
               20
<210> 33
   <211> 20
   <212> PRT
   <213> Artificial Sequence
<220>
   <223> Peptide-4
<400> 33
   Asp Lys Lys Cys Gln Cys Asp Glu Leu Cys Ser Tyr Tyr Gln Ser Cys
   1               5                   10                  15
   Cys Thr Asp Tyr
               20
<210> 34
   <211> 20
   <212> PRT
   <213> Artificial Sequence
<220>
   <223> Peptide-5
<400> 34
   Cys Cys Thr Asp Tyr Thr Ala Glu Cys Lys Pro Gln Val Thr Arg Gly
   1               5                   10                  15
   Asp Val Phe Thr
               20
<210> 35
   <211> 20
   <212> PRT
   <213> Artificial Sequence
<220>
   <223> Peptide-6
<400> 35
   Thr Ala Glu Cys Lys Pro Gln Val Thr Arg Gly Asp Val Phe Thr Met
   1 5 10 15
   Pro Glu Asp Glu
   20
<210> 36
   <211> 20
   <212> PRT
   <213> Artificial Sequence
<220>
   <223> Peptide-7
<400> 36
   Cys Cys Thr Asp Tyr Thr Ala Glu Cys Lys Pro Gln Val Thr Arg Gly
   1 5 10 15
   Glu Val Phe Thr
   20
<210> 37
   <211> 20
   <212> PRT
   <213> Artificial Sequence
<220>
   <223> Peptide-8
<400> 37
<210> 38
   <211> 142
   <212> PRT
   <213> Artificial Sequence
<220>
   <223> RCP-17aa
<400> 38
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Ser Gln Glu Glu Ser Glu Gly Ser Glu
       50                  55                  60
   Asp Ile Phe Glu Leu Leu Phe Trp Gly Arg Thr Ser Ala Gly Thr Arg
   65                  70                  75                  80
   Gln Pro Gln Phe Ile Ser Arg Asp Trp His Gly Val Pro Gly Gln Val
                   85                  90                  95
   Asp Ala Ala Met Ala Gly Arg Ile Tyr Ile Ser Gly Met Ala Pro Arg
               100                 105                 110
   Pro Ser Leu Ala Lys Lys Gln Arg Phe Arg His Arg Asn Arg Lys Gly
           115                 120                 125
   Tyr Arg Ser Gln Arg Gly His Ser Arg Gly Arg Asn Gln Asn
       130                 135                 140
<210> 39
   <211> 143
   <212> PRT
   <213> Artificial Sequence
<220>
   <223> RCP-17
<400> 39
   Met Asp Gln Glu Ser Cys Lys Gly Arg Cys Thr Glu Gly Phe Asn Val
   1               5                   10                  15
   Asp Lys Lys Cys Gln Cys Asp Glu Leu Cys Ser Tyr Tyr Gln Ser Cys
               20                  25                  30
   Cys Thr Asp Tyr Thr Ala Glu Cys Lys Pro Gln Val Thr Arg Gly Asp
           35                  40                  45
   Val Phe Thr Met Pro Glu Asp Glu Ser Gln Glu Glu Ser Glu Gly Ser
       50                  55                  60
   Glu Asp Ile Phe Glu Leu Leu Phe Trp Gly Arg Thr Ser Ala Gly Thr
   65                  70                  75                  80
   Arg Gln Pro Gln Phe Ile Ser Arg Asp Trp His Gly Val Pro Gly Gln
                   85                  90                  95
   Val Asp Ala Ala Met Ala Gly Arg Ile Tyr Ile Ser Gly Met Ala Pro
               100                 105                 110
   Arg Pro Ser Leu Ala Lys Lys Gln Arg Phe Arg His Arg Asn Arg Lys
           115                 120                 125
   Gly Tyr Arg Ser Gln Arg Gly His Ser Arg Gly Arg Asn Gln Asn
       130                 135                 140